# EUROPEAN PATENT APPLICATION

(11) **EP 1 949 933 A2**
(43) Date of publication of application: **30.07.2008**
(21) Application number: 08008505.3
(22) Date of filing: 17.12.2004
(51) Int. Cl.: A61M 39/06, A61M 39/28, A61M 25/00, A61B 10/00

(54) **Medical instrument for accessing a breast duct**

(30) Priority: 23.12.2003 US 746128; 23.12.2003 US 746950; 23.12.2003 US 746940; 23.12.2003 US 746121; 23.12.2003 US 746117
(62) Divisional of application: 04815131.0
(71) Applicant: Cytyc Corporation, Marlborough MA 01752 (US)
(72) Inventor: Sakal, Robert, Bolton, MA 01740 (US); Brennan, Meghan, Newsburyport, MA 01950 (US); Furnish, Greg, Louisville, KY 40206 (US); Whatley, Charles, Louisville, KY 40207 (US); Morris, Ben, Louisville, KY 40205 (US); Wood, Nathan, Winchendon, MA 01475 (US); Macarthur, Douglas, Acton, MA 01720 (US); Hall, Todd, Goshen, KY 40026 (US); Furnish, Simon, New York, NY 10009 (US); Sheets, Ellen, Concord, MA 01742 (US); Gunasekara, Indi, Louisville, KY 40220 (US)
(74) Representative: Tombling, Adrian George

(57) **Abstract**

A medical instrument including a ductal access device comprising a low profile, ergonomic manifold hub usable to introduce fluids into a breast duct and collect ductal fluid samples including ductal epithelial cells from within a breast duct. The ductal access device also comprises an activate valve and an elongated access catheter having a distal end, one lumen and dimensions which permit introduction of the distal end through a ductal orifice and adapted to slideably receive a ductal introducer. The introducer may extend beyond the distal tip of the catheter and serve to penetrate the ductal orifice. The methods comprise the opening a ductal sphincter of the manipulation of a breast duct using controlled fluid pressure through a ductal device.

## Description

### RELATED APPLICATIONS

The present application claims the benefit of U.S. Patent Application Ser. No. 10/746,128, filed on Dec. 23, 2004, of U.S. Patent Application Ser. No. 10/746,950, filed on Dec. 23, 2004, of U.S. Patent Application Ser. No. 10/746,940, filed on Dec. 23, 2004, of U.S. Patent Application Ser. No. 10/746,121, filed on Dec. 23, 2004, and U.S. Patent Application Ser. No. 10/746,117, filed on Dec. 23, 2004. The entire contents of these patent applications are hereby incorporated in their entirety by this reference.
INDEX
Medical Instrument for Accessing a Breast Duct (page 1)
Ductal Lavage Catheter Having an Off-Axis Tip (page 25)
Ductal Lavage Catheter (page 44)
A Method of Opening a Ductal Sphincter Using Controlled Fluid Pressure (page 69)
Ductal Lavage Microcatheter with User Activated Valve and Nitinol Introducer (page 91)

### FIELD OF THE INVENTION

The present invention relates to a medical instrument having at least a portion that is introduced into the body of a mammal in order to perform diagnostic or therapeutic medical procedures, more specifically, the present invention relates to a medical instrument useable during a diagnostic or therapeutic medical procedure that has a low profile and at least a portion sized for introducing into a breast duct through a ductal orifice.

### BACKGROUND OF THE INVENTION

The breast is a specialized, glandular structure including a system of complicated breast ducts that radiate from the nipple and that are bound together by fairly dense connective tissue. Each of these breast ducts includes an associated ductal orifice on the surface of a nipple through which ductal fluid may be expressed. Each duct includes a series of successive interlobular branches that drain through the main, lactiferous branch, which terminates and exits the breast at the nipple via the associated ductal orifice. Immediately proximate the ductal orifice, each lactiferous duct includes a lactiferous sinus in which ductal fluid may accumulate. A ductal sphincter resides within the lactiferous sinus and prevents ductal fluid from unintentionally exiting the breast duct through its associated ductal orifice.

Breast cancer is believed to begin in the lining of these breast ducts. For several decades significant members of the medical community dedicated to studying breast cancer have believed and shown that the cytological analysis of cells retrieved from nipple discharge fluid from within breast ducts may provide valuable information leading to identifying patients at risk for breast cancer. Indeed, Papanicolaou contributed to the genesis of such a possibility of a "Pap" smear for breast cancer by analyzing the cells contained in nipple discharge. More recently, cancer specific markers have been detected in ductal fluid obtained by nipple aspiration. However, the retrieval techniques and instruments used by these members of the medical community did not routinely obtain meaningful ductal fluid samples.

In their attempts to retrieve the breast duct fluid sample including ductal epithelial cells, practitioners introduced wash fluids into a breast duct using indwelling hair-like single lumen catheters. After the fluid was introduced into the duct, the fluid introduction catheters were removed. Then, externally applied nipple aspiration techniques or external pressure applied to the breast were used to collect samples of the ductal fluid. However, these techniques required that significant, sometimes painful, pressure be created on the nipple surface or along the sides of the breast to overcome the fluid retaining properties of the ductal sphincter. Also, these techniques did not routinely provide meaningful ductal fluid samples with a sufficient number of ductal epithelial cells for a meaningful cellular analysis. These techniques typically caused the recovery of samples with twenty or fewer ductal epithelial cells. Additionally, these techniques did not provide samples with cell clusters of 10 or more cells. As a result, the obtained fluid samples could not consistently provide an accurate indication of whether or not the duct from which they were retrieved included precancerous or cancerous cells. Consistent, meaningful ductal epithelial cell samples have been provided by the medical instrument disclosed in U.S. Patent No. 6,413,228 to Hung et al. that is hereby incorporated by reference in its entirety.

Other medical instruments, such as those used during galactography, are introduced into the breast duct in order to visually determine the presence of cancerous cells within a breast duct. However, these devices typically extend a significant distance out of the breast duct during the performed procedure. These distances may be twelve inches or greater. As a result, when an operator is not holding the tool, the moment created by the weight and length of the section of the instrument extending out of the duct may cause the indwelling portion of the instrument to engage the sidewalls of the duct, torque and/or kink the duct and distort the nipple. These effects on the duct and nipple may impede the procedure by twisting or crimping the indwelling portion of the instrument, possibly injuring the patient's duct and causing significant discomfort to the patient. As a result, a patient must either endure the pain and discomfort caused by these long instruments or an attendant must constantly support the instrument above the patient during the medical procedure. However, in the confined space around an operating table and in the area surrounding a nipple surface, it is not practical to have an attendant constantly holding the end of the instrument that is extending into the breast duct. Therefore, prior to receiving the procedure, a patient must decide to either experience discomfort during the procedure or choose not to have the procedure performed. Prior art instruments are also not ergonomically designed for easy grasping and adjusting by a practitioner or attendant while acting in the area surrounding a nipple.

Patients with tight ductal sphincters or tortuous ductal orifices may experience difficulties with the lavage procedure due to twisting or crimping the indwelling portion of the catheter, possibly injuring the patient's duct and causing significant discomfort to the patient. Thus, improved methods for accessing breast ducts with minimal discomfort to the patient are needed.

The human breasts are composed of fatty tissue, fibrous tissue, breast ducts and milk glands. Human breasts are believed to contain from 6 to 8, or more breast ducts. The ductal lavage procedure discussed above, and sampling results may be greatly effective in screening patients for an early warning of breast cancer risk. However, in performing the ductal lavage procedure, a physician may have difficulty inserting the catheter into a breast duct. The breast duct is a complex anatomical pathway to the breast milk glands. The physician must access the breast duct so as not to cause damage to the inner walls of the duct and/or avoid puncturing the duct. However, it is believed the deeper a catheter is inserted into breast duct, the greater the risk of puncturing the breast duct walls. Therefore, a need exists for a ductal access catheter that allows the physician to adjust its flexibility and rigidity so as to adapt the catheter to the ductal geometry and direct the catheter deep into branches of the ductal network.

During the ductal access procedure, a catheter is inserted into a duct opening in the nipple that may cause some discomfort to the patient. Thus, improved insertions systems and methods for ductal access are needed.

### SUMMARY OF THE INVENTION

In accordance with one aspect of the present invention, a medical instrument including a ductal access device comprising a low profile manifold hub usable to introduce fluids into a breast duct and collect ductal fluid samples including ductal epithelial cells and clumps of ductal epithelial cells from within a breast duct is provided. The ductal access device also includes an elongated access catheter having a distal end, one lumen and dimensions which permit introduction of the distal end through a ductal orifice and positioning a distal end distal to the ductal sphincter of a human breast. The catheter may include length indicia on its outer surface that permits a user to determine the depth to which the distal end of the catheter has been introduced. The medical instrument may also include at least one spacing member (spacer) for adjustably positioning the manifold hub a desired distance above the surface of the nipple. The spacing member may control the insertion depth of the catheter into the duct. The medical instrument may also include at least one member for anchoring the device to the breast.

In another aspect of the present invention, an apparatus for being introduced and positioned within a breast duct is provided. The apparatus may introduce or remove material within the breast duct. The apparatus comprises a manifold hub including a plurality of port openings in fluid communication with an interior of the manifold hub. At least two of the openings are in fluid communication with a pair of elongated channels that extend through a portion of the manifold hub. The apparatus also includes a catheter that extends from the manifold hub. The catheter is sized and configured for positioning within a breast duct. The apparatus further includes a retractable spacer that may be moved in a direction parallel to the length of the catheter to space the manifold hub a distance above the surface of a nipple.

In yet another aspect of the present invention, a medical device for introducing a fluid into a breast duct is provided. The medical device comprises a catheter that is sized and configured to extend within a breast duct. The catheter includes an internal lumen that extends substantially parallel to a longitudinal axis of the catheter. The medical device also comprises a manifold hub connected to and in fluid communication with the catheter. The manifold hub includes at least four ports in fluid communication within an interior of the manifold hub. At least two of the at least four ports are in fluid communication with channels formed within the manifold hub for receiving a fluid introduction line and a ductal fluid collection line. The manifold hub has a height that extends parallel the longitudinal axis of the catheter and a length that extends perpendicular to the longitudinal axis of the catheter. The length of the manifold hub is greater than the height of the manifold hub.

In still another aspect of the present invention, a ductal access device for aspirating fluid from a breast duct is provided. The ductal access device comprises a manifold hub for receiving a fluid to be introduced into a breast duct and a catheter that extends from the manifold hub. The catheter is sized for positioning within a breast duct and includes a lumen for introducing and receiving fluid within the breast duct. The lumen is in fluid communication with the manifold hub and sized to receive a ductal fluid sample from within the breast duct. The ductal access device also includes a source of negative pressure in fluid communication with the manifold hub such that ductal fluid samples within the manifold hub are drawn to the source of negative pressure for being received within a collection device.

A catheter in accordance with the present invention may have an outer diameter of about 0.01 inch (0.254 mm) to about 0.05 inch (or 1.27 mm). The catheter may have an inner lumen diameter in the range from about 0.007 inch (or 0.178 mm) to about 0.047 inch (or 1.19 mm). The associated manifold hub may further comprise an infusion connector providing a fluid flow path into the lumen of the catheter from an infusion device; and a collection connector providing a fluid outlet path from the lumen of the catheter to a fluid collection device.

A watertight sealing system may be located at a proximal end of the manifold hub. This sealing system may seal around a dilator or other introducer positioned within and extending through the medical instrument. Such a sealing system may include a Touhy-Borst fitting. A dilator or other introducer member(s) for use with the catheter may have an outer diameter of 0.024 inch (or 0.61 mm) to about 0.001 inch. The introducer may also be tapered.

### BRIEF DESCRIPTION OF THE FIGURES

Figure 1 is a perspective view of a medical instrument according to aspects of the present invention;

Figures 2A and 2B are cross sections of alternative embodiments of the medical instrument illustrated in Figure 1;

Figure 3 is an exploded perspective view of the medical instrument of Figure 1;

Figure 4 is a top view of the medical instrument of Figure 1 with infusion and collection lines extending between a manifold hub and respective infusion and collection devices;

Figure 5 is a side view of the medical instrument illustrated in Figure 1 carrying infusion and collection lines;

Figure 6 is another perspective view of the medical instrument illustrated in Figure 1;

Figures 7 and 8 illustrate an alternative embodiment of the medical instrument according to aspects of the present invention;

Figures 9-10 illustrate a method for introducing the medical instrument of Figure 1 into a breast duct using a stiff introducer; and

Figures 12-14 illustrate an alternative method for introducing the medical instrument of Figure 1 into a breast duct using a flexible introducer.

### DETAILED DESCRIPTION OF THE INVENTION

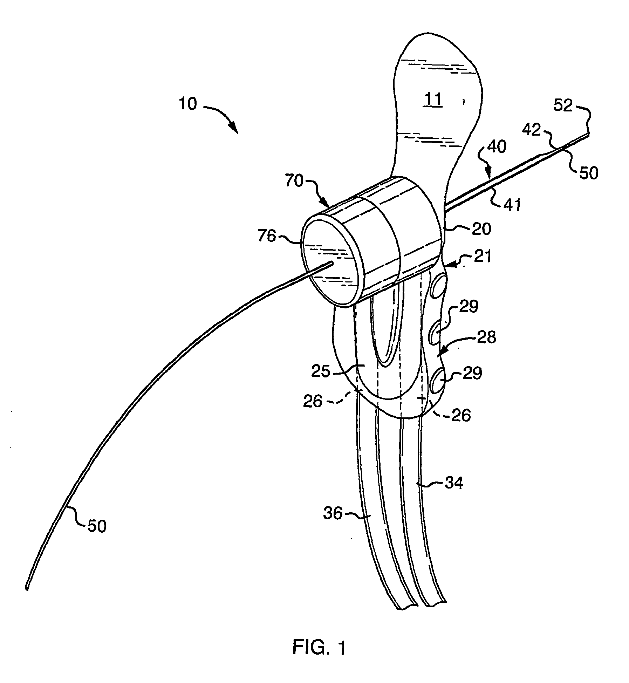

Figure 1 illustrates an embodiment of a low profile, single lumen medical instrument 10 for performing a medical procedure within a breast duct. As used herein, the phrase "medical procedure" may include preparatory procedures, diagnostic procedures or therapeutic procedures. These procedures could include the steps of delivering material(s) into the breast duct and/or retrieving material(s) from within the breast duct.

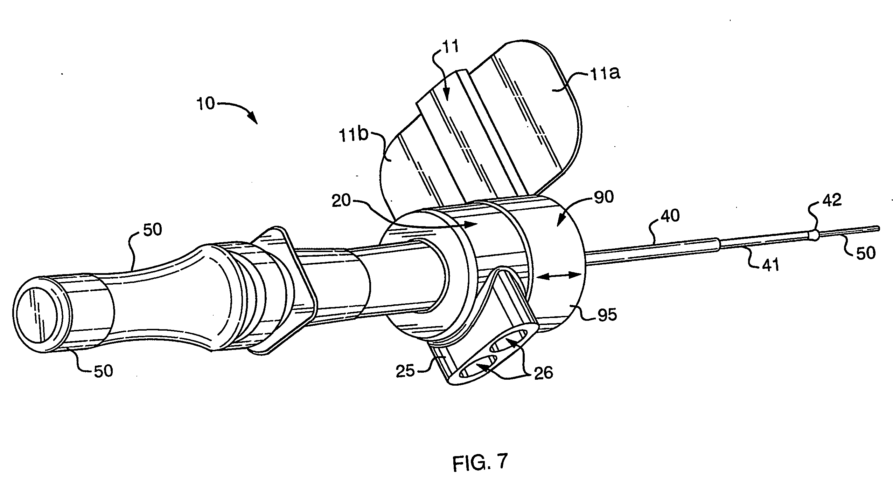

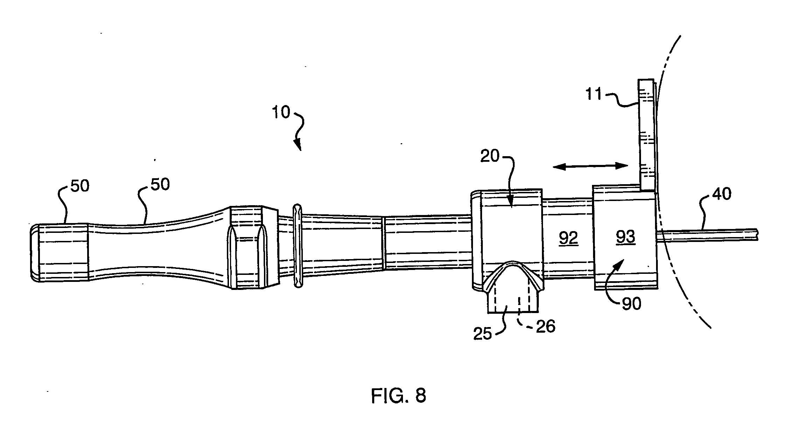

In an embodiment, the medical instrument 10 may be used to infuse ductal wash fluid delivered to the manifold hub 20 into the breast duct, and collect or draw up ductal fluid samples, including hundreds of ductal epithelial cells and/or cell clusters of greater than ten cells, from within the breast duct for analysis. In another embodiment, the medical instrument 10 may be used to infuse a diagnostic agent or therapeutic agent into a breast duct. As shown in Figures 1-8, the instrument 10 may also include a member 11 for securing the instrument 10 to a patient. The securing member 11 may have a biocompatible adhesive on one side for contacting and attaching the instrument 10 to the patient. The member 11 is sized to prevent movement of the manifold hub 20 relative to the body of the patient. In an embodiment illustrated in Figures 7 and 8, sections 11A and 11B of the member 11 may be folded onto each other so that the size of the securing member 11 may be adjusted to the patient and the forces created during the procedure. Additionally, the member 11 may be positioned distal a spacer 90 (discussed below).

As illustrated in Figures 1-6, the medical instrument 10 includes a manifold hub 20 and a ductal access catheter 40 that extends from a distal end 21 of the manifold hub 20. The access catheter 40 is sized to accesses the breast duct. As illustrated, the manifold hub 20 may have a low profile (height) in a direction that extends parallel to the length of the catheter 40. As illustrated in Figures 1-6, the height of the manifold hub 20 may be less than its length (the direction it extends perpendicular to the length of the catheter 40). In a first embodiment, the manifold hub 20 may have a width in a range from about 0.25 inch to about 0.375 inch and a height in a range from about 0.75 inch to about 1.0 inch. In another embodiment, the manifold hub 20 has an internal fluid capacity of 1 ml or less. The low profile of the manifold hub 20 will help to prevent a pivot point from being formed at a location along the length of the catheter 40 at which a large torque may be applied to the duct of a patient during a medical procedure. By eliminating or, at least, significantly reducing any torquing of the duct, the duct will not be kinked, closed due to a change in the position of ductal tissue or injured due to the catheter 40 pushing against the epithelial lining of the duct. The instrument 10 also has an ergonomic design that allows easily handling and grasping by an attendant or practitioner so that the manifold hub 20 and catheter 40 may be easily manipulated.

In the event that the manifold hub 20 needs to be spaced from the nipple surface, a retractable spacer 90 may be positioned on the distal end of the manifold hub and at the proximal end of the catheter 40 as shown in Figures 7 and 8. The retractable spacer 90 may have a spacing distance in the range from about 1mm to about 10mm, most typically in the range of about 5mm. In a first embodiment, the retractable spacer 90 may include a first spacing member 92 received within a second spacing member 93. Alternatively, the first spacing member 92 may telescopically receive the second spacing member 93. In this embodiment, the first member 92 is secured against movement relative to the manifold hub 20, while the second member 93 is moveable relative to both the first member 92 and the manifold hub 20. In an alternative embodiment, both of the spacing members 92, 93 are moveable relative to the manifold hub 20. The distances required for spacing the manifold hub 20 from the surface of the nipple, for example distances of between about 15 mm and 20 mm, may be controlled by locking the spacing members 92, 93 in an extended position (Figure 8). Alternatively, the retractable spacer 90 may be locked in a retracted position (Figure 7). In an alternative embodiment, the retractable spacer 90 includes a single moveable spacing member 95 that slidably receives a portion of the manifold hub 20 to achieve the retracted position and that may be locked at the end of the manifold hub 20 to achieve the extended position. In any of the above-discussed embodiments, the spacing members 92, 93, 95 may be rotated relative to each other and the manifold hub 20 in order to lock each spacing member 92, 93 and 95 against translational movement relative to each other and the manifold hub 20. Any known rotational locking system for telescoping members may be used. Alternatively, the spacing members 92, 93, 95 may be snapped into a locked position using well known snap locks. When additional spacing is needed, members 92, 93, 95 of different sizes or more than two telescoping members may be provided.

The manifold hub 20 may be formed of a transparent material so that an attendant or practitioner may easily view fluid(s) and material(s) within the manifold hub 20. The transparent material may be a plastic, such as ABS plastics or other known plastic materials. As illustrated in Figures 1-8, an embodiment of the manifold hub 20 may have a substantially "F" shape.

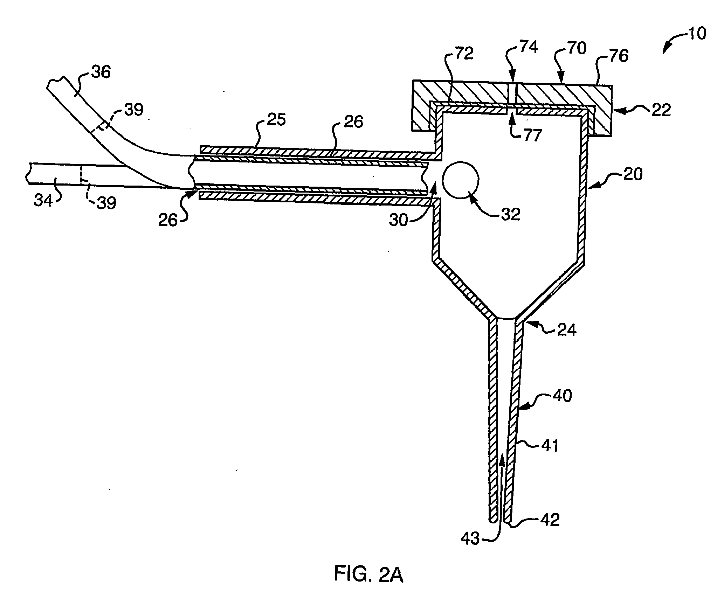

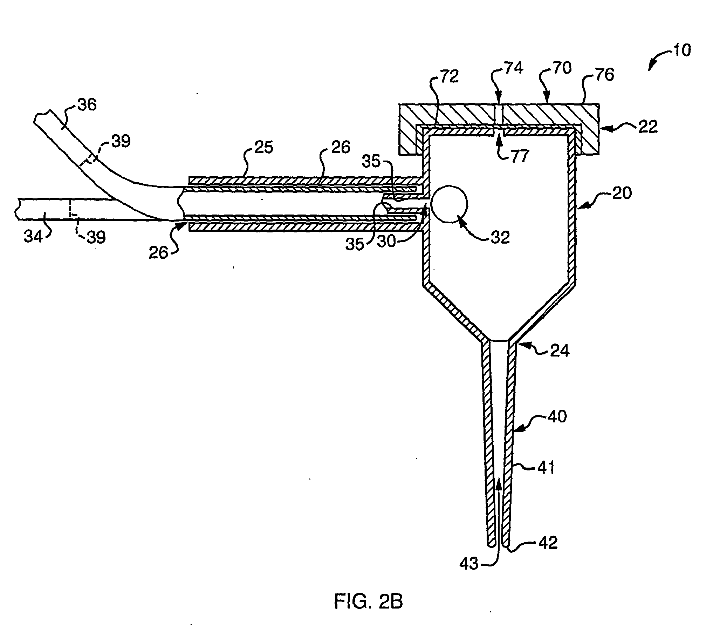

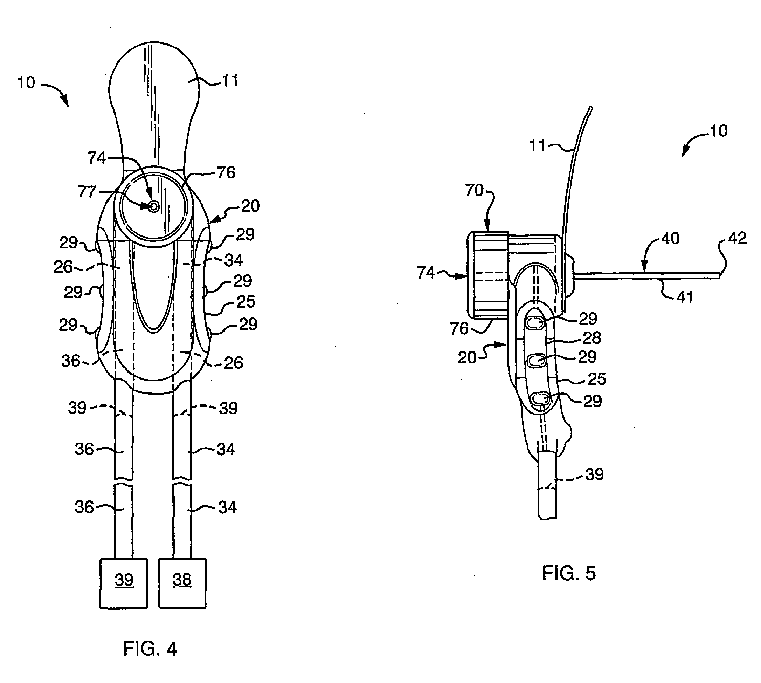

As shown in Figures 2A, 2B and 4, the manifold hub 20 includes a first port 30 for connecting to an infusion tube 34 through which materials including wash fluids, diagnostic agents or treatment agents are delivered from an infusion device 38 to the first port 30, the manifold hub 20 and, eventually, the ductal access catheter 40. The connected infusion device 38 may include a syringe or other known fluid containers. In an embodiment, the infusion device 38 may include a fluid receptacle, such as a bag or a container, positioned at a location above the breast of the patient. In this embodiment, the height of the container above the breast of the patient and gravity are used to deliver the fluid from the infusion device 38 to the infusion tube 34.

As shown in Figures 2A, 2B and 4, the manifold hub 20 also includes a second port 32 for connection to a collection tube 36. Ductal fluid samples collected from within the breast duct may be delivered from the manifold hub 20 to a collection receptacle 39 via the collection tube 36. The collection receptacle 39 may include a syringe or other known fluid collection device including a medical fluid bag or container. In an embodiment, the collection receptacle 39 may include or be connected to a source of negative pressure so that an area of low pressure may be created within the collection tube 36 and, if needed, the manifold hub 20 for assisting in the delivery of the retrieved ductal fluid sample to the collection receptacle 39. The area of low pressure, for example a vacuum in an embodiment, may be created using a bulb syringe, a hand-operated vacuum source, a foot operated vacuum source or motor controlled vacuum source. These vacuum sources may include a pump that creates negative pressure within the collection tube 36. In addition to a source of lower pressure, or in place of the source of lower pressure, low pressure within collection tube 36 may be created by infusing fluid into the manifold hub 20, thereby increasing the pressure within the manifold hub 20 relative to the collection tube 36. In any of these embodiments, the collection receptacle 39 may be positioned at a location below the patient during the procedure so that the collected ductal fluid sample may be delivered to the receptacle 39 by gravity.

The first and second ports 30, 32 may include an opening along the sidewall of the manifold hub 20 that is round, oval or any other geometric shape conducive to fluid flow either into the duct or out from the duct as shown in Figures 2A and 2B. The diameter of the ports 30, 32 may be that diameter which is suitable to achieve a desired flow rate into the duct or aspiration or collection rate out from the duct. Thus, the diameters of the ports 30, 32 may be in a range from about 0.060 inches to about 0.090 inches. One side port 30, 32 may be larger or smaller than the other, especially where such differential port size provides a desired flow rate into or out from one of the lumens, or an overall lavage efficiency of infusion and aspiration or collection of lavage and ductal fluid.

Figures 1-6 illustrate that the first and second ports 30, 32 and their associated tubes 34, 36, respectively, are positioned within a connector housing 25 that extends transverse to the longitudinal axis of the manifold hub 20 and the catheter 40. The connector housing 25 may be integrally formed with the manifold hub 20 as a unitary element. Alternatively, the connector housing 25 may be formed separate of the manifold hub 20 and secured to the manifold hub 20 as discussed below with respect to the catheter 40. The connector housing 25 includes two channels 26 that each receives one of the tubes 34, 36. The channels 26 extend from the ports 30, 32 on the manifold hub 20 to the outer, end surface of the connector housing 25. Each channel 26 aligns the received tube 34, 36 with its respective manifold hub port 30, 32 for easy, reliable and quick connection of the tubes 34, 36 to their respective ports 30, 32. The channels 26 also support the tubes 34, 36 at their point of connection to their ports 30, 32 so that the tubes 34, 36 do not create a moment (that may torque the duct) about the point where they connect to their respective ports 30, 32. The connector housing 25 may also include contoured sidewalls 28 with integrally formed, or otherwise secured, ridges 29 that may be gripped by an attendant or a practitioner. The contoured sidewalls 28 permit easy grasping by the attendant or practitioner and allow the attendant or practitioner to orient the instrument 10 during a procedure without looking at the instrument 10.

In an embodiment shown in Figure 2B, the first and second ports 30, 32 may include posts 35 that extend within connector housing 25 and receive the flexible infusion and collection tubes 34, 36. In an embodiment, the infusion and collection tubes 34, 36 may be formed of flexible tubing such as surgical tubing. However, the tubes 34, 36 may be formed of any flexible material including a flexible plastic. In one embodiment, the tubes 34, 36 are formed of flexible PVC.

The posts 35 may securely receive the tubes 34, 36 when the tubes 34, 36 are positioned over, or within, the posts 35. Alternatively, the ports 30, 32 and their associated posts 35 may include luer lock fittings (not shown) that cooperate with corresponding luer lock fittings on a first end of the tubes 34, 36. The second end of the tubes 34, 36 may also include luer lock fittings that mate with standard luer lock fittings on the syringes or other fluid containers. The luer lock fittings may be either male or female fittings. As discussed herein, the syringes and other fluid containers may carry and infuse saline, diagnostic materials, such as contrast materials, and therapeutic treatment materials into the infusion tube 34. The tubes 34, 36 may be secured to the posts 35 of the manifold hub 20 and the luer locks using a UV curable adhesive or other known bonding agents. In alternative embodiments, the tubes 34, 36 may be secured to the manifold hub 20 and the luer locks by overmolding.

As shown in Figures 1-6, the first port 30 may be positioned adjacent the second port 32 along the perimeter of the manifold hub 20. In the illustrated embodiment, the circumferentially adjacent ports 30, 32 are spaced the same longitudinal distance from the first and second ends 22, 24 of the manifold hub 20. This spacing of the ports 30, 32 provides for a compact and low profile manifold hub 20 that, as discussed above, will not create a moment and associated forces that toque the duct when the manifold hub 20 is positioned on the patient and free of support from a practitioner or other attendant.

The second port 32 may be circumferentially spaced any distance from the first port 30 around the wall of the manifold hub 20. In an embodiment, the first port 30 may be between forty-five and ninety degrees offset from the second port 32 around the circumference of the manifold hub 20. In an alternative embodiment, the first port 30 may be circumferentially offset along the manifold wall from the second port 32 by between ninety and one hundred-eighty degrees. In an embodiment, the first port 30 is circumferentially offset from the second port 32 by about one hundred-eighty degrees so that the first and second ports 30, 32 oppose each other within the manifold hub 20.

In an alternative embodiment, it is possible for the second port 32 to be located along the hub 20 at a position that is spaced a greater longitudinal distance away from the catheter 40 than the first port 30. In this embodiment, the second port 32 may be used to collect the fluid that enters the manifold hub 20 from the collection catheter 40. For example one port may be located about 2.0 cm from the distal tip of the catheter 40 and one port may be located about 2.5 cm from the distal tip of the catheter 40.

The tubes 34 and 36 may each include a one-way check valve 39 (Figure 2A) to control the fluid flow into and out of the manifold hub 20. The check valve 39 in the tube 34 may prevent, for example, wash fluid from flowing back into a syringe connected to tube 34 after being infused into tube 34. Similarly, check valve 39 in tube 36 may be used to prevent retrieved ductal fluid samples in tube 36 from flowing back into the manifold hub 20. In an alternative embodiment, pinch clamps on the tubes 34, 36, may replace one or both of the check valves 39. For example, a check valve 39 may be positioned within the infusion tube 34 and a conventional pinch clamp may be positioned on the collection tube 36. Other known devices for controlling the direction and timing of fluid flow within a tube 34, 36 may also be used.

As shown in Figures 1-6, the catheter 40 includes a thin walled microcatheter 41 that is secured to the manifold hub 20. In a first embodiment, the microcatheter 41 is integrally formed as part of the manifold hub 20. In another embodiment, the microcatheter 41 is formed as a separate piece and then secured to the manifold hub 20 by microwelding or a UV curable adhesive. Other known techniques for securing the microcatheter 41 to the manifold hub 20 could be used. In any of the above-discussed embodiments, the catheter 40 may be coated with a known agent to provide a lubricious coating that allows it to be easily introduced into the breast duct openings. The coating may include a lubricant, a cleaning agent, anesthetic and/or a dilating agent. The microcatheter 41 may be formed of any known biocompatible material such as FEP. The catheter 40 may have an outer diameter in a range from about 0.01 inch (about 0.25 mm) to about 0.05 inch (about 1.25 mm) with an inner lumen 43 having a diameter in the range from about 0.008 inch (about 0.2 mm) to about 0.047 inch (about 1.2 mm). In an embodiment, the microcatheter 41 has an inner lumen 43 having an outer diameter of about 0.030 inch (about 0.762 mm) and an inner diameter of about 0.025 inch (about 0.63 mm).

The catheter 40 may include length indicia (not shown) on an outer surface of the catheter 40 that permits a user to determine the depth to which the distal end of the catheter has been introduced into the breast duct. In an alternative embodiment, the catheter 40 could include an integrally formed or attached stop element (not shown) that prevents insertion of the catheter into the duct beyond a predetermined distance. In one embodiment, the stop element may comprise a knob on the catheter 40 having an increased diameter for preventing the distal portion of the catheter 40 from entering a duct a greater distance than the knob is spaced from the distal end of the catheter40.

As illustrated in Figures 1-7, the catheter 40 may be tapered along its length to make a smooth transition with a received introducer 50 so that a perceptible transition between the catheter 40 and the introducer 50 that would cause any pain to the patient is not formed and felt by the patient. The catheter 40 may also include an atraumatic distal tip portion 42 at its distal end. The distal tip portion 42 may be tapered, contoured and/or rounded so as to produce an atraumatic tip that will reduce or eliminate trauma to the duct upon entry through the ductal orifice and introduction into the ductal lumen past the ductal sphincter. The distal tip portion 42 may also reduce or eliminate trauma upon withdrawal of the catheter 40 from the duct after the medical procedure, such as ductal lavage or the infusion of a diagnostic and/or treatment agent, has been completed. The tip portion 42 may be composed of a soft polymeric material, e.g. including polyvinyl chloride, polyethers, polyamides, polyethylenes, polyurethanes, copolymers thereof and the like. The tip portion 42 may have a diameter in the range from about 0.012 inches (about 0.031 mm) to about 0.020 inches (about 0.051 mm). In an embodiment, the tip portion 42 has a diameter in the range from about 0.014 inches (about 0.036 mm) to about 0.018 inches (about 0.046 mm). The length of the tip portion 42 (extending from the distal end of the distal portion of the catheter 40 toward the proximal end of the catheter 40) may be in a range from about 0.10 inch (about 0.25 cm) to about 1.0 inch (about 2.5 cm), more typically in the range from about 0.20 inch (about 0.50 cm) to about 0.70 inch (about 1.8 cm).

The stiffened distal portion of the catheter 40, including the distal tip 42, may have an average bending stiffness in the range from about 0.010 inch-lbs to about 0.5 inch-lbs. The catheter 40 may also have a stiffness that is similar to that of a heavy suture (approximately 0.025 OD). The proximal portion of the catheter 40 may have a cross-sectional geometry and/or size that inhibits insertion through the ductal orifice and into the ductal lumen.

A Touhy-Borst fitting 70 may be positioned at a proximal end 22 of the manifold hub 20 to allow a user to easily receive and move the catheter 40 over an introducer 50 as shown in Figures 1-6. The Touhy-Borst fitting 70 is positioned at the end of the manifold hub 20 to cover and seal the opening 77 through which an introducer 50 (discussed-below) including a guidewire, stylet, dilator or the like may extend. The Touhy-Borst fitting 70 comprises a silicone plug 72 including a small aperture 74 for receiving the introducer 50, and a threaded cap 76. When the cap 76 is rotated in a first direction, the silicone plug 72 is altered and the size of the aperture 74 is reduced. This results in the silicone plug 72 forming a seal around the inserted introducer 50. When the cap 76 is turned in a second, opposite direction, the aperture 74 and created seal open, thereby allowing the introducer 50 to be removed. The silicone plug 72 may also be closed to seal the proximal end of the manifold hub 20 so when the introducer 50 is not present so that the distal end of the manifold hub 20 is sealed against fluid flow when the proximal end 22 is free of an introducer 50.

The introducer 50 may be located within the manifold hub 20 to assist in placing the catheter 40 into the breast duct and ductal lumen via the ductal opening as shown in Figure 1. The introducer 50 may include a tapered dilator, a series of progressively larger diameter dilators, a guidewire, including tapered guidewires, a stylet or other known introducers. As illustrated, the introducer 50 will pass through the Touhy-Borst fitting 70 at the proximal end 22 of the manifold hub 20 so that the introducer 50 may be removed after positioning of the catheter 40 and prior to the infusion/collection of the wash fluid. As discussed above, prior to being inserted into the breast duct, the Touhy-Borst fitting 70 may be turned down over the introducer 50 during introduction and then backed off when the catheter 40 has been positioned within the breast duct to the desired depth. The introducer 50 may be formed of a stiff material such as a metal wire or a flexible plastic cord. In an embodiment, the introducer 50 may be formed of stainless steel or a flexible material such as polypropylene monofilament. In an alternative embodiment, the introducer 50 may be formed of multiple materials or the same materials having different stiffnesses. As a result, the introducer 50 may have sections that are more flexible than adjacent sections of the same introducer 50. As a result, for example, the introducer 50 may have a first, stiff portion for guiding the introducer 50 within the ductal lumen and a second, flexible portion that allows the introducer 50 to conform to the shape of the ductal lumen or lumen branch into which it is introduced. In any of the above-discussed embodiments, the introducer 50 may be coated with a liquid or dry lubricant material that reduces the friction between the introducer 50 and the breast duct during the introduction and advancement of the introducer 50 in the duct.

The introducer 50 may be made of metal or plastics, including shape memory metals and plastics, and may have a tapered and/or an atraumatic tip for gently probing and accessing a breast duct. Preferably, a tapered tip 52 will extend distally of the catheter 40 during the introduction of the catheter 40 into the breast duct. After access of the duct is complete, the introducer 50 may be withdrawn, the Touhy-Borst fitting 70 may be closed and the indwelling catheter 40 may be positioned at a location distal to the ductal sphincter. The introducer 50 may have an outer diameter of from about 0.005 inch to about 0.030 inch. In an embodiment, the introducer 50 has an outer diameter of about 0.010 inch. The introducer 50 may extend through the manifold hub 20 and the lumen of the catheter 40. The introducer 50 may be tapered over its length.

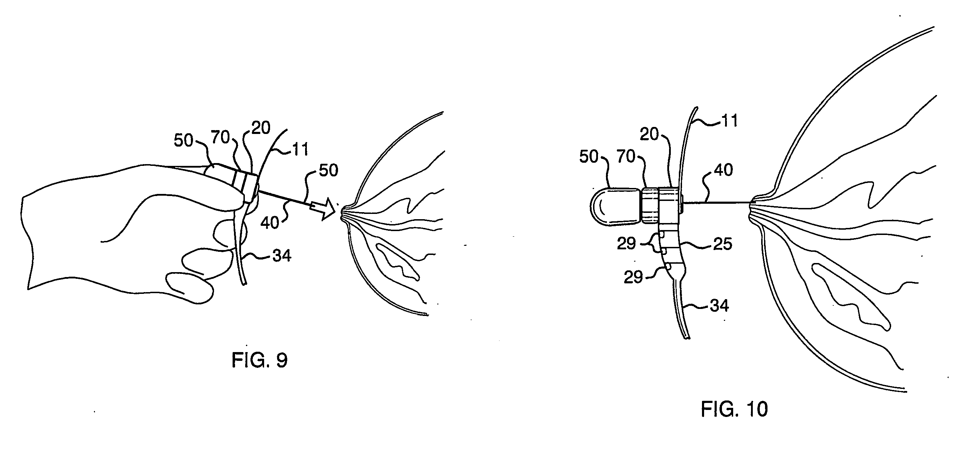

During the process of introducing the catheter 40 into the duct, a ductal opening is located on the surface of a nipple by a practitioner or attendant and a first introducer 50 is advanced through the ductal opening into the duct. The introducer 50 may be a long flexible guide wire, a shorter dilator or any of the other above-mentioned introducers. Prior to, or after the introducer 50 is positioned within the duct, the manifold hub 20 and catheter 40 may receive the first or a second introducer 50. As previously discussed, the Touhy-Borst fitting 70 may be locked about the received introducer 50 to form a fluid tight seal at the distal end of the manifold hub 20 so that fluid does not exit the manifold hub 20 around the introducer 50 during the insertion catheter 40 into the duct (See Figures 9, 10, 12 and 13).

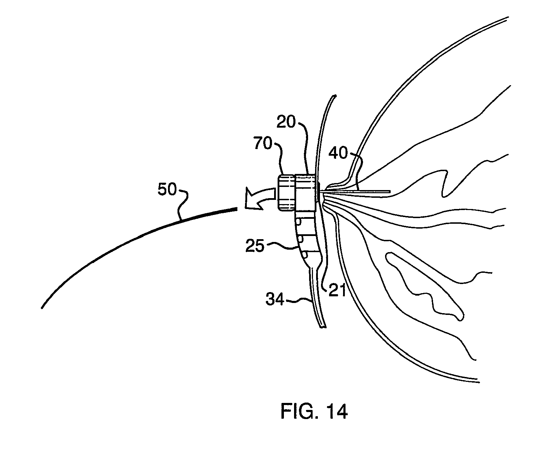

When the catheter 40 is positioned within the duct as intended, the Touhy-Borst fitting 70 is opened and the introducer 50 removed (See Figures 11 and 14). The Touhy-Borst 70 may then be closed again to seal the hub 20. Fluid is then introduced into the manifold hub 20, through the catheter 40 and into the breast duct until resistance is met during the infusion. At this time, it is assumed that the duct is filled. The infusion tube, for example tube 34, may then be closed and the fluid allowed to remain in the duct for a preselected time. During this preselected time, the breast may be massaged and squeezed to stimulate mixing of the wash fluid and ductal fluid, and also ultimately to encourage the fluid to leave the duct and enter the manifold hub 20. The collection tube, for example tube 36, may be opened and the breast squeezed to urge the fluid to progress through the catheter 40 and into the manifold hub 20. If desired, when cloudy return fluid is seen in the hub 20 (which may be transparent or include a transparent window), the infusion tube 34 may be opened and fluid infused into the manifold hub 20 to push the ductal fluid sample that has collected in the hub 20 into the collection tube 36 and a waiting collection receptacle. Alternatively, and possibly additionally, aspiration pressure may be applied within the manifold hub 20 and at the collection tube 36 to aspirate any fluid remaining in the hub 20 into the collection receptacle. The process is repeated either following another infusion of fluid into the duct or by another round of squeezing to encourage return and collection of the infused fluid and cellular material from within the breast duct.

In an embodiment, the method of lavage may include seating a patient substantially upright in a chair during the lavage procedure, rather than the standard or classic supine (face up) position. Alternatively, the patient may be lavaged in a prone position, face down, with nipples and breast down. The prone face down position takes advantage of gravity and allows the breast ducts to drain into the collection receptacle during the procedure when the outflow port is open. Thus, as discussed above, the lavaging procedure may include infusing the breast duct with a wash fluid through an open inflow lumen while an outflow lumen is closed; closing the inflow lumen when the duct is filled; squeezing or massaging the breast or both; and opening the outflow lumen to collect the wash fluid.

The cells collected may comprise ductal epithelial cells; the ductal fluid collected may comprise molecular and cellular material. Analysis of the ductal epithelial cells and/or the molecular and cellular material in the ductal fluid may proceed as described below discussing the diagnostic methods possible of these collected materials. The collected cells and fluid and fluid components may be analyzed. The lavage fluid including the ductal cells may be analyzed for diagnostic purposes. Conditions in a breast milk duct that are desirable to diagnose include a cancer or precancer condition. The precancer condition may include atypical ductal hyperplasia (ADH) or low-grade ductal carcinoma in situ (LG-DCIS). The diagnostic agent may also have the ability to diagnose other breast related conditions, including, e.g. fibrotic, cystic or conditions relating to lactation. Diagnostic agents may be mixed with the ductal fluid (either in the lavage procedure, or after the fluid is collected).

The fluid infused into the duct to lavage the duct may include known, biocompatible fluids. These lavage fluids may include saline, phosphate buffered saline, a nonabsorbable fluid, an isotonic solution, an osmotic solution, a hypotonic solution, and a hypertonic solution. The wash fluid may comprise for example, saline, phosphate buffered saline, a nonabsorbable fluid, an isotonic solution, an osmotic solution, a hypotonic solution, a hypertonic solution.a protein, a colloid, a sugar, a polymer, mannitol, sorbitol, glucose, glycerol, sucrose, raffinose, fructose, lactulose, sodium chloride, polyethyleneglycol (PEG), maltodextrin, dextran (e.g. dextran 70), hydroxyethyl starch, fluid gelatin, a synthetic colloid, an antibody, a binding protein, or albumin.

As mentioned above, a diagnostic or therapeutic agent may be introduced into a breast duct through the manifold hub 20 and catheter 40. The introduced agent for infusing into the duct may comprise a non-absorbable fluid and/or an oncotic agent and/or an osmotic agent. The agent may be soluble. The agent may comprise a molecule that is a protein, a colloid, a sugar, or a polymer. The agent may be mannitol, sorbitol, glucose, glycerol, sucrose, raffinose, fructose, lactulose, sodium chloride, polyethyleneglycol (PEG), maltodextrin, dextran (e.g. dextran 70), hydroxyethyl starch, fluid gelatin, or a synthetic colloid. The agent may comprise a protein and the protein may be a binding protein or an antibody. The binding protein may be albumin. Administering may comprise administering locally, and local administration may comprise administering intraductally. A system for increasing or standardizing an amount of fluid collectable from a milk duct of a breast may comprise infusing a nonabsorbable fluid and/or an osmotic agent and/or an oncotic agent into the ductal lumen, a medical tool for delivering the agent to the ductal lumen, and instructions for use.

Any number of alternative combinations could exist for defining the invention, which incorporate one or more elements from the specification, including the description, claims, and drawings, in various combinations or sub combinations. It will be apparent to those skilled in the relevant technology, in light of the present specification, that alternate combinations of aspects of the invention, either alone or in combination with one or more elements or steps defined herein, may be utilized as modifications or alterations of the invention or as part of the invention. It may be intended that the written description of the invention contained herein covers all such modifications and alterations.

### DUCTAL LAVAGE CATHETER HAVING AN OFF-AXIS TIP

### SUMMARY OF THE INVENTION

Aspects of the present invention pertain to systems and methods for intraductal access and navigation, including a catheter having different ductal tip configurations.

In accordance with one aspect of present invention, an elongated member sized for positioning within a breast duct is provided. The elongated member includes an elongated internal lumen extending along a length of the elongated member and an elongated internal pathway extending along at least a portion of the length of the elongated member for receiving an introducer. The elongated member includes a central longitudinal axis positioned and extending within the internal lumen. The elongated internal passageway includes a centrally disposed longitudinal axis that is spaced from and offset from the central longitudinal axis; and the elongated member includes an elongated distal tip extending beyond a distal end of the internal lumen and along the longitudinal axis.

In another aspect of the present invention, a ductal access device for accessing a breast duct and collecting biological material from within the duct is provided. The access device includes an elongated member having an outer diameter sized for positioning within the breast duct and an internal lumen for infusing a fluid into the breast duct and collecting fluid from the breast duct. The elongated member includes a central axis extending through the internal lumen and a different axis being parallel to the central axis defining an off axis configuration. An elongated distal tip extends beyond the distal end of the internal lumen and along the different axis. The elongated distal tip has a closed distal end for navigating within the breast duct.

In yet another aspect of the present invention, a ductal access system for accessing a breast duct and collecting biological material from within the duct is provided. The access system includes a cannula having an outer diameter sized for positioning within the breast duct and an internal lumen for infusing a fluid into the breast duct and collecting fluid from the breast duct. The cannula has a central axis extending through the internal lumen and a second axis that is parallel to the first axis so as to define an off axis arrangement. An elongated distal member extends along the second axis and beyond the distal end of the internal lumen. The elongated distal member includes an internal passageway which is sized to slidiably or removably receive an elongated introducer. A multiport hub body is attached to the proximal end of the internal lumen and operatively communicated with the internal lumen of the cannula for infusing fluid into the breast duct and for retrieving fluid from the breast duct.

In still another aspect of the present invention, a method for obtaining cellular material from a human breast milk duct using a ductal access device is provided. The method includes the steps of inserting a ductal introducer into an internal passageway of the elongated distal member to form a composite insertion member; inserting the composite insertion member into a ductal orifice of the breast duct; and advancing the internal lumen into the breast duct.

The above and other aspects, features and advantages of the present invention will be readily apparent and fully understood from the following detailed description illustrative embodiments in conjunction with the accompanying drawings, which are included by way of example, and not by way of limitation with regard to the claimed invention.

### BRIEF DESCRIPTION OF THE DRAWINGS

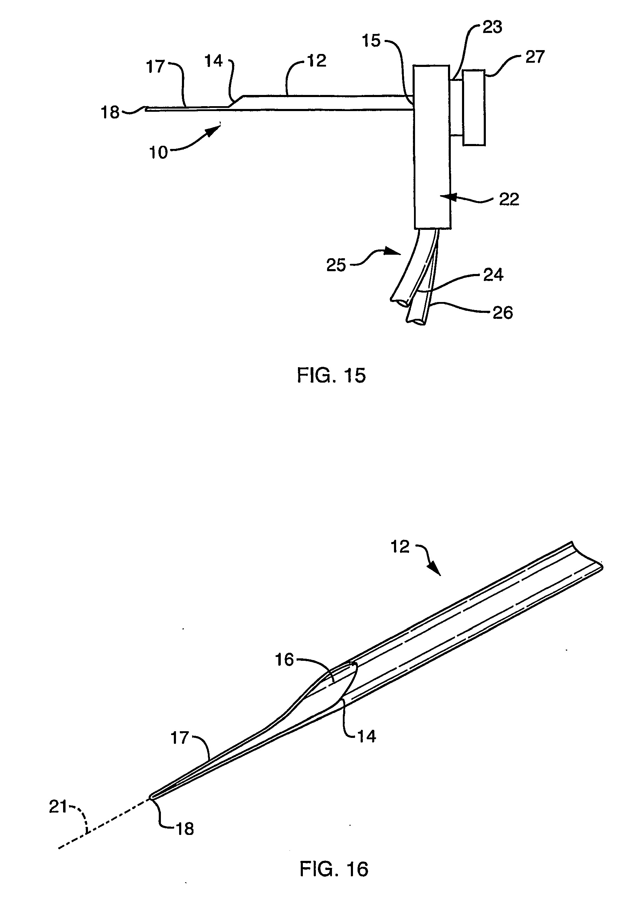

Figure 15 is a schematic representation of a first ductal access device in accordance with one or more aspects of the present invention;

Figure 16 is a perspective view of a catheter in accordance with one or more aspects of the present invention;

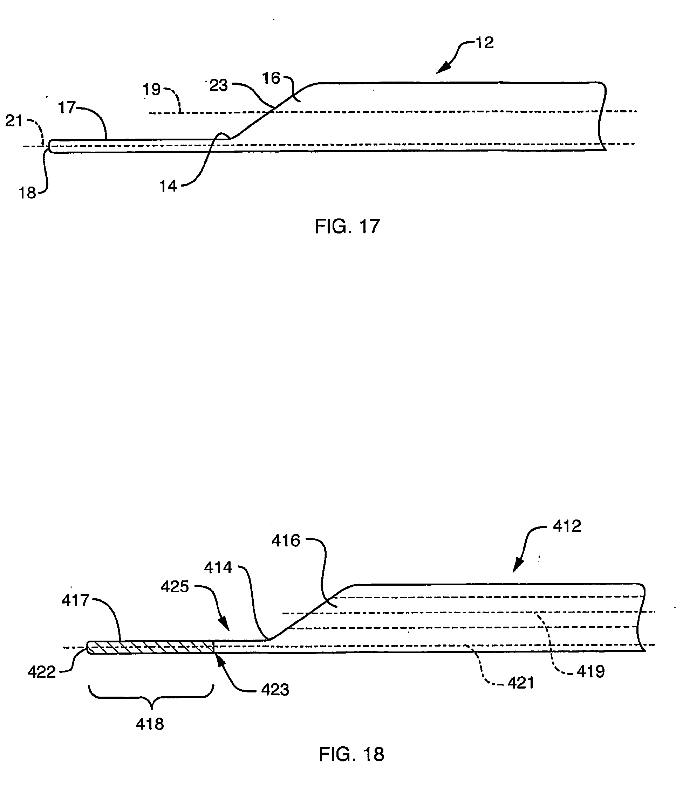

Figure 17 is a partial schematic side view of a catheter in accordance with one or more aspects of the present invention;

Figure 18 is a schematic side view of a catheter with a removable tip in accordance with one or more aspects of the present invention;

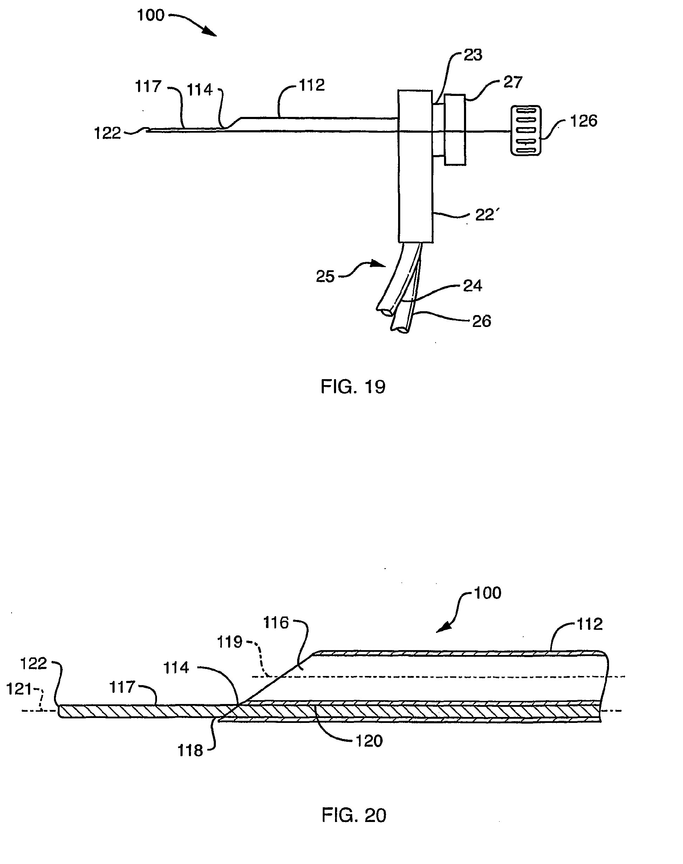

Figure 19 is a schematic representation of a second ductal access device in accordance with one or more aspects of the present invention

Figure 20 is a sectional view of a catheter shown in FIG. 19 taken along an central axis to illustrate an interior construction and components in accordance with one or more aspects of the present invention;

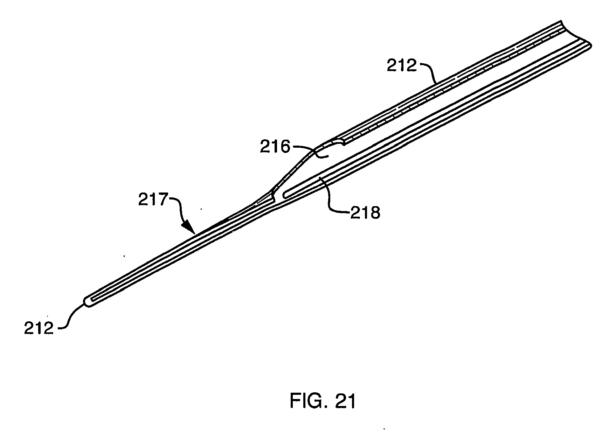

Figure 21 is a perspective view of a catheter in accordance with one or more aspects of the present invention;

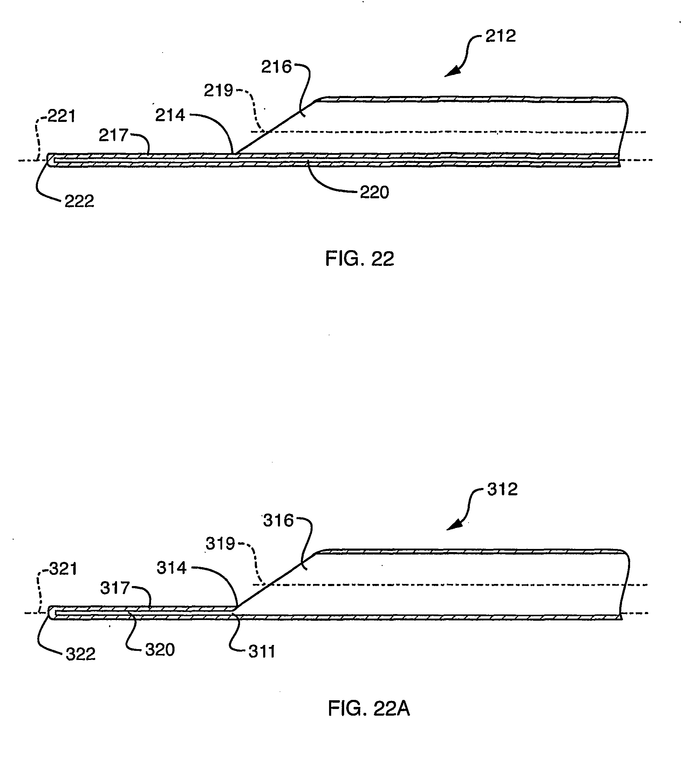

Figure 22 is a sectional view of a catheter shown in FIG. 21 taken along an central axis to illustrate an interior construction and components in accordance with one or more aspects of the present invention;

Figure 22A is a sectional view similar to FIG. 22 showing an alterative embodiment of a catheter in accordance with one or more aspects of the present invention;

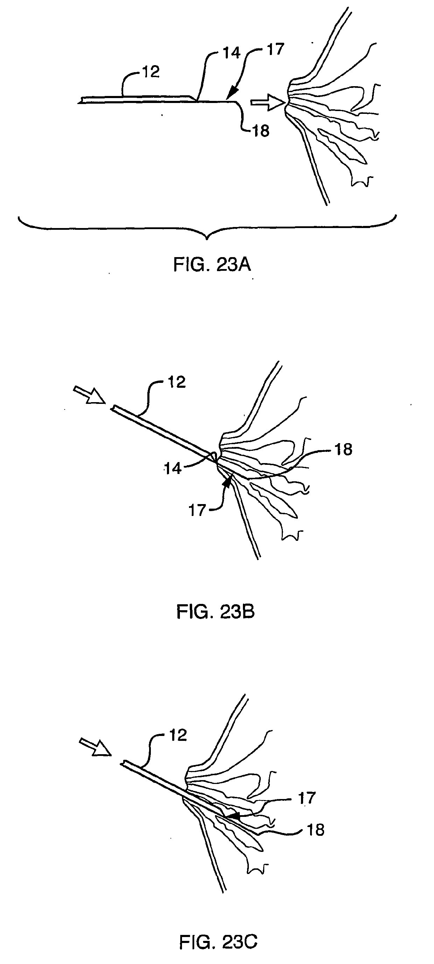

Figures 23A-24C are diagrams illustrating a method of accessing a breast duct which a catheter of FIG. 16;

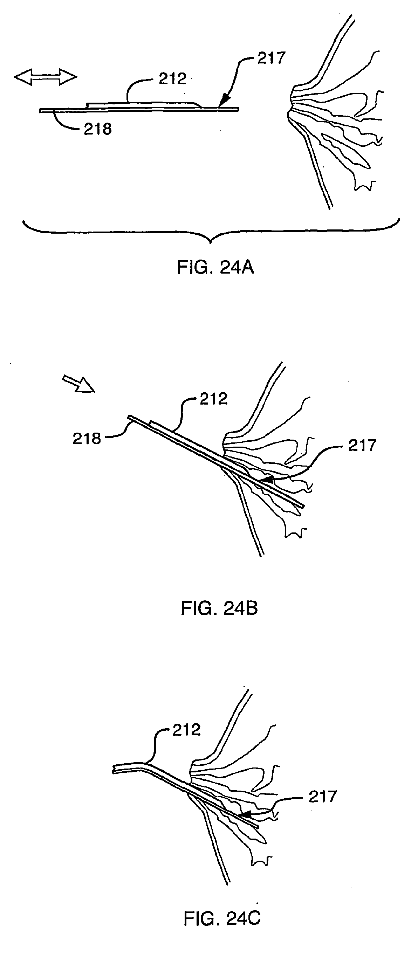

Figures 24A-24C are diagrams illustrating a method of accessing a breast duct which a catheter of FIG. 21;

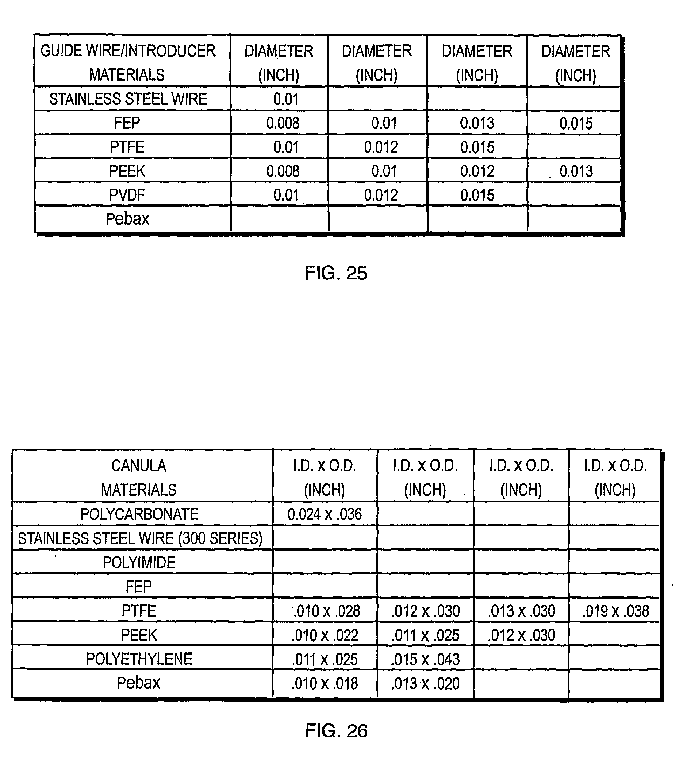

Figure 25 is a table illustrating exemplary parametric values and materials for guide wire and introducers that may be implemented with aspects of the present invention;

Figure 26 is a table illustrating exemplary parametric values and materials for cannula that may be implemented with aspects of the present invention; and

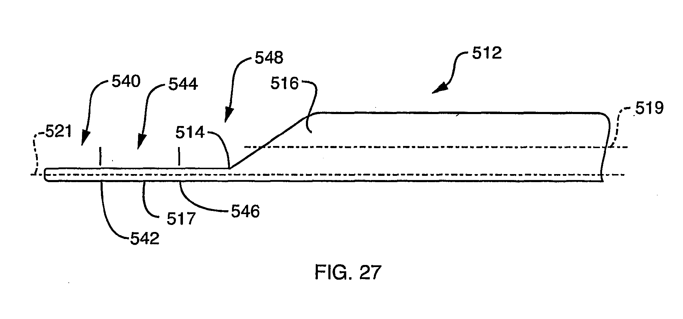

Figure 27 is a partial schematic side view of a catheter in accordance with one or more aspects of the present invention.

### DETAILED DESCRIPTION OF ILLUSTRATIVE EMBODIMENTS

Figures 15-17 illustrate preferred embodiments of an inventive access device 10 for accessing a body orifice, such as a breast duct. The access device 10 comprises a catheter 12 having a distal end 14 with a centrally disposed single lumen 16 which extends the length of the catheter 12, and an elongated distal tip portion 17 which extends beyond the distal end 14 a predetermined distance. The center axis 21 of distal tip portion 17 extends substantially parallel to the center axis 19 of single lumen 16 and is disposed within the wall of the catheter. In such an exemplary construction, the distal tip portion 17 is disposed "off-axis" or "off-center" from center axis 19 of lumen 16. The off-axis construction of distal tip portion 17 advantageously maintains the open inner diameter of the catheter for maximum fluid flow in and out of the breast duct. Further, the off axis configuration allows substantially improved flow because it does not to interfere with the introduction or removal of fluid and collected samples so not to compromise the ability to retrieve a meaningful sample while still being in direction of the catheter and lumen. The elongated configuration of the distal tip portion 17 acts as an effective guide while not interfering with fluid infusion or compromising ductal fluid sample collection. Distal tip portion 17 also provides ease of insertion of catheter 12 in the ductal orifice. In one construction, distal tip portion 17 is flexible in nature so as to allow the catheter to traverse the potentially tortuous and/or angled geometry of the human breast duct. Further, distal tip portion 17 distends the ductal orifice to reduce the associated pain upon insertion of the catheter therein.

Catheter 12 has dimensions which permit introduction of the distal end 14 through a ductal orifice and positioning a distal end thereof distal to the ductal sphincter of a human breast. In one construction, the catheter 12 has a maximum outer diameter of approximately 0.039 inches so as to cannulate the ductal orifices of the breast. Nevertheless, other dimensions are possible for the outer diameter (e.g., approximately 0.025 to 0.039 inches). Single lumen 16 with an internal ID of approximately 0.025 inches accesses the breast duct and through which fluid is infused, and from which ductal fluid samples including ductal epithelial cells are collected or drawn up out of the duct. Distal tip portion 17 has dimensions which permit introduction through a ductal orifice so as to lead the catheter 12 into the breast duct. In one exemplary construction, distal tip portion 17 has a smaller diameter than the catheter 12 to allow ease, of insertion into the breast duct. Distal tip portion 17 may have a diameter of approximately 0.010 inches, although, other dimensions are possible (e.g., 0.008 to 0.012 inches). In an alternative construction, the distal tip portion 17 may be flexible.

Referring to Figures 16 and 17, the distal tip portion 17 may have a tapered configuration being largest at the distal end 14 of the lumen 16 (i.e., proximal end of distal tip 17) extending therefrom to be smallest at the distal end 18. In use, the taper and flexibility of the distal tip portion, 17 guides the catheter for intraductal movement. Shown in Figure 17, the transition between the distal tip portion 17 and catheter 12 includes a beveled surface 23. This beveled surface 23 provides a smooth transition between distal tip portion 17 of catheter 12 which makes the catheter 12 easy to introduce into the ductal opening after the insertion of the tip portion 17 into the breast duct. Hence, the distal tip portion 17 may hold the duct opening in position so that the catheter 12 enters with relative ease. As may be appreciated by one of ordinary skill in the art, the beveled surface 23 reduces the stress level on the tissue being penetrated and spread open by the distal end 14 of the catheter 12. In contrast to traditional catheters, discomfort to the patient is greatly reduced with the access device 10 of the present invention. The edges of the catheter at the distal end and the end 18 of the distal tip 17 may include an atraumatic configuration. In one atraumatic configuration, the edges and end 18 are rounded to reduce friction and eliminate surfaces that could puncture or otherwise injure the duct. Thus, this construction reduces localized trauma to the tissue verses non-atraumatic designs.

Referring to Figure 15, a multiport hub 22 is coupled to the proximal end 15 of catheter 12. In a preferred construction, hub 22 includes transparent material so that the user may visualize the flow to and from the breast duct during a lavage procedure. In another construction, hub 22 has a low profile so as to reduce the torque on the breast nipple after insertion of catheter 12. This overcomes the excessive generated torque on the breast nipple known to cause obstruction of ductal fluid due to compression of the tissue. Thus, improved collection of ductal cellular material is provided.

Hub 22 is attached to a tubing set 25 that comprises an infusion tube 24 from which fluid is infused into lumen 16 through hub 22 and a collection tube 26 from which fluid is collected from lumen 16 via hub 22. Infusion tube 24 and collection tube 26 are attached to hub 22 in a conventional manner to allow fluid flow. In one construction, infusion tube 24 and collection tube 26 are translucent and have standard luer connections at their distal ends that mate with ports on the hub 22 and receive fluids. The proximal ends of the tubes 24, 26 also include standard luer connection that a practitioner or attendant to manage the various ductal fluids using an appropriate syringe with a standard male luer. If desired, tubes 24, 26 may be labeled to indicate the inflow and outflow paths, e.g. infusion or collection functions.

In a further construction, an optional pinch clamp or other flow control device (not shown) may be disposed on the outflow tube, collection tube 26. In use, the clamp closes the collection tube 24 to prevent fluid leakage from the tubing during fluid infusion into a cannulated breast duct. In one construction, hub 22 includes an ergonomic handle 27 for the user to grasp during a ductal lavage procedure. It should be recognized that a fluid used in the hub 22 and catheter 12 may be virtually any appropriate fluid for the ductal lavage procedure. Exemplary ductal wash fluids which may be used with hub 22 includes but is not limited to saline, phosphate buffered saline, a nonabsorbable fluid, an isotonic solution, an osmotic solution, a hypotonic solution, and a hypertonic solution. Nevertheless, an appropriate therapeutic fluid may be provided by way of the ductal access devices described herein. Alternatively, the fluids could include diagnostic or therapeutic fluids carrying diagnostic and/or therapeutic agents.

In an alternative construction depicted in Figures 19 and 20, an inventive ductal access system 100, includes a catheter 112 having a distal end 114 with a centrally disposed single lumen 116 which extends the length of the catheter 112. Catheter 112 includes a tubular introducer pathway 120 adapted to slideably receive a ductal introducer 118 therein. Long axis 121 of pathway 120 is offset but substantially parallel to long axis 119 of lumen 116. For ease of explanation, as used herein the term "introducer" is defined to include guidewires, dilators, stylettes or portion of any of these that may be inserted within a passageway of a catheter or into the ductal orifice. Ductal introducer 118 is provided for ease of varying the length of tip portion 117 so as to improve intraductal travel of the catheter 112 within the breast duct and to reduce risk of puncturing the ductal wall and rupturing the breast duct. By removably inserting the ductal introducer 118 into the axis 121 of pathway 120, the user is able to choose an introducer having a desired stiffness for the particular patient. Additionally, this enables the practitioner or attendant to change the flexibility and stiffness of the introducer 118. The stiffness and flexibility may be a function of a material property and/or cross-sectional shape. One example of a material property that relates to flexibility is the modulus of elasticity. In another example, the cross-sectional shape of the introducer 118 may be circular, elliptical, oval or other shapes that provide predetermined stiffness in one or more directions. One of skill in the art would recognize these various shapes relate to a section modulus of the introducer 118. Therefore, it should be recognized that introducer pathway 120 may be virtually any appropriate cross-sectional shape to meet the cross-sectional shape of the introducer 118.

Ductal introducer 118 includes a distal end 122, which enters the breast duct. The opposing proximal end 124 of ductal introducer 118 includes a handle 126. Handle 126 provides ease of operation so that a user may grasp and manipulate the introducer 118 to access and navigate the breast duct anatomy. Ductal access device 100 includes a hub 22 which has similar construction and components as hub 22 shown in Figure 15. In hub 22, the introducer pathway 120 extends through so that the introducer 118 may be inserted. The pathway may be a tube extending through the exterior of the hub 22 or the tube may be located on the outer surface of the hub. An adjustable tip portion 117 of introducer 118 is defined from the distal end 122 of ductal introducer 118 to the distal end portion 114 of catheter 112. In this configuration, the length of the tip portion 117 is selectively adjustable by the user for accessing and traversing the breast duct. In one aspect, ductal introducer 118 may be embodied as a guide wire which is easily inserted into pathway 120 and into the breast duct. In one construction shown in Figure 20, the center axis 121 of introducer pathway 120 extends substantially parallel to the center axis 119 of single lumen 116. As discussed above, in such an exemplary construction, the introducer pathway 120 is disposed "off axis" or "off-center" from center axis 119 of lumen 116. Thus, ductal introducer 118 with the off axis construction of catheter 112 provides similar benefits as the ductal access device 10.

The introducers described herein may be formed of appropriate cross-sectional shapes and various materials for medical use. The shapes and materials enable desired rigidity and flexibility for intraductal movement. Alternative materials for the introducer 118 may include but are not limited to: Stainless Steel Wire; FEP; PTFE; PEEK; and PVDF and PEBAX. Nevertheless, other appropriate materials may be employed. Specific dimensional value introducers are shown in Figure 25, but are provided by way of example. Alternate coatings for the introducer and/or catheter may include but are not limited to: hydromer coating; STS SLIP-COAT; MDX; silicone dry; silicone lubricant; PTFE coatings. The specific thickness of the coatings and application may be readily determined by one of ordinary skill in the art.

The introducer may be made of metal or plastics and may have a tapered and/or an atraumatic tip for gently probing and accessing a breast duct. In one example, the introducer may be constructed of a superelastic or shape memory material. As used herein, the term "superelastic shape memory material" refers to a class of metal alloys that have a stress-induced phase change or temperate from austenite to martensite and upon stress release, the material springs back to this original phase and shape. The material structure of a superelastic shape memory material regarding austenite and martensite is well-known to one of ordinary skill in the metallurgy art. A NiTi material or NiTi alloy may be used as an alloy material for the introducer. As used herein, a NiTi superelastic shape memory material refers to an alloy that is an intermetallic compound of nickel and titanium having nearly equal mixtures as measured by weight. One composition of a NiTi superelastic shape memory material generally has a greater percentage of nickel by weight than titanium, such as 51%-56% of nickel, and preferably 54-55% nickel. The specific percentages of nickel and titanium may be adjusted by one of ordinary skill in the art.

It is not necessary for the introducer to be composed of a material that has shape memory characteristics. The introducer may also be formed of a stiff material such as a metal wire or a flexible material such as plastic. In an embodiment of the invention, the combined introducer may be formed of Nitinol due to its flexibility, durability, and biocompatibility. In an alternative embodiment, the introducer may be formed of multiple materials or the same materials having different stiffnesses. As a result, the introducer may have sections that are more flexible than adjacent sections of the same introducer. As a result, for example, the introducer may have a first, stiff portion for guiding the introducer within the ductal lumen and a second, flexible portion that allows the introducer to conform to the shape of the ductal lumen or lumen branch into which it is introduced. In any of the above-discussed embodiments, the introducer may be coated with a liquid or dry lubricant material that reduces the friction between the introducer and the breast duct during the introduction and advancement of the introducer in the duct.

Figures 21 and 22 illustrate an alternative construction of an inventive catheter body 212 having a distal end 214 with a centrally (about axis 219) disposed single lumen 216 that traverses the length of the catheter body. In an off-axis configuration, an elongated flexible tip 217 extends beyond the distal end 214 along axis 221. The tip 217 is configured to receive a removable introducer 218. This feature is achieved in that the tip 217 and catheter body 212 include an internal passageway 220 adapted to slideably receive the introducer 218. The passageway 220 may be tubular at the tip 217 and is located in the walls of catheter body 212. Passageway 220 has a closed distal end 122, which is in the initial portion that enters the breast duct. The passageway 220 is opened at the proximal end 211. The closed end feature enables the practitioner or user to adjust the flexibility of the distal 217 during cannulation of the breast duct but prevent fluid from entering the passageway 220. Further, the closed end feature allows the introducer 218 to be removed and another introducer inserted therein when the catheter 212 is positioned within the breast duct. Thus, introducer 218 may be removed or inserted into the tip 217 to selectively adjust the flexibility of tip 217. This allows the user to insert the introducer 218 making the flexible tip more rigid for insertion into the duct, or remove the stylette making the tip more flexible so the tip may transverse tortuous ductal geometry. In this configuration, passageway 220 extends the length of the lumen 216 and is parallel thereto. Advantageously, an aspect of the present invention provides an option to change to tip flexibility which greatly aids the cannulation of the breast duct. The material and thickness of flexible tip 217 may be chosen to achieve a desired stiffness in combination with the flexure characteristics of introducer 218. The closed distal end 222 is rounded to reduce friction between the ductal tissue and tip 217 and prevent the distal end 222 from puncturing duct.

Figure 22A illustrates an alternative catheter body 312, which has similar construction as catheter body 212 except that a passageway 320 is in the distal tip 317 and not in the walls of the catheter body 312. This allows the user to insert the introducer 218 through the lumen 316 and into the proximal end 311 of the passageway 320. Flexible elongated distal tip 317 includes a closed distal end 322 extending to the distal end 314 of catheter body 312.

In the example of the embodiments shown Figures 22 and 22A, the catheter 212, 312 may be provided with a plurality of introducers each of which have a different stiffness characteristic or property. Introducers are provided depending on the portion of duct being accessed. One introducer may be used to introduce the distal tip 217, 317 past the ductal sphincter. Another introducer may be provided to enter a desired branch of breast duct. Yet another introducer, if desired, may be used to enter a final portion of branch. This allows practitioner to design stiffness of tip 217, 317 to match needs presented by different portions of the duct.

An introducer may be designated with a stiffness value to provide a certain stiffness property relative to the other introducers. Purely by way of example, a practitioner may be provided with an introducer having a stiffness value of two which is designed to be rigid so as to allow penetrating the ductal opening. Another introducer may be provided having a stiffness value four which would be less stiff than a value of two. Yet another introducer may have a value of eight which would be less stiff than a value of four. In this example, a practitioner is enabled to access the breast duct with a rigid introducer inserted within the passageway 220, 320. The practitioner, while the catheter 212, 312 resides within the breast duct, may remove the rigid introducer and an insert another introducer to continue to advance within the breast duct. Thus, the practitioner is allowed to enter the breast with the catheters 212, 312 without removing the catheter once the breast duct is accessed. Advantageously, catheters 212, 312 allow the practitioner to be more efficient in the ductal access procedure because steps are eliminated or substantially reduced over conventional procedures, e.g., the use of dilators be significantly reduced. Further, patient comfort is increased because less traumatic movement is provided to the breast.

In another construction shown in Figure 18, a catheter 412 has a distal end 414 with a single lumen 416 disposed about a central axis 419 which extends the length of the catheter, and an elongated distal tip portion 417 which extends beyond the distal end 414. Catheter 412 has a bevel face and the tip portion 417 includes an extensible introducer 418 formed as a stylette portion. Stylette portion 418 comprises a distal end 422 extending from a distal opening 423 of a transition portion 425 of the lumen 416. Transition portion 425 includes a passageway configured to receive a stylette so that the distal end 422 exits the opening 433. The stylette portion 418 may be made of a flexible metal, plastic material or other materials described above. The stylette portion 418 may be fixed in position and serve as a flexible tip, or it may be removed after insertion of the catheter into the duct. The stylette portion 418 may be removed and replaced with a shorter stylette portion so as to shorten the tip length. Alternatively, the stylette portion 418 may have a longer length. In this manner, the tip length may be adjusted to accommodate the particular duct geometry.

Figure 27 illustrates an alternative construction of an inventive catheter body 512 having a distal end 514 with a centrally (about axis 519) disposed single lumen 516 that traverses the length of the catheter body. In an off-axis configuration, an elongated tip 517 extends beyond the distal end 514 along axis 521. The elongated tip 517 may have a multi-flexion configuration that has separate regions of different flexions that each corresponds to the flexibility, or lack thereof, needed to access a respective portion of a breast duct. This multi-flexion zone configuration provides adaptability for a practitioner to reduce steps for accessing a breast and/or customize the access of the duct to increase patient comfort. In one exemplary example, the elongated tip 517 may have three flex zones to accommodate to access a breast duct. A first flexion zone 540 may extend from the distal end to a first intermediate position 542 along the length of the elongated tip. The first flexion zone 540 maybe substantially rigid for entering into the breast duct to overcome the resistance force provided by the tissue at the ductal opening. An adjacent second flexion zone 544 may extend to another intermediate position away from first intermediate position 542 along the length of the elongated tip 517. The second flexion zone 544 may be less rigid than a first flex zone 544 so as to allow the elongated tip 517 to traverse the breast duct geometry. A third flex zone 548 may be provided adjacent to the second flexion zone 544. The third flexion zone 548 may be more flexible than the second flexion zone. In a specific example, the dimensions of the first flexion zone maybe 3 cm from the distal end; second flexion zone may have a length of 4 to 8 cm; and the third flexion own may have a length of 2 to 3 cm. Nevertheless, the length of the zones maybe configured as desired by the practitioner. "

Figures 23A-23C are schematic diagrams illustrating a method of accessing a breast duct by way of a catheter 12 of Figure 16. Referring to Figure 23A, the catheter 12 is prepared to be introduced into a ductal orifice so that a distal end thereof will be positioned within the duct beyond the ductal sphincter during ductal lavage, introduction of diagnostic materials and/or introduction of therapeutic materials. As shown in Figure 23B, the distal end 14 of the catheter is outside of the duct. The catheter has a flexible distal tip 17 which provides ease of insertion of catheter 12 in the ductal orifice. Further, the flexible distal tip 17 is inserted into the duct so as to follow the intraductal geometry but not cause damage to the duct. As shown in Figure 23C, the distal end 14 has entered the duct. The distal tip 17 continues to follow the intraductal geometry to guide the catheter 12 there through. Further, the catheter 12 is more rigid than the distal tip 17 so as to straighten the duct for infusion and collection. Although catheter 12 is described with reference to Figure 23A-23C, other catheters 112, 212, 312, 412, and 512 may be used in a similar fashion.

Figures 24A-24C are schematic diagrams illustrating a method of accessing a breast duct with a catheter body 212 of Figure 21. Referring to Figure 24A, the catheter body 212 is prepared to be introduced into a ductal orifice. The practitioner may choose an introducer 218 of a desired flexibility or rigidity. For example, the introducer 218 of different rigidity properties may be inserted for adjusting to a desired tip characteristic during insertion of the distal tip 217 into the breast duct. This arrangement creates a composite insertion member for accessing the breast duct. The desired tip characteristic may relate to the stiffness and flexibility, which may be functions of a material property or a cross-sectional shape. One of skill in the art would recognize that the composite insertion member may have various shapes and material properties to vary the section modulus.

In particular, the tip 217 may be selectively made rigid to penetrate the duct orifice. In this configuration, the length of the catheter 212 and distal tip 217 is relatively rigid. In Figure 24B, the introducer 218, which is rigid or somewhat rigid, has been removed from the distal tip 217 while the catheter 212 is inserted in the duct. In this removed configuration, the catheter 212 may flex and bend through the intraductal geometry. If desired, an introducer 218, which is less stiff, may be inserted into the distal tip 217. With or without an introducer 218, the catheter 212 is advanced further into the duct. In Figure 24C, once a desired position is reached within the duct, a rigid or somewhat rigid introducer 218 may be inserted and advanced into the catheter 212 to straighten the duct for infusion and collection. In a shape memory embodiment, the stiffness or flexibility of introducer 218 may be adjusted in response to the addition of stimulus or removal of a stimulus without removal from the catheter 212. While not shown, it should be recognized that distal tip 217 could be inserted into the ductal orifice without the need of an introducer 218. The introducer of an appropriate stiffness may be inserted into the distal tip 217 during or after intraductal entry of the catheter 212. Although catheter 212 is described with reference to Figure 24A-24C, other catheters 112, 312, 412 and 512 may be used in a similar fashion.

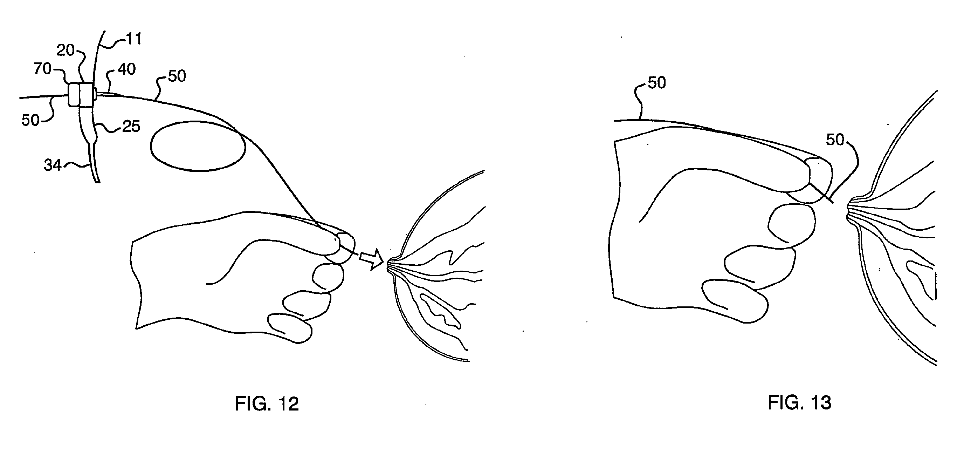

The catheters as described herein may be constructed of a wide range of appropriate materials for medical use. In a preferred construction, the catheter is formed with PTFE and coated with STS Slipcoat to reduce friction on the device during placement in the breast duct. In lieu of PTFE, alternate materials for the catheter may include but are not limited to: polycarbonate; stainless steel (300 Series); polymide; FEP; PEEK; polyethylene; and PEBAX. Specific dimensional values of catheters are shown in Figure 12, but are provided by way of example. Nevertheless, other appropriate materials and dimensional values may be employed.

The hub 22 housing may be molded from a polycarbonate. The tubing set 25 may be made of PVC with luer connectors made of polycarbonate. The tubing set may be made of a variety of conventional materials including but not limited to: silicone; low density polyethylene; PVC; tygon; or polypropylene.

In one manufacturing process, the catheters according to one or more aspects of the present invention may be constructed by using an extrusion process. After the process, the distal end of catheter is shaped by molding and/or putting. An optional, bevel may be is cut to provide the transition between the distal tip portion and the catheter. The edges of the catheter may be rounded using an RF tipping process known to one of ordinary skill in the art. The catheter may be bonded to the hub using a desired medical grade UV adhesive. In turn, the tubes may be bonded to the hub and to the luer connector using UV adhesive as well. If desired, a pinch clamp may be placed on the outflow tube.

The present invention provides a method for obtaining cellular material from a human breast milk duct includes introducing a ductal access device such as devices 12, 112, 212, 312, 412, and 512 having at least one lumen into a duct. A wash fluid may be introduced through the access device internal lumen into the milk duct. In the method, a volume of at least 2 ml of wash fluid may be present within the duct for a preselected time, and then at least a portion of the wash fluid is collected from the duct through the lumen of the access device. The method preferably further comprises massaging and squeezing the breast tissue after introducing the wash fluid but prior to and/or during collecting a portion of the wash fluid. Introducing the ductal access device typically comprises positioning a distal end of the catheter distal to the ductal sphincter in the breast duct. The access device preferably includes only a single lumen that extends into the duct. The preselected time may be less than one second, but will usually be in the range from one second to one hour.

Accordingly, there are any number of alternative combinations for defining the invention, which incorporate one or more elements from the specification, including the description, claims, and drawings, in various combinations or sub combinations. It will be apparent to those skilled in the relevant technology, in light of the present specification, that alternate combinations of aspects of the invention, either alone or in combination with one or more elements or steps defined herein, may be utilized as modifications or alterations of the invention or as part of the invention. It may be intended that the written description of the invention contained herein covers all such modifications and alterations.

### DUCTAL LAVAGE CATHETER

### SUMMARY OF THE INVENTION

Aspects of the present invention pertain to systems and methods for intraductal access and navigation, including a catheter having different distal tip configurations and an introducer having different lengths. In accordance with one aspect of present invention, a device is disclosed for being introduced and positioned within a breast duct for introducing or removing material within the breast duct, the apparatus comprising a manifold hub comprising a plurality of port openings in fluid communication with an interior of the manifold hub, at least two of the openings being in fluid communication with a pair of elongated channels that extend through a portion of the manifold hub and a catheter extending from the manifold hub, the catheter comprising an elongated internal lumen extending substantially parallel to a longitudinal axis of the catheter, said lumen being in fluid communication with the manifold hub and adapted to slidiably receive a ductal introducer. The introducer may extend at least approximately 0.250, 0.492, or 0.984 inches beyond the distal tip of the catheter. The distal tip of the catheter may have an atraumatic configuration.

In yet another aspect of the present invention, a device is disclosed for being introduced and positioned within a breast duct for introducing or removing material within the breast duct, said apparatus comprising a manifold hub comprising a plurality of port openings in fluid communication with an interior of said manifold hub, at least two of said openings being in fluid communication with a pair of elongated channels that extend through a portion of said manifold hub and a catheter extending from the manifold hub and a sheath which extends internally through both the hub and the catheter, comprising an elongated internal lumen extending substantially parallel to a longitudinal axis of said sheath, said lumen being in fluid communication with said manifold hub and adapted to slidiably receive a ductal introducer therein and said sheath extends beyond the distal tip of said catheter. The distal tip of the sheath may extend at least approximately 1.5mm, 6.0, or 18.5 millimeters beyond the distal tip of the catheter. The distal tip of the catheter may have an atraumatic configuration.

In still another aspect of the present invention, a method for obtaining cellular material from a human breast milk duct using a ductal access comprising a manifold hub comprising a plurality of port openings in fluid communication with an interior of the manifold hub, at least two of the openings being in fluid communication with a pair of elongated channels that extend through a portion of the manifold hub and a catheter extending from the manifold hub, the catheter comprising an elongated internal lumen extending substantially parallel to a longitudinal axis of the catheter, said lumen being in fluid communication with the manifold hub and adapted to slideably receive a ductal introducer; including the steps of inserting the introducer into the internal passageway of a breast duct; and advancing the catheter into the breast duct.

In still another aspect of the present invention, a method for obtaining cellular material from a human breast milk duct using the device comprising a manifold hub comprising a plurality of port openings in fluid communication with an interior of said manifold hub, at least two of said openings being in fluid communication with a pair of elongated channels that extend through a portion of said manifold hub and a catheter extending from the manifold hub and a sheath which extends internally through both the hub and the catheter, comprising an elongated internal lumen extending substantially parallel to a longitudinal axis of said sheath, said lumen being in fluid communication with said manifold hub and adapted to slideably receive a ductal introducer therein and said sheath extends beyond the distal tip of said catheter; including the steps of inserting the introducer into the internal passageway of a breast duct advancing the sheath into the breast duct and advancing the catheter into the breast duct.

The above and other aspects, features and advantages of the present invention will be readily apparent and fully understood from the following detailed description illustrative embodiments in conjunction with the accompanying drawings, which are included by way of example, and not by way of limitation with regard to the claimed invention.

### BRIEF DESCRIPTION OF THE DRAWINGS

Figure 15 is a schematic representation of a first ductal access device in accordance with one or more aspects of the present invention;

Figure 16 is a perspective view of a catheter in accordance with one or more aspects of the present invention;

Figure 17 is a partial schematic side view of a catheter in accordance with one or more aspects of the present invention;

Figure 18 is a schematic side view of a catheter with a removable tip in accordance with one or more aspects of the present invention;

Figure 19 is a schematic representation of a second ductal access device in accordance with one or more aspects of the present invention

Figure 20 is a sectional view of a catheter shown in FIG. 19 taken along an central axis to illustrate an interior construction and components in accordance with one or more aspects of the present invention;

Figure 21 is a perspective view of a catheter in accordance with one or more aspects of the present invention;

Figure 22 is a sectional view of a catheter shown in FIG. 21 taken along an central axis to illustrate an interior construction and components in accordance with one or more aspects of the present invention;

Figure 22A is a sectional view similar to FIG. 22 showing an alterative embodiment of a catheter in accordance with one or more aspects of the present invention;

Figures 23A-24C are diagrams illustrating a method of accessing a breast duct which a catheter of FIG. 16;

Figures 24A-24C are diagrams illustrating a method of accessing a breast duct which a catheter of FIG. 21;

Figure 25 is a table illustrating exemplary parametric values and materials for guide wire and introducers that may be implemented with aspects of the present invention;

Figure 26 is a table illustrating exemplary parametric values and materials for cannula that may be implemented with aspects of the present invention; and

Figure 27 is a partial schematic side view of a catheter in accordance with one or more aspects of the present invention.

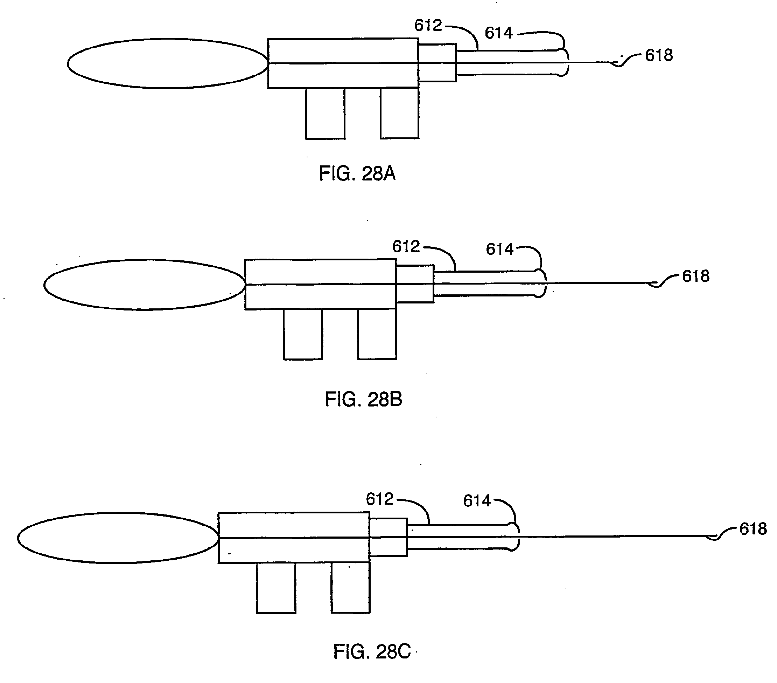

Figure 28 is a schematic representation of an alternative ductal access device in accordance with one or more aspects of the present invention.

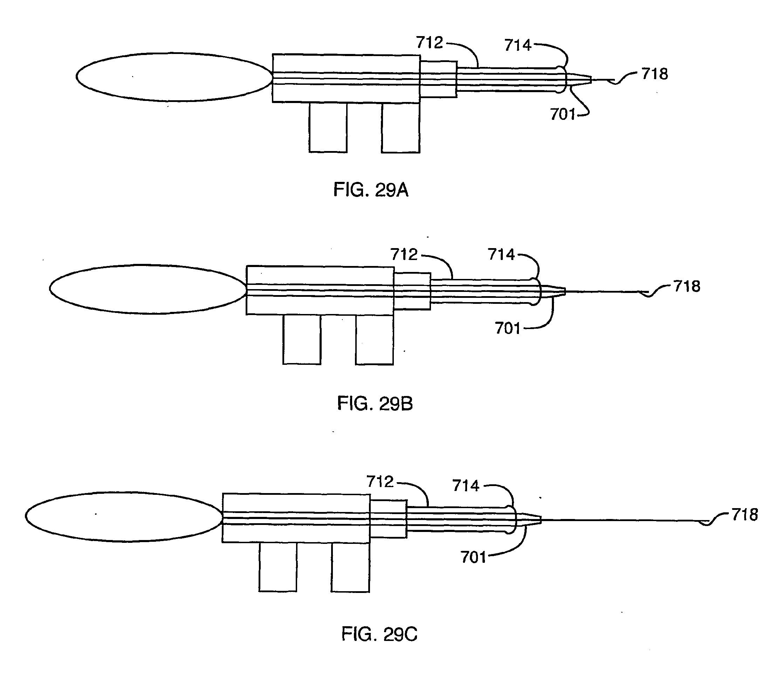

Figure 29 is a schematic representation of an alternative ductal access device in accordance with one or more aspects of the present invention.

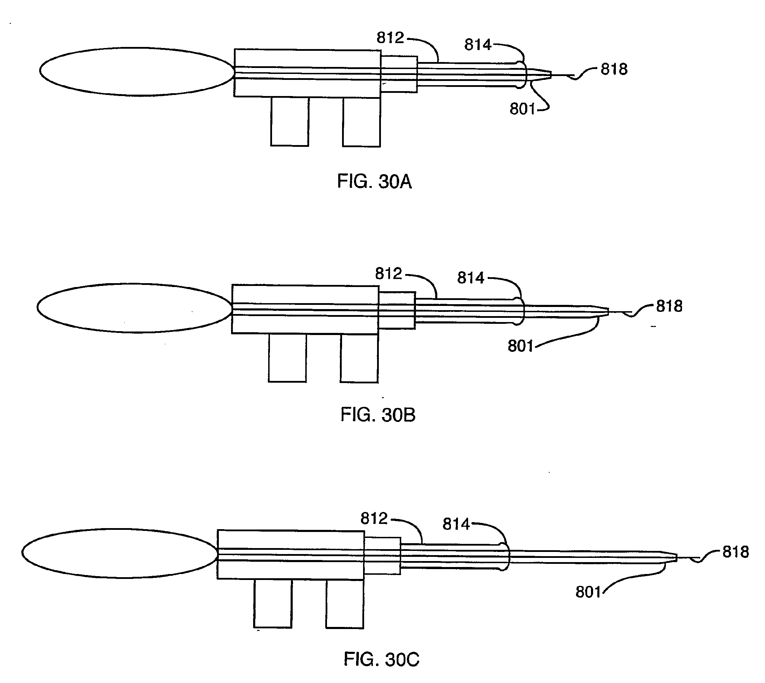

Figure 30 is a schematic representation of an alternative ductal access device in accordance with one or more aspects of the present invention.

### DETAILED DESCRIPTION OF ILLUSTRATIVE EMBODIMENTS

Figures 15-17 illustrate preferred embodiments of an inventive access device 10 for accessing a body orifice, such as a breast duct. The access device 10 comprises a catheter 12 having a distal end 14 with a centrally disposed single lumen 16 which extends the length of the catheter 12, and an elongated distal tip portion 17 which extends beyond the distal end 14 a predetermined distance. The center axis 21 of distal tip portion 17 extends substantially parallel to the center axis 19 of single lumen 16 and is disposed within the wall of the catheter. In such an exemplary construction, the distal tip portion 17 is disposed "off-axis" or "off-center" from center axis 19 of lumen 16. The off-axis construction of distal tip portion 17 advantageously maintains the open inner diameter of the catheter for maximum fluid flow in and out of the breast duct. Further, the off axis configuration allows substantially improved flow because it does not to interfere with the introduction or removal of fluid and collected samples so not to compromise the ability to retrieve a meaningful sample while still being in direction of the catheter and lumen. The elongated configuration of the distal tip portion 17 acts as an effective guide while not interfering with fluid infusion or compromising ductal fluid sample collection. Distal tip portion 17 also provides ease of insertion of catheter 12 in the ductal orifice. In one construction, distal tip portion 17 is flexible in nature so as to allow the catheter to traverse the potentially tortuous and/or angled geometry of the human breast duct. Further, distal tip portion 17 distends the ductal orifice to reduce the associated pain upon insertion of the catheter therein.

Catheter 12 has dimensions which permit introduction of the distal end 14 through a ductal orifice and positioning a distal end thereof distal to the ductal sphincter of a human breast. In one construction, the catheter 12 has a maximum outer diameter of approximately 0.039 inches so as to cannulate the ductal orifices of the breast. Nevertheless, other dimensions are possible for the outer diameter (e.g., approximately 0.025 to 0.039 inches). Single lumen 16 with an internal ID of approximately 0.025 inches accesses the breast duct and through which fluid is infused, and from which ductal fluid samples including ductal epithelial cells are collected or drawn up out of the duct. Distal tip portion 17 has dimensions which permit introduction through a ductal orifice so as to lead the catheter 12 into the breast duct. In one exemplary construction, distal tip portion 17 has a smaller diameter than the catheter 12 to allow ease, of insertion into the breast duct. Distal tip portion 17 may have a diameter of approximately 0.010 inches, although other dimensions are possible (e.g., 0.008 to 0.012 inches). In an alternative construction, the distal tip portion 17 may be flexible.

Referring to Figures 16 and 17, the distal tip portion 17 may have a tapered configuration being largest at the distal end 14 of the lumen 16 (i.e., proximal end of distal tip 17) extending therefrom to be smallest at the distal end 18. In use, the taper and flexibility of the distal tip portion 17 guides the catheter for intraductal movement. Shown in Figure 17, the transition between the distal tip portion 17 and catheter 12 includes a beveled surface 23. This beveled surface 23 provides a smooth transition between distal tip portion 17 of catheter 12, which makes the catheter 12 easy to introduce into the ductal opening after the insertion of the tip portion 17 into the breast duct. Hence, the distal tip portion 17 may hold the duct opening in position so that the catheter 12 enters with relative ease. As may be appreciated by one of ordinary skill in the art, the beveled surface 23 reduces the stress level on the tissue being penetrated and spread open by the distal end 14 of the catheter 12. In contrast to traditional catheters, discomfort to the patient is greatly reduced with the access device 10 of the present invention. The edges of the catheter at the distal end and the end 18 of the distal tip 17 may include an atraumatic configuration. In one atraumatic configuration, the edges and end 18 are rounded to reduce friction and eliminate surfaces that could puncture or otherwise injure the duct. Thus, this construction reduces localized trauma to the tissue verses non-atraumatic designs.

Referring to Figure 15, a multiport hub 22 is coupled to the proximal end 15 of catheter 12. In a preferred construction, hub 22 includes transparent material so that the user may visualize the flow to and from the breast duct during a lavage procedure. In another construction, hub 22 has a low profile so as to reduce the torque on the breast nipple after insertion of catheter 12. This overcomes the excessive generated torque on the breast nipple known to cause obstruction of ductal fluid due to compression of the tissue. Thus, improved collection of ductal cellular material is provided.

Hub 22 is attached to a tubing set 25 which comprises an infusion tube 24 from which fluid is infused into lumen 16 through hub 22 and a collection tube 26 from which fluid is collected from lumen 16 via hub 22. Infusion tube 24 and collection tube 26 are attached to hub 22 in a conventional manner to allow fluid flow. In one construction, infusion tube 24 and collection tube 26 are translucent and have standard luer connections at their distal ends that mate with ports on the hub 22 and receive fluids. The proximal ends of the tubes 24, 26 also include standard luer connection that a practitioner or attendant to manage the various ductal fluids using an appropriate syringe with a standard male luer. If desired, tubes 24, 26 may be labeled to indicate the inflow and outflow paths, e.g. infusion or collection functions.

In a further construction, an optional pinch clamp or other flow control device (not shown) may be disposed on the outflow tube, collection tube 26. In use, the clamp closes the collection tube 24 to prevent fluid leakage from the tubing during fluid infusion into a cannulated breast duct. In one construction, hub 22 includes an ergonomic handle 27 for the user to grasp during a ductal lavage procedure. It should be recognized that a fluid used in the hub 22 and catheter 12 may be virtually any appropriate fluid for the ductal lavage procedure. Exemplary ductal wash fluids which may be used with hub 22 includes but is not limited to saline, phosphate buffered saline, a nonabsorbable fluid, an isotonic solution, an osmotic solution, a hypotonic solution, and a hypertonic solution. Nevertheless, an appropriate therapeutic fluid may be provided by way of the ductal access devices described herein. Alternatively, the fluids could include diagnostic or therapeutic fluids carrying diagnostic and/or therapeutic agents.

In an alternative construction depicted in Figures 19 and 20, an inventive ductal access system 100, includes a catheter 112 having a distal end 114 with a centrally disposed single lumen 116 which extends the length of the catheter 112. Catheter 112 includes a tubular introducer pathway 120 adapted to slideably receive a ductal introducer 118 therein. Long axis 121 of pathway 120 is offset but substantially parallel to long axis 119 of lumen 116. For ease of explanation, as used herein the term "introducer" is defined to include guidewires, dilators, stylettes or portion of any of these that may be inserted within a passageway of a catheter or into the ductal orifice. Ductal introducer 118 is provided for ease of varying the length of tip portion 117 so as to improve intraductal travel of the catheter 112 within the breast duct and to reduce risk of puncturing the ductal wall and rupturing the breast duct. By removably inserting the ductal introducer 118 into the axis 121 of pathway 120, the user is able to choose an introducer having a desired stiffness for the particular patient. Additionally, this enables the practitioner or attendant to change the flexibility and stiffness of the introducer 118. The stiffness and flexibility may be a function of a material property and/or cross-sectional shape. One example of a material property that relates to flexibility is the modulus of elasticity. In another example, the cross-sectional shape of the introducer 118 may be circular, elliptical, oval or other shapes that provide predetermined stiffness in one or more directions. One of skill in the art would recognize these various shapes relate to a section modulus of the introducer 118. Therefore, it should be recognized that introducer pathway 120 may be virtually any appropriate cross-sectional shape to meet the cross-sectional shape of the introducer 118.

Ductal introducer 118 includes a distal end 122, which enters the breast duct. The opposing proximal end 124 of ductal introducer 118 includes a handle 126. Handle 126 provides ease of operation so that a user may grasp and manipulate the introducer 118 to access and navigate the breast duct anatomy. Ductal access device 100 includes a hub 22 which has similar construction and components as hub 22 shown in Figure 15. In hub 22, the introducer pathway 120 extends through so that the introducer 118 may be inserted. The pathway may be a tube extending through the exterior of the hub 22 or the tube may be located on the outer surface of the hub. An adjustable tip portion 117 of introducer 118 is defined from the distal end 122 of ductal introducer 118 to the distal end portion 114 of catheter 112. In this configuration, the length of the tip portion 117 is selectively adjustable by the user for accessing and traversing the breast duct. In one aspect, ductal introducer 118 may be embodied as a guide wire, which is easily inserted into pathway 120 and into the breast duct. In one construction shown in Figure 20, the center axis 121 of introducer pathway 120 extends substantially parallel to the center axis 119 of single lumen 116. As discussed above, in such an exemplary construction, the introducer pathway 120 is disposed "off-axis" or "off-center" from center axis 119 of lumen 116. Thus, ductal introducer 118 with the off-axis construction of catheter 112 provides similar benefits as the ductal access device 10.

The introducers described herein may be formed of appropriate cross-sectional shapes and various materials for medical use. The shapes and materials enable desired rigidity and flexibility for intraductal movement. Alternative materials for the introducer 118 may include but are not limited to: Stainless Steel Wire; FEP; PTFE; PEEK; and PVDF and PEBAX. Nevertheless, other appropriate materials may be employed. Specific dimensional value introducers are shown in Figure 25, but are provided by way of example. Alternate coatings for the introducer and/or catheter may include but are not limited to: hydromer coating; STS SLIP-COAT; MDX; silicone dry; silicone lubricant; PTFE coatings. The specific thickness of the coatings and application may be readily determined by one of ordinary skill in the art.

The introducer may be made of metal or plastics and may have a tapered and/or an atraumatic tip for gently probing and accessing a breast duct. In one example, the introducer may be constructed of a superelastic or shape memory material. As used herein, the term "superelastic shape memory material" refers to a class of metal alloys that have a stress-induced phase change or temperate from austenite to martensite and upon stress release, the material springs back to this original phase and shape. The material structure of a superelastic shape memory material regarding austenite and martensite is well known to one of ordinary skill in the metallurgy art. A NiTi material or NiTi alloy may be used as an alloy material for the introducer. As used herein, a NiTi superelastic shape memory material refers to an alloy that is an intermetallic compound of nickel and titanium having nearly equal mixtures as measured by weight. One composition of a NiTi superelastic shape memory material generally has a greater percentage of nickel by weight than titanium, such as 51 %-56% of nickel, and preferably 54-55% nickel. The specific percentages of nickel and titanium may be adjusted by one of ordinary skill in the art.

It is not necessary for the introducer to be composed of a material that has shape memory characteristics. The introducer may also be formed of a stiff material such as a metal wire or a flexible material such as plastic. In an embodiment of the invention, the combined introducer may be formed of Nitinol due to its flexibility, durability, and biocompatibility. In an alternative embodiment, the introducer may be formed of multiple materials or the same materials having different stiffnesses. As a result, the introducer may have sections that are more flexible than adjacent sections of the same introducer. As a result, for example, the introducer, may have a first, stiff portion for guiding the introducer within the ductal lumen and a second, flexible portion that allows the introducer to conform to the shape of the ductal lumen or lumen branch into which it is introduced. In any of the above-discussed embodiments, the introducer may be coated with a liquid or dry lubricant material that reduces the friction between the introducer and the breast duct during the introduction and advancement of the introducer in the duct.

Figures 21 and 22 illustrate an alternative construction of an inventive catheter body 212 having a distal end 214 with a centrally (about axis 219) disposed single lumen 216 that traverses the length of the catheter body. In an off-axis configuration, an elongated flexible tip 217 extends beyond the distal end 214 along axis 221. The tip 217 is configured to receive a removable introducer 218. This feature is achieved in that the tip 217 and catheter body 212 include an internal passageway 220 adapted to slideably receive the introducer 218. The passageway 220 may be tubular at the tip 217 and is located in the walls of catheter body 212. Passageway 220 has a closed distal end 122, which is in the initial portion that enters the breast duct. The passageway 220 is opened at the proximal end 211. The closed end feature enables the practitioner or user to adjust the flexibility of the distal 217 during cannulation of the breast duct but prevent fluid from entering the passageway 220. Further, the closed end feature allows the introducer 218 to be removed and another introducer inserted therein, when the catheter 212 is positioned within the breast duct. Thus, introducer 218 may be removed or inserted into the tip 217 to selectively adjust the flexibility of tip 217. This allows the user to insert the introducer 218 making the flexible tip more rigid for insertion into the duct, or remove the stylette making the tip more flexible so the tip may transverse tortuous ductal geometry. In this configuration, passageway 220 extends the length of the lumen 216 and is parallel thereto. Advantageously, an aspect of the present invention provides an option to change to tip flexibility which greatly aids the cannulation of the breast duct. The material and thickness of flexible tip 217 may be chosen to achieve a desired stiffness in combination with the flexure characteristics of introducer 218. The closed distal end 222 is rounded to reduce friction between the ductal tissue and tip 217 and prevent the distal end 222 from puncturing duct.

Figure 22A illustrates an alternative catheter body 312, which has similar construction as catheter body 212 except that a passageway 320 is in the distal tip 317 and not in the walls of the catheter body 312. This allows the user to insert the introducer 218 through the lumen 316 and into the proximal end 311 of the passageway 320. Flexible elongated distal tip 317 includes a closed distal end 322 extending to the distal end 314 of catheter body 312.

In the example of the embodiments shown Figures 22 and 22A, the catheter 212, 312 may be provided with a plurality of introducers each of which have a different stiffness characteristic or property. Introducers are provided depending on the portion of duct being accessed. One introducer may be used to introduce the distal tip 217, 317 past the ductal sphincter. Another introducer may be provided to enter a desired branch of breast duct. Yet another introducer, if desired, may be used to enter a final portion of branch. This allows practitioner to design stiffness of tip 217, 317 to match needs presented by different portions of the duct.

An introducer may be designated with a stiffness value to provide certain a stiffness property relative to the other introducers. Purely by way of example, a practitioner may be provided with an introducer having a stiffness value of two, which is designed to be rigid so as to allow penetrating the ductal opening. Another introducer may be provided having a stiffness value four which would be less stiff than a value of two. Yet another introducer may have a value of eight, which would be less stiff than a value of four. In this example, a practitioner is enabled to access the breast duct with a rigid introducer inserted within the passageway 220, 320. The practitioner, while the catheter 212, 312 resides within the breast duct, may remove the rigid introducer and an insert another introducer to continue to advance within the breast duct. Thus, the practitioner is allowed to enter the breast with the catheters 212, 312 without removing the catheter once the breast duct is accessed. Advantageously, catheters 212, 312 allow the practitioner to be more efficient in the ductal access procedure because steps are eliminated or substantially reduced over conventional procedures, e.g., the use of dilators be significantly reduced. Further, patient comfort is increased because less traumatic movement is provided to the breast.

In another construction shown in Figure 18, a catheter 412 has a distal end 414 with a single lumen 416 disposed about a central axis 419, which extends the length of the catheter, and an elongated distal, tip portion 417, which extends beyond the distal end 414. Catheter 412 has a bevel face and the tip portion 417 includes an extensible introducer 418 formed as a stylette portion. Stylette portion 418 comprises a distal end 422 extending from a distal opening 423 of a transition portion 425 of the lumen 416. Transition portion 425 includes a passageway configured to receive a stylette so that the distal end 422 exits the opening 433. The stylette portion 418 may be made of a flexible metal, plastic material or other materials described above. The stylette portion 418 may be fixed in position and serve as a flexible tip, or it may be removed after insertion of the catheter into the duct. The stylette portion 418 may be removed and replaced with a shorter stylette portion so as to shorten the tip length. Alternatively, the stylette portion 418 may have a longer length. In this manner, the tip length may be adjusted to accommodate the particular duct geometry.

Figures 27 illustrates an alternative construction of an inventive catheter body 512 having a distal end 514 with a centrally (about axis 519) disposed single lumen 516 that traverses the length of the catheter body. In an off axis configuration, an elongated tip 517 extends beyond the distal end 514 along axis 521. The elongated tip 517 may have a multi-flexion configuration that has separate regions of different flexions that each corresponds to the flexibility, or lack thereof, needed to access a respective portion of a breast duct. This multi-flexion zone configuration provides adaptability for a practitioner to reduce steps for accessing a breast and/or customize the access of the duct to increase patient comfort. In one exemplary example, the elongated tip 517 may have three flex zones to accommodate to access a breast duct. A first flexion zone 540 may extend from the distal end to a first intermediate position 542 along the length of the elongated tip. The first flexion zone 540 maybe substantially rigid for entering into the breast duct to overcome the resistance force provided by the tissue at the ductal opening. An adjacent second flexion zone 544 may extend to another intermediate position away from first intermediate position 542 along the length of the elongated tip 517. The second flexion zone 544 may be less rigid than a first flex zone 544 so as to allow the elongated tip 517 to traverse the breast duct geometry. A third flex zone 548 may be provided adjacent to the second flexion zone 544. The third flexion zone 548 may be more flexible than the second flexion zone. In a specific example, the dimensions of the first flexion zone maybe 3 cm from the distal end; second flexion zone may have a length of 4 to 8 cm; and the third flexion own may have a length of 2 to 3 cm. Nevertheless, the length of the zones maybe configured as desired by the practitioner.

Figures 23A-23C are schematic diagrams illustrating a method of accessing a breast duct by way of a catheter 12 of Figure 16. Referring to Figure 23A, the catheter 12 is prepared to be introduced into a ductal orifice so that a distal end thereof will be positioned within the duct beyond the ductal sphincter during ductal ravage, introduction of diagnostic materials and/or introduction of therapeutic materials. As shown in Figure 23B, the distal end 14 of the catheter is outside of the duct. The catheter has a flexible distal tip 17, which provides ease of insertion of catheter 12 in the ductal orifice. Further, the flexible distal tip 17 is inserted into the duct so as to follow the intraductal geometry but not cause damage to the duct. As shown in Figure 23C, the distal end 14 has entered the duct. The distal tip 17 continues to follow the intraductal geometry to guide the catheter 12 therethrough. Further, the catheter 12 is more rigid than the distal tip 17 so as to straighten the duct for infusion and collection. Although catheter 12 is described with reference to Figures 23A-23C, other catheters 112, 212, 312, 412, and 512 may be used in a similar fashion.

Figures 24A-24C are schematic diagrams illustrating a method of accessing a breast duct with a catheter body 212 of Figure 21. Referring to Figure 24, the catheter body 212 is prepared to be introduced into a ductal orifice. The practitioner may choose an introducer 218 of a desired flexibility or rigidity. For example, the introducer 218 of different rigidity properties may be inserted for adjusting to a desired tip characteristic during insertion of the distal tip 217 into the breast duct. This arrangement creates a composite insertion member for accessing the breast duct. The desired tip characteristic may relate to the stiffness and flexibility, which may be functions of a material property or a cross-sectional shape. One of skill in the art would recognize that the composite insertion member may have various shapes and material properties to vary the section modulus.

In particular, the tip 217 may be selectively made rigid to penetrate the duct orifice. In this configuration, the length of the catheter 212 and distal tip 217 is relatively rigid. In Figure 24B, the introducer 218, which is rigid or somewhat rigid, has been removed from the distal tip 217 while the catheter 212 is inserted in the duct. In this removed configuration, the catheter 212 may flex and bend through the intraductal geometry. If desired, an introducer 218, which is less stiff, may be inserted into the distal tip 217. With or without an introducer 218, the catheter 212 is advanced further into the duct. In Figure 24C, once a desired position is reached within the duct, a rigid or somewhat rigid introducer 218 may be inserted and advanced into the catheter 212 to straighten the duct for infusion and collection. In a shape memory embodiment, the stiffness or flexibility of introducer 218 may be adjusted in response to the addition of stimulus or removal of a stimulus without removal from the catheter 212. While not shown, it should be recognized that distal tip 217 could be inserted into the ductal orifice without the need of an introducer 218. The introducer of an appropriate stiffness may be inserted into the distal tip 217 during or after intraductal entry of the catheter 212. Although catheter 212 is described with reference to Figure 24A-24C, other catheters 112, 312, 412 and 512 may be used in a similar fashion.

As illustrated in Figures 19 and 20, the catheter 112 may be tapered along its length to make a smooth transition with a received introducer 118 so that a perceptible transition between the catheter 112 and the introducer 118 that would cause any pain to the patient is not formed and felt by the patient. The catheter 112 may also include an atraumatic distal tip portion 114 at its distal end. The distal tip portion 114 may be tapered, contoured and/or rounded so as to produce an atraumatic tip that will reduce or eliminate trauma to the duct upon entry through the ductal orifice and introduction into the ductal lumen past the ductal sphincter. The distal tip portion 114 may also reduce or eliminate trauma upon withdrawal of the catheter 112 from the duct after the medical procedure, such as ductal lavage or the infusion of a diagnostic and/or treatment agent, has been completed. The tip portion 114 may be composed of a soft polymeric material, e.g. including polyvinyl chloride, polyethers, polyamides, polyethylenes, polyurethanes, copolymers thereof and the like. The tip portion 114 may have a diameter in the range from about 0.012 inches (about 0.031 mm) to about 0.020 inches (about 0.051 mm). In an embodiment, the tip portion 114 has a diameter in the range from about 0.014 inches (about 0.036 mm) to about 0.018 inches (about 0.046 mm). The length of the tip portion 114 (extending from the distal end of the distal portion of the catheter toward the proximal end of the catheter) may be in a range from about 0.10 inch (about 0.25 cm) to about 1.0 inch (about 2.5 cm), more typically in the range from about 0.20 inch (about 0.50 cm) to about 0.70 inch (about 1.8 cm).

Figures 28A, 28B, and 28C depict three different formats of the distal end of an introducer 618. Referring to Figure 28A, a preferred embodiment of the device is shown in a catheter 612 comprising an elongated internal lumen extending substantially parallel to a longitudinal axis of the catheter and adapted to slideably receive a ductal introducer 618. The distal tip portion 614 of the catheter 612 may be contoured and/or rounded so as to produce an atraumatic tip to form an opening through which the ductal introducer 618 may pass. Such a contour will reduce or eliminate trauma to the duct upon entry through the ductal orifice and introduction into the ductal lumen past the ductal sphincter. The distal tip portion 614 may also reduce or eliminate trauma upon withdrawal of the catheter 612 from the duct after the medical procedure, such as ductal lavage or the infusion of a diagnostic and/or treatment agent, has been completed. The tip portion 614 may be composed of a soft polymeric material, e.g. including polyvinyl chloride, polyethers, polyamides, polyethylenes, polyurethanes, copolymers thereof and the like. The ductal introducer 618 which passes through the internal lumen of the catheter 612, may extend past the distal tip of the catheter 612 and is of sufficient length such that it may penetrate through the ductal orifice and into the ductal lumen past the ductal sphincter prior to the distal tip of the catheter 614 passes through the ductal orifice. The extended length of the probe introducer 618 provides ease of use as well as increasing patient comfort during the ductal lavage procedure. The extended probe introducer 618 may be used to identify the ductal path as well as to introduce the catheter 612 into the ductal orifice. Previously, the ductal lavage process used a separate device (dilator) to identify the ductal path prior to the insertion of the catheter. The extra device and extra procedural step resulted in additional cost, time, as well as an increase in patient anxiety or discomfort due to an additional insertion step to relocate the ductal orifice and ductal path. The increased length of the probe introducer allows the practitioner to locate the ductal path prior to catheter insertion. The catheter may then be inserted into the ductal path by slipping it over the probe introducer without withdrawing the introducer. In an embodiment of the present invention, the length of the ductal introducer that extends beyond the distal tip of the catheter shown in Figure 28A is at least 0.025 inches.

In Figure 28B, the introducer 618 extends beyond the distal tip of the catheter 614 at least 0.0492 inches.

In Figure 28C, the introducer 618 extends beyond the distal tip of the catheter 614 at least 0.0984 inches.

Figures. 29A, 29B, and 29C depict three different formats of the distal end of a catheter 712. Referring to Figure 29A, a preferred embodiment of the device is shown in a catheter 712 comprising an elongated internal lumen extending substantially parallel to a longitudinal axis of the catheter and adapted to slideably receive a ductal introducer 718. The distal tip portion 714 of the catheter 712 may be contoured and/or rounded so as to produce an atraumatic tip to form an opening through which the ductal introducer 718 may pass. Such a contour will reduce or eliminate trauma to the duct upon entry through the ductal orifice and introduction into the ductal lumen past the ductal sphincter. The present invention also provides a step transition from the distal tip portion 714 of the catheter to a probe introducer sheath 701. The distal tip portion of the sheath 701 may be contoured and/or rounded so as to produce an atraumatic tip to form an opening through which the ductal introducer 718 may pass. Such a contour will reduce or eliminate trauma to the duct upon entry through the ductal orifice and introduction into the ductal lumen past the ductal sphincter. The distal tip portion may also reduce or eliminate trauma upon withdrawal of the catheter 712 from the duct after the medical procedure, such as ductal lavage or the infusion of a diagnostic and/or treatment agent, has been completed. The tip portion may be composed of a soft polymeric material, e.g. including polyvinyl chloride, polyethers, polyamides, polyethylenes, polyurethanes, copolymers thereof and the like. The ductal introducer 718 that passes through the internal lumen of the catheter 712 and the sheath 701, may extend past the distal tip of the sheath and is of sufficient length such that it may penetrate through the ductal orifice and into the ductal lumen past the ductal sphincter prior to the distal tip of the sheath passes through the ductal orifice. The length of the ductal introducer that extends beyond the distal tip of the sheath shown in Figure 29A is at least 6.5 millimeters.

In Figure 29B, the introducer 718 extends beyond the distal tip of the sheath 701 at least 12.5 millimeters.

In Figure 29C, the introducer 718 extends beyond the distal tip of the sheath 701 at least 25.5 millimeters.

Figures 30A, 30B, and 30C depict three different formats of the distal end of a catheter 812. Referring to Figure 30A, a preferred embodiment of the device is shown in a catheter 812 comprising an elongated internal lumen extending substantially parallel to a longitudinal axis of the catheter and adapted to slideably receive a ductal introducer 818. The distal tip portion 814 of the catheter 812 may be contoured and/or rounded so as to produce an atraumatic tip to form an opening through which the ductal introducer 818 may pass. Such a contour will reduce or eliminate trauma to the duct upon entry through the ductal orifice and introduction into the ductal lumen past the ductal sphincter. The present invention also provides a step transition from the distal tip portion 814 of the catheter to a probe introducer sheath 801. The distal tip portion of the sheath 801 may be contoured and/or rounded so as to produce an atraumatic tip to form an opening through which the ductal introducer 818 may pass. Such a contour will reduce or eliminate trauma to the duct upon entry through the ductal orifice and introduction into the ductal lumen past the ductal sphincter. The distal tip portion may also reduce or eliminate trauma upon withdrawal of the catheter 812 from the duct after the medical procedure, such as ductal lavage or the infusion of a diagnostic and/or treatment agent, has been completed. The tip portion may be composed of a soft polymeric material, e.g. including polyvinyl chloride, polyethers, polyamides, polyethylenes, polyurethanes, copolymers thereof and the like. The probe introducer 801 sheath may extend beyond the distal end of the catheter 812. The extended probe introducer sheath facilitates a smooth transition and entry of the catheter 812 into the ductal orifice. The length of the probe introducer sheath 801 that extends beyond the distal tip of the catheter 812 shown in FIG 16A is at least 1.5 millimeters.

In Figure 30B, the probe introducer sheath 801 extends beyond the distal tip of the catheter 812 at least 6.0 millimeters.

In Figure 30C, the probe introducer sheath 801 extends beyond the distal tip of the catheter 812 at least 18.5 millimeters.

In another embodiment, the probe introducer sheath 801 and the introducer 818, may be combined into a single unit (not shown). The combined sheath/introducer may be formed of a stiff material such as a metal wire or a flexible material such as plastic. In an embodiment of the invention, the combined sheath/introducer may be formed of Nitinol due to its flexibility, durability, and biocompatibility. The combined sheath/introducer may be constructed from a Nitinol wire that is ground to include a tapered small diameter tip. In an alternative embodiment, the combined sheath/introducer may be formed of multiple materials or the same materials having different stiffnesses. As a result, the combined sheath/introducer may have sections that are more flexible than adjacent sections of the same combined sheath/introducer. As a result, for example, the combined sheath/introducer may have a first, stiff portion for guiding the combined sheath/introducer within the ductal lumen and a second, flexible portion that allows the combined sheath/introducer to conform to the shape of the ductal lumen or lumen branch into which it is introduced. In any of the above-discussed embodiments, the combined sheath/introducer may be coated with a liquid or dry lubricant material that reduces the friction between the combined sheath/introducer and the breast duct during the introduction and advancement of the combined sheath/introducer in the duct.

The combined sheath/introducer may be made of metal or plastics, including shape memory metals and plastics, and may have a tapered and/or an atraumatic tip for gently probing and accessing a breast duct. Preferably, a tapered tip will extend distally of the catheter during the introduction of the catheter into the breast duct. After access of the duct is complete, the catheter may be slipped over the combined sheath/introducer and positioned at a location distal to the ductal sphincter.

The catheters as described herein may be constructed of a wide range of appropriate materials for medical use. In a preferred construction, the catheter is formed with PTFE and coated with STS Slipcoat to reduce friction on the device during placement in the breast duct. In lieu of PTFE, alternate materials for the catheter may include but are not limited to: polycarbonate; stainless steel (300 Series); polymide; FEP; PEEK; polyethylene; and PEBAX. Specific dimensional values of catheters are shown in Figure 26, but are provided by way of example. Nevertheless, other appropriate materials and dimensional values may be employed.

The hub 22 housing may be molded from a polycarbonate. The tubing set 25 may be made of PVC with luer connectors made of polycarbonate. The tubing set may be made of a variety of conventional materials including but not limited to: silicone; low-density polyethylene; PVC; tygon; or polypropylene.

In one manufacturing process, the catheters according to one or more aspects of the present invention may be constructed by using an extrusion process. After the process, the distal end of catheter is shaped by molding and/or cutting. An optional, bevel may be is cut to provide the transition between the distal tip portion and the catheter. The edges of the catheter may be rounded using an RF tipping process known to one of ordinary skill in the art. The catheter may be bonded to the hub using a desired medical grade UV adhesive. In turn, the tubes may be bonded to the hub and to the luer connector using UV adhesive as well. If desired, a pinch clamp may be placed on the outflow tube.

The present invention provides a method for obtaining cellular material from a human breast milk duct includes introducing a ductal access device such as devices 12, 112, 212, 312, 412, and 512 having at least one lumen into a duct. A wash fluid may be introduced through the access device internal lumen into the milk duct. In the method, a volume of at least 2 ml of wash fluid may be present within the duct for a preselected time, and then at least a portion of the wash fluid is collected from the duct through the lumen of the access device. The method preferably further comprises massaging and squeezing the breast tissue after introducing the wash fluid but prior to and/or during collecting a portion of the wash fluid. Introducing the ductal access device typically comprises positioning a distal end of the catheter distal to the ductal sphincter in the breast duct. The access device preferably includes only a single lumen that extends into the duct. The preselected time may be less than one second, but will usually be in the range from one second to one hour.

Accordingly, there are any number of alternative combinations for defining the invention, which incorporate one or more elements from the specification, including the description, claims, and drawings, in various combinations or sub combinations. It will be apparent to those skilled in the relevant technology, in light of the present specification, that alternate combinations of aspects of the invention, either alone or in combination with one or more elements or steps defined herein, may be utilized as modifications or alterations of the invention or as part of the invention. It may be intended that the written description of the invention contained herein covers all such modifications and alterations.

### A METHOD OF OPENING A DUCTAL SPHINCTER USING CONTROLLED FLUID PRESSURE

### SUMMARY OF THE INVENTION

In accordance with one aspect of the present invention, a medical instrument including a ductal access device comprising a low profile manifold hub usable to introduce fluids into a breast duct and collect ductal fluid samples including ductal epithelial cells and clumps of ductal epithelial cells from within a breast duct is provided. The ductal access device also includes an elongated access catheter having a distal end, one lumen and dimensions which permit introduction of the distal end through a ductal orifice and positioning a distal end distal to the ductal sphincter of a human breast. The catheter may include length indicia on its outer surface that permits a user to determine the depth to which the distal end of the catheter has been introduced. The medical instrument may also include at least one spacing member (spacer) for adjustably positioning the manifold hub a desired distance above the surface of the nipple. The spacing member may control the insertion depth of the catheter into the duct. The medical instrument may also include at least one member for anchoring the device to the breast.

In another aspect of the present invention, an apparatus for being introduced and positioned within a breast duct is provided. The apparatus may introduce or remove material within the breast duct. The apparatus comprises a manifold hub including a plurality of port openings in fluid communication with an interior of the manifold hub. At least two of the openings are in fluid communication with a pair of elongated channels that extend through a portion of the manifold hub. The apparatus also includes a catheter that extends from the manifold hub. The catheter is sized and configured for positioning within a breast duct. The apparatus further includes a retractable spacer that may be moved in a direction parallel to the length of the catheter to space the manifold hub a distance above the surface of a nipple.

In yet another aspect of the present invention, a medical device for introducing a fluid into a breast duct is provided. The medical device comprises a catheter that is sized and configured to extend within a breast duct. The catheter includes an internal lumen that extends substantially parallel to a longitudinal axis of the catheter. The medical device also comprises a manifold hub connected to and in fluid communication with the catheter. The manifold hub includes at least four ports in fluid communication within an interior of the manifold hub. At least two of the at least four ports are in fluid communication with channels formed within the manifold hub for receiving a fluid introduction line and a ductal fluid collection line. The manifold hub has a height that extends parallel the longitudinal axis of the catheter and a length that extends perpendicular to the longitudinal axis of the catheter. The length of the manifold hub is greater than the height of the manifold hub.

In still another aspect of the present invention, a ductal access device for aspirating fluid from a breast duct is provided. The ductal access device comprises a manifold hub for receiving a fluid to be introduced into a breast duct and a catheter that extends from the manifold hub. The catheter is sized for positioning within a breast duct and includes a lumen for introducing and receiving fluid within the breast duct. The lumen is in fluid communication with the manifold hub and sized to receive a ductal fluid sample from within the breast duct. The ductal access device also includes a source of negative pressure in fluid communication with the manifold hub such that ductal fluid samples within the manifold hub are drawn to the source of negative pressure for being received within a collection device.

In yet another aspect of the present invention, a method of opening a ductal sphincter using controlled fluid pressure is provided. The method comprises bringing a medical instrument into proximity to a sphincter introducing fluid through said medical instrument such that said fluid comes into contact with said sphincter, applying pressure to said fluid such that an increase in fluid pressure causes said sphincter to open, and passing the medical instrument through to said opened sphincter.

A catheter in accordance with the present invention may have an outer diameter of about 0.01 inch (0.254 mm) to about 0.05 inch (or 1.27 mm). The catheter may have an inner lumen diameter in the range from about 0.007 inch (or 0.178 mm) to about 0.047 inch (or 1.19 mm). The associated manifold hub may further comprise an infusion connector providing a fluid flow path into the lumen of the catheter from an infusion device; and a collection connector providing a fluid outlet path from the lumen of the catheter to a fluid collection device.

A watertight sealing system may be located at a proximal end of the manifold hub. This sealing system may seal around a dilator or other introducer positioned within and extending through the medical instrument. Such a sealing system may include a Touhy-Borst fitting. A dilator or other introducer member(s) for use with the catheter may have an outer diameter of 0.024 inch (or 0.61 mm) to about 0.001 inch. The introducer may also be tapered.

### BRIEF DESCRIPTION OF THE FIGURES

Figure 1 is a perspective view of a medical instrument according to aspects of the present invention;

Figures 2A and 2B are cross sections of alternative embodiments of the medical instrument illustrated in Figure 1;

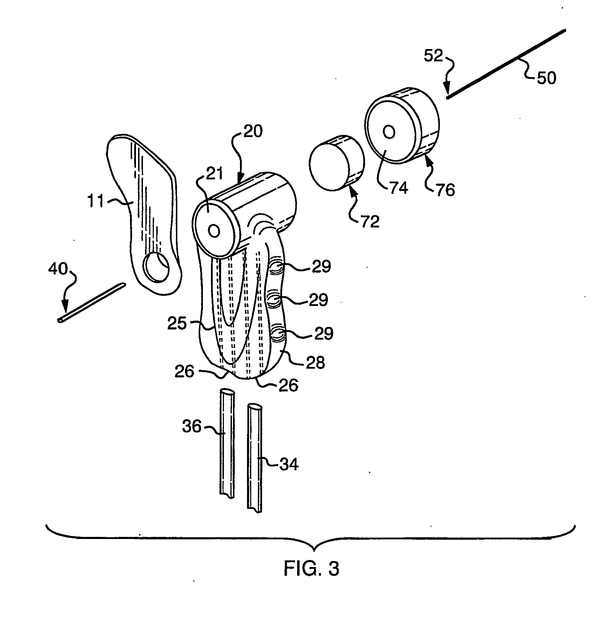

Figure 3 is an exploded perspective view of the medical instrument of Figure 1;

Figure 4 is a top view of the medical instrument of Figure 1 with infusion and collection lines extending between a manifold hub and respective infusion and collection devices;

Figure 5 is a side view of the medical instrument illustrated in Figure 1 carrying infusion and collection lines;

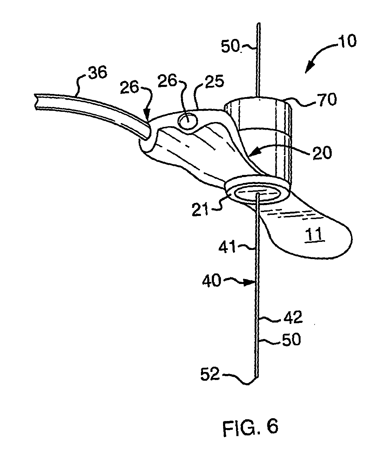

Figure 6 is another perspective view of the medical instrument illustrated in Figure 1;

Figures 7 and 8 illustrate an alternative embodiment of the medical instrument according to aspects of the present invention;

Figures 9-10 illustrate a method for introducing the medical instrument of Figure 1 into a breast duct using a stiff introducer; and

Figures 12-14 illustrate an alternative method for introducing the medical instrument of Figure 1 into a breast duct using a flexible introducer.

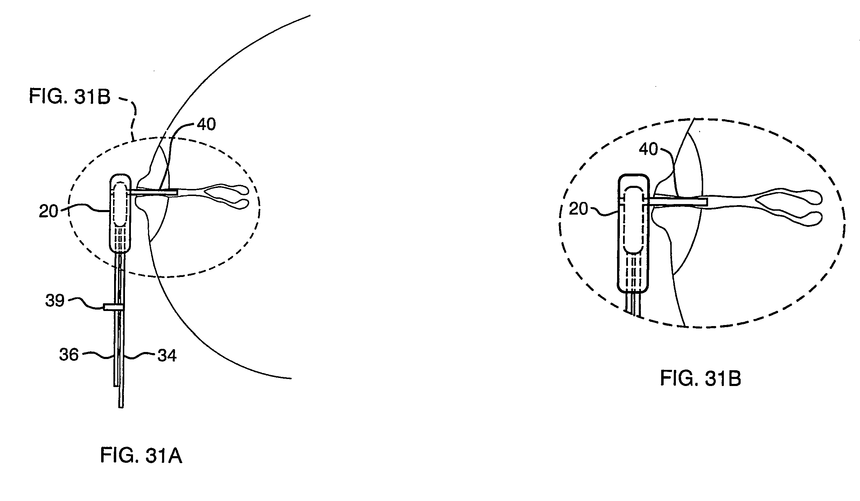

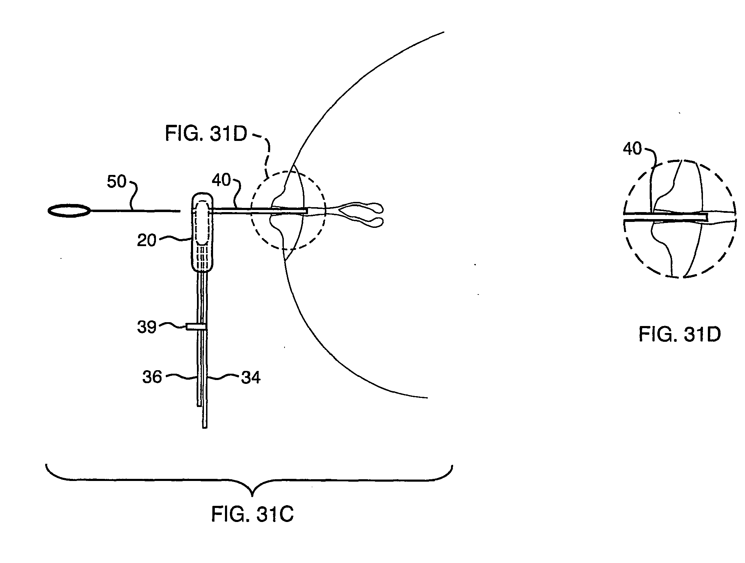

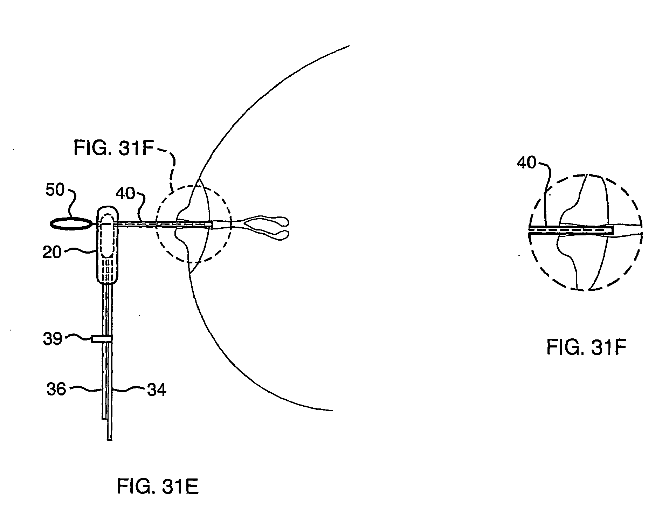

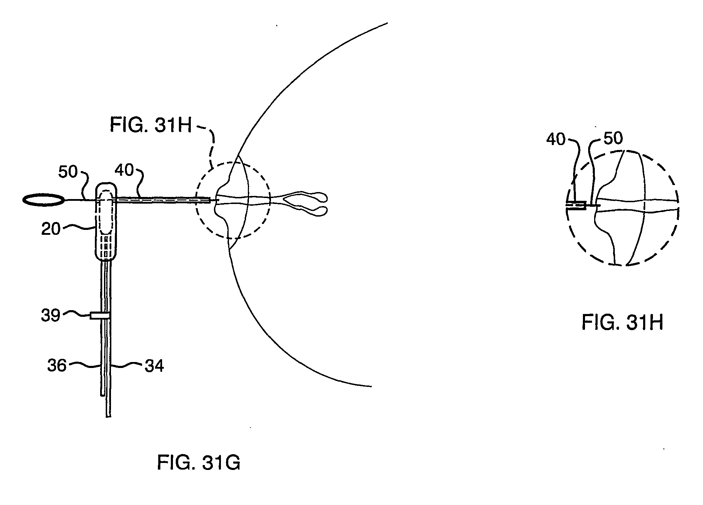

Figures 31A-D illustrates an alternative method of breast microcatheter insertion aided by fluid pressure application.

### DETAILED DESCRIPTION OF THE INVENTION

Figure 1 illustrates an embodiment of a low profile, single lumen medical instrument 10 for performing a medical procedure within a breast duct. As used herein, the phrase "medical procedure" may include preparatory procedures, diagnostic procedures or therapeutic procedures. These procedures could include the steps of delivering material(s) into the breast duct and/or retrieving material(s) from within the breast duct.

In an embodiment, the medical instrument 10 may be used to infuse ductal wash fluid delivered to the manifold hub 20 into the breast duct, and collect or draw up ductal fluid samples, including hundreds of ductal epithelial cells and/or cell clusters of greater than ten cells, from within the breast duct for analysis. In another embodiment, the medical instrument 10 may be used to infuse a diagnostic agent or therapeutic agent into a breast duct. As shown in Figures 1-8, the instrument 10 may also include a member 11 for securing the instrument 10 to a patient. The securing member 11 may have a biocompatible adhesive on one side for contacting and attaching the instrument 10 to the patient. The member 11 is sized to prevent movement of the manifold hub 20 relative to the body of the patient. In an embodiment illustrated in Figures 7 and 8, sections 11A and 11B of the member 11 may be folded onto each other so that the size of the securing member 11 may be adjusted to the patient and the forces created during the procedure. Additionally, the member 11 may be positioned distal a spacer 90 (discussed below).

As illustrated in Figures 1-6, the medical instrument 10 includes a manifold hub 20 and a ductal access catheter 40 that extends from a distal end 21 of the manifold hub 20. The access catheter 40 is sized to accesses the breast duct. As illustrated, the manifold hub 20 may have a low profile (height) in a direction that extends parallel to the length of the catheter 40. As illustrated in Figures 1-6, the height of the manifold hub 20 may be less than its length (the direction it extends perpendicular to the length of the catheter 40). In a first embodiment, the manifold hub 20 may have a width in a range from about 0.25 inch to about 0.375 inch and a height in a range from about 0.75 inch to about 1.0 inch. In another embodiment, the manifold hub 20 has an internal fluid capacity of 1 ml or less. The low profile of the manifold hub 20 will help to prevent a pivot point from being formed at a location along the length of the catheter 40 at which a large torque may be applied to the duct of a patient during a medical procedure. By eliminating or, at least, significantly reducing any torquing of the duct, the duct will not be kinked, closed due to a change in the position of ductal tissue or injured due to the catheter 40 pushing against the epithelial lining of the duct. The instrument 10 also has an ergonomic design that allows easily handling and grasping by an attendant or practitioner so that the manifold hub 20 and catheter 40 may be easily manipulated.

In the event that the manifold hub 20 needs to be spaced from the nipple surface, a retractable spacer 90 may be positioned on the distal end of the manifold hub and at the proximal end of the catheter 40 as shown in Figures 7 and 8. The retractable spacer 90 may have a spacing distance in the range from about 1mm to about 10mm, most typically in the range of about 5mm. In a first embodiment, the retractable spacer 90 may include a first spacing member 92 received within a second spacing member 93. Alternatively, the first spacing member 92 may telescopically receive the second spacing member 93. In this embodiment, the first member 92 is secured against movement relative to the manifold hub 20, while the second member 93 is moveable relative to both the first member 92 and the manifold hub 20. In an alternative embodiment, both of the spacing members 92, 93 are moveable relative to the manifold hub 20. The distances required for spacing the manifold hub 20 from the surface of the nipple, for example distances of between about 15 mm and 20 mm, may be controlled by locking the spacing members 92, 93 in an extended position (Figure 8). Alternatively, the retractable spacer 90 may be locked in a retracted position (Figure 7). In an alternative embodiment, the retractable spacer 90 includes a single moveable spacing member 95 that slideably receives a portion of the manifold hub 20 to achieve the retracted position and that may be locked at the end of the manifold hub 20 to achieve the extended position. In any of the above-discussed embodiments, the spacing members 92, 93, 95 may be rotated relative to each other and the manifold hub 20 in order to lock each spacing member 92, 93 and 95 against translational movement relative to each other and the manifold hub 20. Any known rotational locking system for telescoping members may be used. Alternatively, the spacing members 92, 93, 95 may be snapped into a locked position using well known snap locks. When additional spacing is needed, members 92, 93, 95 of different sizes or more than two telescoping members may be provided.

The manifold hub 20 may be formed of a transparent material so that an attendant or practitioner may easily view fluid(s) and material(s) within the manifold hub 20. The transparent material may be a plastic, such as ABS plastics or other known plastic materials. As illustrated in Figures 1-8, an embodiment of the manifold hub 20 may have a substantially "F" shape.

As shown in Figures 2A, 2B and 4, the manifold hub 20 includes a first port 30 for connecting to an infusion tube 34 through which materials including wash fluids, diagnostic agents or treatment agents are delivered from an infusion device 38 to the first port 30, the manifold hub 20 and, eventually, the ductal access catheter 40. The connected infusion device 38 may include a syringe or other known fluid containers. In an embodiment, the infusion device 38 may include a fluid receptacle, such as a bag or a container, positioned at a location above the breast of the patient. In this embodiment, the height of the container above the breast of the patient and gravity are used to deliver the fluid from the infusion device 38 to the infusion tube 34.

As shown in Figures 2A, 2B and 4, the manifold hub 20 also includes a second port 32 for connection to a collection tube 36. Ductal fluid samples collected from within the breast duct may be delivered from the manifold hub 20 to a collection receptacle 39 via the collection tube 36. The collection receptacle 39 may include a syringe or other known fluid collection device including a medical fluid bag or container. In an embodiment, the collection receptacle 39 may include or be connected to a source of negative pressure so that an area of low pressure may be created within the collection tube 36 and, if needed, the manifold hub 20 for assisting in the delivery of the retrieved ductal fluid sample to the collection receptacle 39. The area of low pressure, for example a vacuum in an embodiment, may be created using a bulb syringe, a hand-operated vacuum source, a foot operated vacuum source or motor controlled vacuum source. These vacuum sources may include a pump that creates negative pressure within the collection tube 36. In addition to a source of lower pressure, or in place of the source of lower pressure, low pressure within collection tube 36 may be created by infusing fluid into the manifold hub 20, thereby increasing the pressure within the manifold hub 20 relative to the collection tube 36. In any of these embodiments, the collection receptacle 39 may be positioned at a location below the patient during the procedure so that the collected ductal fluid sample may be delivered to the receptacle 39 by gravity.

The first and second ports 30, 32 may include an opening along the sidewall of the manifold hub 20 that is round, oval or any other geometric shape conducive to fluid flow either into the duct or out from the duct as shown in Figures 2A and 2B. The diameter of the ports 30, 32 may be that diameter which is suitable to achieve a desired flow rate into the duct or aspiration or collection rate out from the duct. Thus, the diameters of the ports 30, 32 may be in a range from about 0.060 inches to about 0.090 inches. One side port 30, 32 may be larger or smaller than the other, especially where such differential port size provides a desired flow rate into or out from one of the lumens, or an overall lavage efficiency of infusion and aspiration or collection of lavage and ductal fluid.

Figures 1-6 illustrate that the first and second ports 30, 32 and their associated tubes 34, 36, respectively, are positioned within a connector housing 25 that extends transverse to the longitudinal axis of the manifold hub 20 and the catheter 40. The connector housing 25 may be integrally formed with the manifold hub 20 as a unitary element. Alternatively, the connector housing 25 may be formed separate of the manifold hub 20 and secured to the manifold hub 20 as discussed below with respect to the catheter 40. The connector housing 25 includes two channels 26 that each receives one of the tubes 34, 36. The channels 26 extend from the ports 30, 32 on the manifold hub 20 to the outer, end surface of the connector housing 25. Each channel 26 aligns the received tube 34, 36 with its respective manifold hub port 30, 32 for easy, reliable and quick connection of the tubes 34, 36 to their respective ports 30, 32. The channels 26 also support the tubes 34, 36 at their point of connection to their ports 30, 32 so that the tubes 34, 36 do not create a moment (that may torque the duct) about the point where they connect to their respective ports 30, 32. The connector housing 25 may also include contoured sidewalls 28 with integrally formed or otherwise secured ridges 29 that may be gripped by an attendant or a practitioner. The contoured sidewalls 28 permit easy grasping by the attendant or practitioner and allow the attendant or practitioner to orient the instrument 10 during a procedure without looking at the instrument 10.

In an embodiment shown in Figure 2B, the first and second ports 30, 32 may include posts 35 that extend within connector housing 25 and receive the flexible infusion and collection tubes 34, 36. In an embodiment, the infusion and collection tubes 34, 36 may be formed of flexible tubing such as surgical tubing. However, the tubes 34, 36 may be formed of any flexible material including a flexible plastic. In one embodiment, the tubes 34, 36 are formed of flexible PVC.

The posts 35 may securely receive the tubes 34, 36 when the tubes 34, 36 are positioned over, or within, the posts 35. Alternatively, the ports 30, 32 and their associated posts 35 may include luer lock fittings (not shown) that cooperate with corresponding luer lock fittings on a first end of the tubes 34, 36. The second end of the tubes 34, 36 may also include luer lock fittings that mate with standard luer lock fittings on the syringes or other fluid containers. The luer lock fittings may be either male or female fittings. As discussed herein, the syringes and other fluid containers may carry and infuse saline, diagnostic materials, such as contrast materials, and therapeutic treatment materials into the infusion tube 34. The tubes 34, 36 may be secured to the posts 35 of the manifold hub 20 and the luer locks using a UV curable adhesive or other known bonding agents. In alternative embodiments, the tubes 34, 36 may be secured to the manifold hub 20 and the luer locks by overmolding.

As shown in Figures 1-6, the first port 30 may be positioned adjacent the second port 32 along the perimeter of the manifold hub 20. In the illustrated embodiment, the circumferentially adjacent ports 30, 32 are spaced the same longitudinal distance from the first and second ends 22, 24 of the' manifold hub 20. This spacing of the ports 30, 32 provides for a compact and low profile manifold hub 20 that, as discussed above, will not create a moment and associated forces that torque the duct when the manifold hub 20 is positioned on the patient and free of support from a practitioner or other attendant.

The second port 32 may be circumferentially spaced any distance from the first port 30 around the wall of the manifold hub 20. In an embodiment, the first port 30 may be between forty-five and ninety degrees offset from the second port 32 around the circumference of the manifold hub 20. In an alternative embodiment, the first port 30 may be circumferentially offset along the manifold wall from the second port 32 by between ninety and one hundred-eighty degrees. In an embodiment, the first port 30 is circumferentially offset from the second port 32 by about one hundred-eighty degrees so that the first and second ports 30, 32 oppose each other within the manifold hub 20.

In an alternative embodiment, it is possible for the second port 32 to be located along the hub 20 at a position that is spaced a greater longitudinal distance away from the catheter 40 than the first port 30. In this embodiment, the second port 32 may be used to collect the fluid that enters the manifold hub 20 from the collection catheter 40. For example one port may be located about 2.0 cm from the distal tip of the catheter 40 and one port may be located about 2.5 cm from the distal tip of the catheter 40.

The tubes 34 and 36 may each include a one-way check valve 39 (Figure 2A) to control the fluid flow into and out of the manifold hub 20. The check valve 39 in the tube 34 may prevent, for example, wash fluid from flowing back into a syringe connected to tube 34 after being infused into tube 34. Similarly, check valve 39 in tube 36 may be used to prevent retrieved ductal fluid samples in tube 36 from flowing back into the manifold hub 20. In an alternative embodiment, pinch clamps on the tubes 34, 36, may replace one or both of the check valves 39. For example, a check valve 39 may be positioned within the infusion tube 34 and a conventional pinch clamp may be positioned on the collection tube 36. Other known devices for controlling the direction and timing of fluid flow within a tube 34, 36 may also be used.

As shown in Figures 1-6, the catheter 40 includes a thin walled microcatheter 41 that is secured to the manifold hub 20. In a first embodiment, the microcatheter 41 is integrally formed as part of the manifold hub 20. In another embodiment, the microcatheter 41 is formed as a separate piece and then secured to the manifold hub 20 by microwelding or a UV curable adhesive. Other known techniques for securing the microcatheter 41 to the manifold hub 20 could be used. In any of the above-discussed embodiments, the catheter 40 may be coated with a known agent to provide a lubricious coating that allows it to be easily introduced into the breast duct openings. The coating may include a lubricant, a cleaning agent, anesthetic and/or a dilating agent. The microcatheter 41 may be formed of any known biocompatible material such as FEP. The catheter 40 may have an outer diameter in a range from about 0.01 inch (about 0.25 mm) to about 0.05 inch (about 1.25 mm) with an inner lumen 43 having a diameter in the range from about 0.008 inch (about 0.2 mm) to about 0.047 inch (about 1.2 mm). In an embodiment, the microcatheter 41 has an inner lumen 43 having an outer diameter of about 0.030 inch (about 0.762 mm) and an inner diameter of about 0.025 inch (about 0.63 mm).

The catheter 40 may include length indicia (not shown) on an outer surface of the catheter 40 that permits a user to determine the depth to which the distal end of the catheter has been introduced into the breast duct. In an alternative embodiment, the catheter 40 could include an integrally formed or attached stop element (not shown) that prevents insertion of the catheter into the duct beyond a predetermined distance. In one embodiment, the stop element may comprise a knob on the catheter 40 having an increased diameter for preventing the distal portion of the catheter 40 from entering a duct a greater distance than the knob is spaced from the distal end of the catheter40.

As illustrated in Figures 1-7, the catheter 40 may be tapered along its length to make a smooth transition with a received introducer 50 so that a perceptible transition between the catheter 40 and the introducer 50 that would cause any pain to the patient is not formed and felt by the patient. The catheter 40 may also include an atraumatic distal tip portion 42 at its distal end. The distal tip portion 42 may be tapered, contoured and/or rounded so as to produce an atraumatic tip that will reduce or eliminate trauma to the duct upon entry through the ductal orifice and introduction into the ductal lumen past the ductal sphincter. The distal tip portion 42 may also reduce or eliminate trauma upon withdrawal of the catheter 40 from the duct after the medical procedure, such as ductal lavage or the infusion of a diagnostic and/or treatment agent, has been completed. The tip portion 42 may be composed of a soft polymeric material, e.g. including polyvinyl chloride, polyethers, polyamides, polyethylenes, polyurethanes, copolymers thereof and the like. The tip portion 42 may have a diameter in the range from about 0.012 inches (about 0.031 mm) to about 0.020 inches (about 0.051 mm). In an embodiment, the tip portion 42 has a diameter in the range from about 0.014 inches (about 0.036 mm) to about 0.018 inches (about 0.046 mm). The length of the tip portion 42 (extending from the distal end of the distal portion of the catheter 40 toward the proximal end of the catheter 40) may be in a range from about 0.10 inch (about 0.25 cm) to about 1.0 inch (about 2.5 cm), more typically in the range from about 0.20 inch (about 0.50 cm) to about 0.70 inch (about 1.8 cm).

The stiffened distal portion of the catheter 40, including the distal tip 42, may have an average bending stiffness in the range from about 0.010 inch-lbs to about 0.5 inch-lbs. The catheter 40 may also have a stiffness that is similar to that of a heavy suture (approximately 0.025 OD). The proximal portion of the catheter 40 may have a cross-sectional geometry and/or size that inhibits insertion through the ductal orifice and into the ductal lumen.

A Touhy-Borst fitting 70 may be positioned at a proximal end 22 of the manifold hub 20 to allow a user to easily receive and move the catheter 40 over an introducer 50 as shown in Figures 1-6. The Touhy-Borst fitting 70 is positioned at the end of the manifold hub 20 to cover and seal the opening 77 through which an introducer 50 (discussed-below) including a guidewire, stylet, dilator or the like may extend. The Touhy-Borst fitting 70 comprises a silicone plug 72 including a small aperture 74 for receiving the introducer 50, and a threaded cap 76. When the cap 76 is rotated in a first direction, the silicone plug 72 is altered and the size of the aperture 74 is reduced. This results in the silicone plug 72 forming a seal around the inserted introducer 50. When the cap 76 is turned in a second, opposite direction, the aperture 74 and created seal open, thereby allowing the introducer 50 to be removed. The silicone plug 72 may also be closed to seal the proximal end of the manifold hub 20 so when the introducer 50 is not present so that the distal end of the manifold hub 20 is sealed against fluid flow when the proximal end 22 is free of an introducer 50.

The introducer 50 may be located within the manifold hub 20 to assist in placing the catheter 40 into the breast duct and ductal lumen via the ductal opening as shown in Figure 1. The introducer 50 may include a tapered dilator, a series of progressively larger diameter dilators, a guidewire, including tapered guidewires, a stylet or other known introducers. As illustrated, the introducer 50 will pass through the Touhy-Borst fitting 70 at the proximal end 22 of the manifold hub 20 so that the introducer 50 may be removed after positioning of the catheter 40 and prior to the infusion/collection of the wash fluid. As discussed above, prior to being inserted into the breast duct, the Touhy-Borst fitting 70 may be turned down over the introducer 50 during introduction and then backed off when the catheter 40 has been positioned within the breast duct to the desired depth. The introducer 50 may be formed of a stiff material such as a metal wire or a flexible plastic cord. In an embodiment, the introducer 50 may be formed of stainless steel or a flexible material such as polypropylene monofilament. In an alternative embodiment, the introducer 50 may be formed of multiple materials or the same materials having different stiffnesses. As a result, the introducer 50 may have sections that are more flexible than adjacent sections of the same introducer 50. As a result, for example, the introducer 50 may have a first, stiff portion for guiding the introducer 50 within the ductal lumen and a second, flexible portion that allows the introducer 50 to conform to the shape of the ductal lumen or lumen branch into which it is introduced. In any of the above-discussed embodiments, the introducer 50 may be coated with a liquid or dry lubricant material that reduces the friction between the introducer 50 and the breast duct during the introduction and advancement of the introducer 50 in the duct.

The introducer 50 may be made of metal or plastics, including shape memory metals and plastics, and may have a tapered and/or an atraumatic tip for gently probing and accessing a breast duct. Preferably, a tapered tip 52 will extend distally of the catheter 40 during the introduction of the catheter 40 into the breast duct. After access of the duct is complete, the introducer 50 may be withdrawn, the Touhy-Borst fitting 70 may be closed and the indwelling catheter 40 may be positioned at a location distal to the ductal sphincter. The introducer 50 may have an outer diameter of from about 0.005 inch to about 0.030 inch. In an embodiment, the introducer 50 has an outer diameter of about 0.010 inch. The introducer 50 may extend through the manifold hub 20 and the lumen of the catheter 40. The introducer 50 may be tapered over its length.

During the process of introducing the catheter 40 into the duct, a ductal opening is located on the surface of a nipple by a practitioner or attendant and a first introducer 50 is advanced through the ductal opening into the duct. The introducer 50 may be a long flexible guide wire, a shorter dilator or any of the other above-mentioned introducers. Prior to, or after the introducer 50 is positioned within the duct, the manifold hub 20 and catheter 40 may receive the first or a second introducer 50. As previously discussed, the Touhy-Borst fitting 70 may be locked about the received introducer 50 to form a fluid tight seal at the distal end of the manifold hub 20 so that fluid does not exit the manifold hub 20 around the introducer 50 during the insertion catheter 40 into the duct (See Figures 9, 10, 12 and 13).

When the catheter 40 is positioned within the duct as intended, the Touhy-Borst fitting 70 is opened and the introducer 50 removed (See Figures 11 and 14). The Touhy-Borst 70 may then be closed again to seal the hub 20. Fluid is then introduced into the manifold hub 20, through the catheter 40 and into the breast duct until resistance is met during the infusion. At this time, it is assumed that the duct is filled. The infusion tube, for example tube 34, may then be closed and the fluid allowed to remain in the duct for a preselected time. During this preselected time, the breast may be massaged and squeezed to stimulate mixing of the wash fluid and ductal fluid, and also ultimately to encourage the fluid to leave the duct and enter the manifold hub 20. The collection tube, for example tube 36, may be opened and the breast squeezed to urge the fluid to progress through the catheter 40 and into the manifold hub 20. If desired, when cloudy return fluid is seen in the hub 20 (which may be transparent or include a transparent window), the infusion tube 34 may be opened and fluid infused into the manifold hub 20 to push the ductal fluid sample that has collected in the hub 20 into the collection tube 36 and a waiting collection receptacle. Alternatively, and possibly additionally, aspiration pressure may be applied within the manifold hub 20 and at the collection tube 36 to aspirate any fluid remaining in the hub 20 into the collection receptacle. The process is repeated either following another infusion of fluid into the duct or by another round of squeezing to encourage return and collection of the infused fluid and cellular material from within the breast duct.

In an embodiment, the method of lavage may include seating a patient substantially upright in a chair during the lavage procedure, rather than the standard or classic supine (face up) position. Alternatively, the patient may be lavaged in a prone position, face down, with nipples and breast down. The prone face down position takes advantage of gravity and allows the breast ducts to drain into the collection receptacle during the procedure when the outflow port is open. Thus, as discussed above, the lavaging procedure may include infusing the breast duct with a wash fluid through an open inflow lumen while an outflow lumen is closed; closing the inflow lumen when the duct is filled; squeezing or massaging the breast or both; and opening the outflow lumen to collect the wash fluid.

The cells collected may comprise ductal epithelial cells; the ductal fluid collected may comprise molecular and cellular material. Analysis of the ductal epithelial cells and/or the molecular and cellular material in the ductal fluid may proceed as described below discussing the diagnostic potential of these collected materials. The collected cells and fluid and fluid components may be analyzed. The lavage fluid including the ductal cells may be analyzed for diagnostic purposes. Conditions in a breast milk duct that are desirable to diagnose include a cancer or precancer condition. The precancer condition may include atypical ductal hyperplasia (ADH) or low-grade ductal carcinoma in situ (LG-DCIS). The diagnostic agent may also have the ability to diagnose other breast related conditions, including, e.g. fibrotic, cystic or conditions relating to lactation. Diagnostic agents may be mixed with the ductal fluid (either in the lavage procedure, or after the fluid is collected).

The fluid infused into the duct to lavage the duct may include known, biocompatible fluids. These lavage fluids may include saline, phosphate buffered saline, a nonabsorbable fluid, an isotonic solution, an osmotic solution, a hypotonic solution, and a hypertonic solution. The wash fluid may comprise for example, saline, phosphate buffered saline, a nonabsorbable fluid, an isotonic solution, an osmotic solution, a hypotonic solution, a hypertonic solution.a protein, a colloid, a sugar, a polymer, mannitol, sorbitol, glucose, glycerol, sucrose, raffinose, fructose, lactulose, sodium chloride, polyethyleneglycol (PEG), maltodextrin, dextran (e.g. dextran 70), hydroxyethyl starch, fluid gelatin, a synthetic colloid, an antibody, a binding protein, or albumin.

As mentioned above, a diagnostic or therapeutic agent may be introduced into a breast duct through the manifold hub 20 and catheter 40. The introduced agent for infusing into the duct may comprise a non-absorbable fluid and/or an oncotic agent and/or an osmotic agent. The agent may be soluble. The agent may comprise a molecule that is a protein, a colloid, a sugar, or a polymer. The agent may be mannitol, sorbitol, glucose, glycerol, sucrose, raffinose, fructose, lactulose, sodium chloride, polyethyleneglycol (PEG), maltodextrin, dextran (e.g. dextran 70), hydroxyethyl starch, fluid gelatin, or a synthetic colloid. The agent may comprise a protein and the protein may be a binding protein or an antibody. The binding protein may be albumin. Administering may comprise administering locally, and local administration may comprise administering intraductally. A system for increasing or standardizing an amount of fluid collectable from a milk duct of a breast may comprise infusing a nonabsorbable fluid and/or an osmotic agent and/or an oncotic agent into the ductal lumen, a medical tool for delivering the agent to the ductal lumen, and instructions for use.

In still another aspect of the present invention as shown in Figures 31A-D, the insertion of a medical instrument, such as a catheter, into a breast duct aided by fluid pressure. As mentioned above, during the process of introducing the catheter 40 into the duct, a ductal opening is located on the surface of a nipple and an introducer 50 is advanced through the ductal opening into the duct (See Figures 9, 10, 12 and 13). Once the introducer 50 has located a duct, the catheter 40 is introduced into the ductal orifice just distal to the ductal sphincter. At this point, the introducer 50 may be removed and fluid pressure applied through the catheter. The use of fluid pressure would aid in opening the ductal sphincter as well as opening, straightening, and lubricating during the insertion of the catheter 40 into the duct. Once the catheter 40 has been seated properly within the duct, the fluid pressure is discontinued.

As shown previously in Figures 2A, 2B and 4, the manifold hub 20 includes a first port 30 for connecting to an infusion tube 34 through which materials including wash fluids, diagnostic agents or treatment agents are delivered from an infusion device 38 to the first port 30, the manifold hub 20 and, eventually, the ductal access catheter 40. In an alternative embodiment of the present invention, the infusion device 38 may also be used to apply fluid pressure through the catheter 40 to assist the penetration of the ductal sphincter. The connected infusion device 38 may include a syringe, a pump, or other known fluid containers. In one embodiment, the connected infusion device 38 is a precision fluid pump that provides either continuous or pulsed fluid pressure. The advantage of using pulsed fluid pressure would be that fluid pressure could be controlled with an incremental advancement of the catheter, thus preventing a build up of pressure within a breast duct. Activation of the pump may be achieved by multiple means such as a toggle switch, a push button, a foot pedal or other methods known to one skilled in the art. In yet another embodiment, the infusion device 38 may include a fluid receptacle, such as a bag or a container, positioned at a location above the breast of the patient. In this embodiment, the height of the container above the breast of the patient and gravity are used to deliver the fluid from the infusion device 38 to the infusion tube 34. In still another embodiment, the fluid is warmed to body temperature or above to relax the ductal sphincter and milk duct tissues to assist in inserting the catheter.

As mentioned above, the device of the present invention may include a one-way check valve 39 (Figure 4) to control the fluid flow into and out of the manifold hub 20. The check valve 39 in the tube 34 may prevent, for example, fluid from flowing back into the infusion device 38 when fluid pressure is being applied. Similarly, check valve 39 in tube 36 may be used to prevent fluid in the manifold hub 20 from flowing back into the collection device 39. In an alternative embodiment, pinch clamps on the tubes 34, 36, may replace one or both of the check valves 39. For example, a check valve 39 may be positioned within the infusion tube 34 and a conventional pinch clamp may be positioned on the collection tube 36. Other known devices for controlling the direction and timing of fluid flow within a tube 34, 36 may also be used.

Any number of alternative combinations could exist for defining the invention, which incorporate one or more elements from the specification, including the description, claims, and drawings, in various combinations or sub combinations. It will be apparent to those skilled in the relevant technology, in light of the present specification, that alternate combinations of aspects of the invention, either alone or in combination with one or more elements or steps defined herein, may be utilized as modifications or alterations of the invention or as part of the invention. It may be intended that the written description of the invention contained herein covers all such modifications and alterations.

manipulation of a breast duct using controlled fluid pressure through a ductal access device.

### DUCTAL LAVAGE MICROCATHETER WITH USER ACTIVATED VALVE AND NITINOL INTRODUCER

### FIELD OF THE INVENTION

The present invention relates to a medical instrument having at least a portion that is introduced into the body of a mammal in order to perform diagnostic or therapeutic medical procedures, more specifically, the present invention relates to a medical instrument useable during a diagnostic or therapeutic medical procedure that is sized for introducing into a breast duct through a ductal orifice.

### BACKGROUND OF THE INVENTION

The breast is a specialized, glandular structure including a system of complicated breast ducts that radiate from the nipple and that are bound together by fairly dense connective tissue. Each of these breast ducts includes an associated ductal orifice on the surface of a nipple through which ductal fluid may be expressed. Each duct includes a series of successive interlobular branches that drain through the main, lactiferous branch, which terminates and exits the breast at the nipple via the associated ductal orifice. Immediately proximate the ductal orifice, each lactiferous duct includes a lactiferous sinus in which ductal fluid may accumulate. A ductal sphincter resides within the lactiferous sinus and prevents ductal fluid from unintentionally exiting the breast duct through its associated ductal orifice.

Breast cancer is believed to begin in the lining of these breast ducts. For several decades significant members of the medical community dedicated to studying breast cancer have believed and shown that the cytological analysis of cells retrieved from nipple discharge fluid from within breast ducts may provide valuable information leading to identifying patients at risk for breast cancer. Indeed, Papanicolaou contributed to the genesis of such a possibility of a "Pap" smear for breast cancer by analyzing the cells contained in nipple discharge. More recently, cancer specific markers have been detected in ductal fluid obtained by nipple aspiration. However, the retrieval techniques and instruments used by these members of the medical community did not routinely obtain meaningful ductal fluid samples.

In their attempts to retrieve the breast duct fluid sample including ductal epithelial cells, practitioners introduced wash fluids into a breast duct using indwelling hair-like single lumen catheters. After the fluid was introduced into the duct, the fluid introduction catheters were removed. Then, externally applied nipple aspiration techniques or external pressure applied to the breast were used to collect samples of the ductal fluid. However, these techniques required that significant, sometimes painful, pressure be created on the nipple surface or along the sides of the breast to overcome the fluid retaining properties of the ductal sphincter. Also, these techniques did not routinely provide meaningful ductal fluid samples with a sufficient number of ductal epithelial cells for a meaningful cellular analysis. These techniques typically caused the recovery of samples with twenty or fewer ductal epithelial cells. Additionally, these techniques did not provide samples with cell clusters of 10 or more cells. As a result, the obtained fluid samples could not consistently provide an accurate indication of whether or not the duct from which they were retrieved included precancerous or cancerous cells. Consistent, meaningful ductal epithelial cell samples have been provided by the medical instrument disclosed in U.S. Patent No. 6,413,228 to Hung et al. that is hereby incorporated by reference in its entirety.

Other medical instruments, such as those used during galactography, are introduced into the breast duct in order to visually determine the presence of cancerous cells within a breast duct. However, these devices typically extend a significant distance out of the breast duct during the performed procedure. These distances may be twelve inches or greater. As a result, when an operator is not holding the tool, the moment created by the weight and length of the section of the instrument extending out of the duct may cause the indwelling portion of the instrument to engage the sidewalls of the duct, torque and/or kink the duct and distort the nipple. These effects on the duct and nipple may impede the procedure by twisting or crimping the indwelling portion of the instrument, possibly injuring the patient's duct and causing significant discomfort to the patient. As a result, a patient must either endure the pain and discomfort caused by these long instruments or an attendant must constantly support the instrument above the patient during the medical procedure.

However, in the confined space around an operating table and in the area surrounding a nipple surface, it is not practical to have an attendant constantly holding the end of the instrument that is extending into the breast duct. Therefore, prior to receiving the procedure, a patient must decide to either experience discomfort during the procedure or choose not to have the procedure performed. Prior art instruments are also not ergonomically designed for easy grasping and adjusting by a practitioner or attendant while acting in the area surrounding a nipple.

### SUMMARY OF THE INVENTION

In accordance with one aspect of the present invention, a device for being introduced and positioned within a breast duct for introducing or removing material within the breast duct is provided. The apparatus comprises a manifold hub comprising a sealing fitting for sealing about a ductal introducer extending through the manifold hub at one end of the manifold hub the sealing fitting including an aperture that may be repeatedly opened and closed, the sides of the manifold hub comprising two opposing flexible arms connected at one end to the base of the hub and unconnected at the other end and substantially parallel to one another, the arms are held in a sprung position such that the unconnected ends of said arms compress the sealing fitting such that the aperture is closed and when the arms are compressed, the unconnected ends of the arms no longer compress the sealing fitting and the aperture is opened. The sealing fitting may be comprised of a silicone gasket. The apparatus may also include springs that are located between said flexible arms and the body of said hub such that the springs keep said arms in actuated position. The free ends of the arms may be interlocking hooks. The ductal introducer may be a ring shaped handle. The ductal introducer may be made of a flexible material such as Nitinol and may have a distal tip that tapers from approximately 0.025 inches to approximately 0.008 inches.

In yet another aspect of the present invention, a method for obtaining cellular material from a human breast milk duct is provided. The method may include using a device comprising a manifold hub comprising a fitting for sealing about a ductal introducer extending through said manifold hub at one end of said manifold hub said fitting including an aperture that may be repeatedly opened and closed, the sides of said manifold hub comprising two opposing flexible arms connected at one end to the base of said hub and unconnected at the other end and substantially parallel to one another, said arms are held in a sprung position such that said unconnected ends of said arms compress said fitting such that said aperture is closed and when said arms are compressed, said unconnected ends of said arms no longer compress said fitting and said aperture is opened; and including the steps of inserting the introducer into the internal passageway of a breast duct; and advancing the catheter into the breast duct.

In still yet another aspect of the present invention, a method for obtaining cellular material from a human breast milk duct is provided. The method may include using a device comprising a manifold hub comprising a fitting for sealing about a ductal introducer extending through said manifold hub at one end of said manifold hub said fitting including an aperture that may be repeatedly opened and closed, the sides of said manifold hub comprising two opposing flexible arms connected at one end to the base of said hub and unconnected at the other end and substantially parallel to one another, said arms are held in a sprung position such that said unconnected ends of said arms compress said fitting such that said aperture is closed and when said arms are compressed, said unconnected ends of said arms no longer compress said fitting and said aperture is opened; and including the steps of inserting the introducer into the internal passageway of a breast duct advancing a sheath into the breast duct; and advancing the catheter into the breast duct.

### BRIEF DESCRIPTION OF THE FIGURES

Figure 1 is a perspective view of a medical instrument according to aspects of the present invention;

Figures 2A and 2B are cross sections' of alternative embodiments of the medical instrument illustrated in Figure 1;

Figure 3 is an exploded perspective view of the medical instrument of Figure 1;

Figure 4 is a top view of the medical instrument of Figure 1 with infusion and collection lines extending between a manifold hub and respective infusion and collection devices;

Figure 5 is a side view of the medical instrument illustrated in Figure 1 carrying infusion and collection lines;

Figure 6 is another perspective view of the medical instrument illustrated in Figure 1;

Figures 7 and 8 illustrate an alternative embodiment of the medical instrument according to aspects of the present invention;

Figures 9-10 illustrate a method for introducing the medical instrument of Figure 1 into a breast duct using a stiff introducer; and

Figures 12-14 illustrate an alternative method for introducing the medical instrument of Figure 1 into a breast duct using a flexible introducer.

Figures 32-36 illustrate an alternative embodiment of the medical instrument according to aspects of the present invention including a ductal lavage microcatheter with user activated valve and Nitinol introducer with a ring shaped handle.

### DETAILED DESCRIPTION OF THE INVENTION

Figure 1 illustrates an embodiment of a low profile, single lumen medical instrument 10 for performing a medical procedure within a breast duct. As used herein, the phrase "medical procedure" may include preparatory procedures, diagnostic procedures or therapeutic procedures. These procedures could include the steps of delivering material(s) into the breast duct and/or retrieving material(s) from within the breast duct.

In an embodiment, the medical instrument 10 may be used to infuse ductal wash fluid delivered to the manifold hub 20 into the breast duct, and collect or draw up ductal fluid samples, including hundreds of ductal epithelial cells and/or cell clusters of greater than ten cells, from within the breast duct for analysis. In another embodiment, the medical instrument 10 may be used to infuse a diagnostic agent or therapeutic agent into a breast duct. As shown in Figures 1-8, the instrument 10 may also include a member 11 for securing the instrument 10 to a patient. The securing member 11 may have a biocompatible adhesive on one side for contacting and attaching the instrument 10 to the patient. The member 11 is sized to prevent movement of the manifold hub 20 relative to the body of the patient. In an embodiment illustrated in Figures 7 and 8, sections 11 A and 11B of the member 11 may be folded onto each other so that the size of the securing member 11 may be adjusted to the patient and the forces created during the procedure. Additionally, the member 11 may be positioned distal a spacer 90 (discussed below).

As illustrated in Figures 1-6, the medical instrument 10 includes a manifold hub 20 and a ductal access catheter 40 that extends from a distal end 21 of the manifold hub 20. The access catheter 40 is sized to accesses the breast duct. As illustrated, the manifold hub 20 may have a low profile (height) in a direction that extends parallel to the length of the catheter 40. As illustrated in Figures 1-6, the height of the manifold hub 20 may be less than its length (the direction it extends perpendicular to the length of the catheter 40). In a first embodiment, the manifold hub 20 may have a width in a range from about 0.25 inch to about 0.375 inch and a height in a range from about 0.75 inch to about 1.0 inch. In another embodiment, the manifold hub 20 has an internal fluid capacity of 1 ml or less. The low profile of the manifold hub 20 will help to prevent a pivot point from being formed at a location along the length of the catheter 40 at which a large torque may be applied to the duct of a patient during a medical procedure. By eliminating or, at least, significantly reducing any torquing of the duct, the duct will not be kinked, closed due to a change in the position of ductal tissue or injured due to the catheter 40 pushing against the epithelial lining of the duct. The instrument 10 also has an ergonomic design that allows easily handling and grasping by an attendant or practitioner so that the manifold hub 20 and catheter 40 may be easily manipulated.

In the event that the manifold hub 20 needs to be spaced from the nipple surface, a retractable spacer 90 may be positioned on the distal end of the manifold hub and at the proximal end of the catheter 40 as shown in Figures 7 and 8. The retractable spacer 90 may have a spacing distance in the range from about 1mm to about 10mm, most typically in the range of about 5mm. In a first embodiment, the retractable spacer 90 may include a first spacing member 92 received within a second spacing member 93. Alternatively, the first spacing member 92 may telescopically receive the second spacing member 93. In this embodiment, the first member 92 is secured against movement relative to the manifold hub 20, while the second member 93 is moveable relative to both the first member 92 and the manifold hub 20. In an alternative embodiment, both of the spacing members 92, 93 are moveable relative to the manifold hub 20. The distances required for spacing the manifold hub 20 from the surface of the nipple, for example distances of between about 15 mm and 20 mm, may be controlled by locking the spacing members 92, 93 in an extended position (Figure 8). Alternatively, the retractable spacer 90 may be locked in a retracted position (Figure 7). In an alternative embodiment, the retractable spacer 90 includes a single moveable spacing member 95 that slidably receives a portion of the manifold hub 20 to achieve the retracted position and that may be locked at the end of the manifold hub 20 to achieve the extended position. In any of the above-discussed embodiments, the spacing members 92, 93, 95 may be rotated relative to each other and the manifold hub 20 in order to lock each spacing member 92, 93 and 95 against translational movement relative to each other and the manifold hub 20. Any known rotational locking system for telescoping members may be used. Alternatively, the spacing members 92, 93, 95 may be snapped into a locked position using well known snap locks. When additional spacing is needed, members 92, 93, 95 of different sizes or more than two telescoping members may be provided.

The manifold hub 20 may be formed of a transparent material so that an attendant or practitioner may easily view fluid(s) and material(s) within the manifold hub 20. The transparent material may be a plastic, such as ABS plastics or other known plastic materials. As illustrated in Figures 1-8, an embodiment of the manifold hub 20 may have a substantially "F" shape.

As shown in Figures 2A, 2B and 4, the manifold hub 20 includes a first port 30 for connecting to an infusion tube 34 through which materials including wash fluids, diagnostic agents or treatment agents are delivered from an infusion device 38 to the first port 30, the manifold hub 20 and, eventually, the ductal access catheter 40. The connected infusion device 38 may include a syringe or other known fluid containers. In an embodiment, the infusion device 38 may include a fluid receptacle, such as a bag or a container, positioned at a location above the breast of the patient. In this embodiment, the height of the container above the breast of the patient and gravity are used to deliver the fluid from the infusion device 38 to the infusion tube 34.

As shown in Figures 2A, 2B and 4, the manifold hub 20 also includes a second port 32 for connection to a collection tube 36. Ductal fluid samples collected from within the breast duct may be delivered from the manifold hub 20 to a collection receptacle 39 via the collection tube 36. The collection receptacle 39 may include a syringe or other known fluid collection device including a medical fluid bag or container. In an embodiment, the collection receptacle 39 may include or be connected to a source of negative pressure so that an area of low pressure may be created within the collection tube 36 and, if needed, the manifold hub 20 for assisting in the delivery of the retrieved ductal fluid sample to the collection receptacle 39. The area of low pressure, for example a vacuum in an embodiment, may be created using a bulb syringe, a hand-operated vacuum source, a foot operated vacuum source or motor controlled vacuum source. These vacuum sources may include a pump that creates negative pressure within the collection tube 36. In addition to a source of lower pressure, or in place of the source of lower pressure, low pressure within collection tube 36 may be created by infusing fluid into the manifold hub 20, thereby increasing the pressure within the manifold hub 20 relative to the collection tube 36. In any of these embodiments, the collection receptacle 39 may be positioned at a location below the patient during the procedure so that the collected ductal fluid sample may be delivered to the receptacle 39 by gravity.

The first and second ports 30, 32 may include an opening along the sidewall of the manifold hub 20 that is round, oval or any other geometric shape conducive to fluid flow either into the duct or out from the duct as shown in Figures 2A and 2B. The diameter of the ports 30, 32 may be that diameter which is suitable to achieve a desired flow rate into the duct or aspiration or collection rate out from the duct. Thus, the diameters of the ports 30, 32 may be in a range from about 0.060 inches to about 0.090 inches. One side port 30, 32 may be larger or smaller than the other, especially where such differential port size provides a desired flow rate into or out from one of the lumens, or an overall lavage efficiency of infusion and aspiration or collection of lavage and ductal fluid.

Figures 1-6 illustrate that the first and second ports 30, 32 and their associated tubes 34, 36, respectively, are positioned within a connector housing 25 that extends transverse to the longitudinal axis of the manifold hub 20 and the catheter 40. The connector housing 25 may be integrally formed with the manifold hub 20 as a unitary element. Alternatively, the connector housing 25 may be formed separate of the manifold hub 20 and secured to the manifold hub 20 as discussed below with respect to the catheter 40. The connector housing 25 includes two channels 26 that each receives one of the tubes 34, 36. The channels 26 extend from the ports 30, 32 on the manifold hub 20 to the outer, end surface of the connector housing 25. Each channel 26 aligns the received tube 34, 36 with its respective manifold hub port 30, 32 for easy, reliable and quick connection of the tubes 34, 36 to their respective ports 30, 32. The channels 26 also support the tubes 34, 36 at their point of connection to their ports 30, 32 so that the tubes 34, 36 do not create a moment (that may torque the duct) about the point where they connect to their respective ports 30, 32. The connector housing 25 may also include contoured sidewalls 28 with integrally formed, or otherwise secured, ridges 29 that may be gripped by an attendant or a practitioner. The contoured sidewalls 28 permit easy grasping by the attendant or practitioner and allow the attendant or practitioner to orient the instrument 10 during a procedure without looking at the instrument 10.

In an embodiment shown in Figure 2B, the first and second ports 30, 32 may include posts 35 that extend within connector housing 25 and receive the flexible infusion and collection tubes 34, 36. In an embodiment, the infusion and collection tubes 34, 36 may be formed of flexible tubing such as surgical tubing. However, the tubes 34, 36 may be formed of any flexible material including a flexible plastic. In one embodiment, the tubes 34, 36 are formed of flexible PVC.

The posts 35 may securely receive the tubes 34, 36 when the tubes 34, 36 are positioned over, or within, the posts 35. Alternatively, the ports 30, 32 and their associated posts 35 may include luer lock fittings (not shown) that cooperate with corresponding luer lock fittings on a first end of the tubes 34, 36. The second end of the tubes 34, 36 may also include luer lock fittings that mate with standard luer lock fittings on the syringes or other fluid containers. The luer lock fittings may be either male or female fittings. As discussed herein, the syringes and other fluid containers may carry and infuse saline, diagnostic materials, such as contrast materials, and therapeutic treatment materials into the infusion tube 34. The tubes 34, 36 may be secured to the posts 35 of the manifold hub 20 and the luer locks using a UV curable adhesive or other known bonding agents. In alternative embodiments, the tubes 34, 36 may be secured to the manifold hub 20 and the luer locks by overmolding.

As shown in Figures 1-6, the first port 30 may be positioned adjacent the second port 32 along the perimeter of the manifold hub 20. In the illustrated embodiment, the circumferentially adjacent ports 30, 32 are spaced the same longitudinal distance from the first and second ends 22, 24 of the manifold hub 20. This spacing of the ports 30, 32 provides for a compact and low profile manifold hub 20 that, as discussed above, will not create a moment and associated forces that toque the duct when the manifold hub 20 is positioned on the patient and free of support from a practitioner or other attendant.

The second port 32 may be circumferentially spaced any distance from the first port 30 around the wall of the manifold hub 20. In an embodiment, the first port 30 may be between forty-five and ninety degrees offset from the second port 32 around the circumference of the manifold hub 20. In an alternative embodiment, the first port 30 may be circumferentially offset along the manifold wall from the second port 32 by between ninety and one hundred-eighty degrees. In an embodiment, the first port 30 is circumferentially offset from the second port 32 by about one hundred-eighty degrees so that the first and second ports 30, 32 oppose each other within the manifold hub 20.

In an alternative embodiment, it is possible for the second port 32 to be located along the hub 20 at a position that is spaced a greater longitudinal distance away from the catheter 40 than the first port 30. In this embodiment, the second port 32 may be used to collect the fluid that enters the manifold hub 20 from the collection catheter 40. For example one port may be located about 2.0 cm from the distal tip of the catheter 40 and one port may be located about 2.5 cm from the distal tip of the catheter 40.

The tubes 34 and 36 may each include a one-way check valve 39 (Figure 2A) to control the fluid flow into and out of the manifold hub 20. The check valve 39 in the tube 34 may prevent, for example, wash fluid from flowing back into a syringe connected to tube 34 after being infused into tube 34. Similarly, check valve 39 in tube 36 may be used to prevent retrieved ductal fluid samples in tube 36 from flowing back into the manifold hub 20. In an alternative embodiment, pinch clamps on the tubes 34, 36, may replace one or both of the check valves 39. For example, a check valve 39 may be positioned within the infusion tube 34 and a conventional pinch clamp may be positioned on the collection tube 36. Other known devices for controlling the direction and timing of fluid flow within a tube 34, 36 may also be used.

As shown in Figures 1-6, the catheter 40 includes a thin walled microcatheter 41 that is secured to the manifold hub 20. In a first embodiment, the microcatheter 41 is integrally formed as part of the manifold hub 20. In another embodiment, the microcatheter 41 is formed as a separate piece and then secured to the manifold hub 20 by microwelding or a UV curable adhesive. Other known techniques for securing the microcatheter 41 to the manifold hub 20 could be used. In any of the above-discussed embodiments, the catheter 40 may be coated with a known agent to provide a lubricious coating that allows it to be easily introduced into the breast duct openings. The coating may include a lubricant, a cleaning agent, anesthetic and/or a dilating agent. The microcatheter 41 may be formed of any known biocompatible material such as FEP. The catheter 40 may have an outer diameter in a range from about 0.01 inch (about 0.25 mm) to about 0.05 inch (about 1.25 mm) with an inner lumen 43 having a diameter in the range from about 0.008 inch (about 0.2 mm) to about 0.047 inch (about 1.2 mm). In an embodiment, the microcatheter 41 has an inner lumen 43 having an outer diameter of about 0.030 inch (about 0.762 mm) and an inner diameter of about 0.025 inch (about 0.63 mm).

The catheter 40 may include length indicia (not shown) on an outer surface of the catheter 40 that permits a user to determine the depth to which the distal end of the catheter has been introduced into the breast duct. In an alternative embodiment, the catheter 40 could include an integrally formed or attached stop element (not shown) that prevents insertion of the catheter into the duct beyond a predetermined distance. In one embodiment, the stop element may comprise a knob on the catheter 40 having an increased diameter for preventing the distal portion of the catheter 40 from entering a duct a greater distance than the knob is spaced from the distal end of the catheter40.

As illustrated in Figures 1-7, the catheter 40 may be tapered along its length to make a smooth transition with a received introducer 50 so that a perceptible transition between the catheter 40 and the introducer 50 that would cause any pain to the patient is not formed and felt by the patient. The catheter 40 may also include an atraumatic distal tip portion 42 at its distal end. The distal tip portion 42 may be tapered, contoured and/or rounded so as to produce an atraumatic tip that will reduce or eliminate trauma to the duct upon entry through the ductal orifice and introduction into the ductal lumen past the ductal sphincter. The distal tip portion 42 may also reduce or eliminate trauma upon withdrawal of the catheter 40 from the duct after the medical procedure, such as ductal lavage or the infusion of a diagnostic and/or treatment agent, has been completed. The tip portion 42 may be composed of a soft polymeric material, e.g. including polyvinyl chloride, polyethers, polyamides, polyethylenes, polyurethanes, copolymers thereof and the like. The tip portion 42 may have a diameter in the range from about 0.012 inches (about 0.031 mm) to about 0.020 inches (about 0.051 mm). In an embodiment, the tip portion 42 has a diameter in the range from about 0.014 inches (about 0.036 mm) to about 0.018 inches (about 0.046 mm). The length of the tip portion 42 (extending from the distal end of the distal portion of the catheter 40 toward the proximal end of the catheter 40) may be in a range from about 0.10 inch (about 0.25 cm) to about 1.0 inch (about 2.5 cm), more typically in the range from about 0.20 inch (about 0.50 cm) to about 0.70 inch (about 1.8 cm).

The stiffened distal portion of the catheter 40, including the distal tip 42, may have an average bending stiffness in the range from about 0.010 inch-lbs to about 0.5 inch-lbs. The catheter 40 may also have a stiffness that is similar to that of a heavy suture (approximately 0.025 OD). The proximal portion of the catheter 40 may have a cross-sectional geometry and/or size that inhibits insertion through the ductal orifice and into the ductal lumen.

A Touhy-Borst fitting 70 may be positioned at a proximal end 22 of the manifold hub 20 to allow a user to easily receive and move the catheter 40 over an introducer 50 as shown in Figures 1-6. The Touhy-Borst fitting 70 is positioned at the end of the manifold hub 20 to cover and seal the opening 77 through which an introducer 50 (discussed-below) including a guidewire, stylet, dilator or the like may extend. The Touhy-Borst fitting 70 comprises a silicone plug 72 including a small aperture 74 for receiving the introducer 50, and a threaded cap 76. When the cap 76 is rotated in a first direction, the silicone plug 72 is altered and the size of the aperture 74 is reduced. This results in the silicone plug 72 forming a seal around the inserted introducer 50. When the cap 76 is turned in a second, opposite direction, the aperture 74 and created seal open, thereby allowing the introducer 50 to be removed. The silicone plug 72 may also be closed to seal the proximal end of the manifold hub 20 so when the introducer 50 is not present so that the distal end of the manifold hub 20 is sealed against fluid flow when the proximal end 22 is free of an introducer 50.

The introducer 50 may be located within the manifold hub 20 to assist in placing the catheter 40 into the breast duct and ductal lumen via the ductal opening as shown in Figure 1. The introducer 50 may include a tapered dilator, a series of progressively larger diameter dilators, a guidewire, including tapered guidewires, a stylet or other known introducers. As illustrated, the introducer 50 will pass through the Touhy-Borst fitting 70 at the proximal end 22 of the manifold hub 20 so that the introducer 50 may be removed after positioning of the catheter 40 and prior to the infusion/collection of the wash fluid. As discussed above, prior to being inserted into the breast duct, the Touhy-Borst fitting 70 may be turned down over the introducer 50 during introduction and then backed off when the catheter 40 has been positioned within the breast duct to the desired depth. The introducer 50 may be formed of a stiff material such as a metal wire or a flexible plastic cord. In an embodiment, the introducer 50 may be formed of stainless steel or a flexible material such as polypropylene monofilament. In an alternative embodiment, the introducer 50 may be formed of multiple materials or the same materials having different stiffnesses. As a result, the introducer 50 may have sections that are more flexible than adjacent sections of the same introducer 50. As a result, for example, the introducer 50 may have a first, stiff portion for guiding the introducer 50 within the ductal lumen and a second, flexible portion that allows the introducer 50 to conform to the shape of the ductal lumen or lumen branch into which it is introduced. In any of the above-discussed embodiments, the introducer 50 may be coated with a liquid or dry lubricant material that reduces the friction between the introducer 50 and the breast duct during the introduction and advancement of the introducer 50 in the duct.

The introducer 50 may be made of metal or plastics, including shape memory metals and plastics, and may have a tapered and/or an atraumatic tip for gently probing and accessing a breast duct. Preferably, a tapered tip 52 will extend distally of the catheter 40 during the introduction of the catheter 40 into the breast duct. After access of the duct is complete, the introducer 50 may be withdrawn, the Touhy-Borst fitting 70 may be closed and the indwelling catheter 40 may be positioned at a location distal to the ductal sphincter. The introducer 50 may have an outer diameter of from about 0.005 inch to about 0.030 inch. In an embodiment, the introducer 50 has an outer diameter of about 0.010 inch. The introducer 50 may extend through the manifold hub 20 and the lumen of the catheter 40. The introducer 50 may be tapered over its length.

During the process of introducing the catheter 40 into the duct, a ductal opening is located on the surface of a nipple by a practitioner or attendant and a first introducer 50 is advanced through the ductal opening into the duct. The introducer 50 may be a long flexible guide wire, a shorter dilator or any of the other above-mentioned introducers. Prior to, or after the introducer 50 is positioned within the duct, the manifold hub 20 and catheter 40 may receive the first or a second introducer 50. As previously discussed, the Touhy-Borst fitting 70 may be locked about the received introducer 50 to form a fluid tight seal at the distal end of the manifold hub 20 so that fluid does not exit the manifold hub 20 around the introducer 50 during the insertion catheter 40 into the duct (See Figures 9, 10, 12 and 13).

When the catheter 40 is positioned within the duct as intended, the Touhy-Borst fitting 70 is opened and the introducer 50 removed (See Figure 14). The Touhy-Borst 70 may then be closed again to seal the hub 20. Fluid is then introduced into the manifold hub 20, through the catheter 40 and into the breast duct until resistance is met during the infusion. At this time, it is assumed that the duct is filled. The infusion tube, for example tube 34, may then be closed and the fluid allowed to remain in the duct for a preselected time. During this preselected time, the breast may be massaged and squeezed to stimulate mixing of the wash fluid and ductal fluid, and also ultimately to encourage the fluid to leave the duct and enter the manifold hub 20. The collection tube, for example tube 36, may be opened and the breast squeezed to urge the fluid to progress through the catheter 40 and into the manifold hub 20. If desired, when cloudy return fluid is seen in the hub 20 (which may be transparent or include a transparent window), the infusion tube 34 may be opened and fluid infused into the manifold hub 20 to push the ductal fluid sample that has collected in the hub 20 into the collection tube 36 and a waiting collection receptacle. Alternatively, and possibly additionally, aspiration pressure may be applied within the manifold hub 20 and at the collection tube 36 to aspirate any fluid remaining in the hub 20 into the collection receptacle. The process is repeated either following another infusion of fluid into the duct or by another round of squeezing to encourage return and collection of the infused fluid and cellular material from within the breast duct.

Figures 32-36 are schematic diagrams illustrating an alternative construction of a ductal access device. In a preferred embodiment of the invention, the device includes a manifold hub 20 and a ductal access catheter 40 that extends from a distal end 21 of the manifold hub 20 with a centrally disposed single lumen which extends the length of the catheter 40. Catheter 40 includes a tubular introducer pathway adapted to slideably receive a ductal introducer 50 therein. For ease of explanation, as used herein the term "introducer" is defined to include guidewires, dilators, stylettes or portion of any of these that may be inserted within a ductal orifice or a passageway of a catheter sized to access a breast duct.

As illustrated, the manifold hub 20 may have a low profile (height) in a direction that extends parallel to the length of the catheter 40. In another construction, hub 20 has a low profile so as to reduce the torque on the breast nipple after insertion of catheter 40. This overcomes the excessive generated torque on the breast nipple known to cause obstruction of ductal fluid due to compression of the tissue. Thus, improved collection of ductal cellular material is provided.

The manifold hub 20 is coupled to the proximal end 45 of the ductal access catheter 40. In a preferred construction, hub 20 includes transparent material so that the user may visualize the flow to and from the breast duct during a lavage procedure. The transparent material may be plastic, such as ABS plastics or other known plastic materials. As illustrated in Figures 32-36, an embodiment of the manifold hub 20 may have a substantially "U" shape.

A fitting 70 may be positioned at a proximal end 22 of the manifold hub 20 to allow a user to easily receive and move the catheter 40 over an introducer 50. The fitting 70 is positioned at the end of the manifold hub 20 to cover and seal the opening 77 through which an introducer 50 including a guidewire, stylet, dilator or the like may extend. The fitting 70 comprises a silicone gasket including a small aperture 74 for receiving the introducer 50, and a cap 76. The cap 76 locks the silicon gasket to the manifold hub 20 thus maintaining a seal between the hub and the gasket. The gasket may be made of any suitable flexible biocompatible material. Around the neck of the silicone gasket is a pair of interlocking hooks 110 which at the terminus of flexible side arms 100 which are part of the main body of the manifold hub 20. Located between the flexible side arms and the manifold hub are one or more springs 120 that force the flexible arms 100 away from the main body of the manifold hub 20. As the springs 120 force the flexible side arms 100 away from the manifold hub 20, the interlocking hooks 110 at the end of the flexible side arms 100 apply pressure to opposite sides of the fitting 70. This results in the fitting 70, which may be comprised of silicone or any other flexible material, to compress around the aperture 74 forming a seal around the inserted introducer 50. When pressure is applied to the flexible arms 100 thus compressing the springs 120, the interlocking hooks 110 are actuated apart thus releasing pressure on the silicon gasket and thus breaking seal around the inserted introducer 50. The flexible side arms 100 and the interlocking hooks 110 may be constructed of metal or plastic or a combination thereof.

In an alternative embodiment, the manifold hub 20 is comprised of a single flexible arm 100 with a single hooked end 110 which, when activated, opens or closes the fitting 70.

The introducer 50 may be located within the manifold hub 20 to assist in placing the catheter 40 into the breast duct and ductal lumen via the ductal opening as shown in Figures 9-13. The introducer 50 may include a tapered dilator, a series of progressively larger diameter dilators, a guidewire, including tapered guidewires, a stylet or other known introducers. As illustrated, the introducer 50 will pass through the fitting 70 at the proximal end 22 of the manifold hub 20. The introducer of the present invention may consist of two parts, a wire and a ring shaped handle located at the end of the introducer. The introducer 50 has a diameter equal to or slightly less than the ID of the catheter 40. The length of the introducer 50 may extend approximately 20 -30 mm beyond the distal end of the catheter 40 when the introducer is fully seated. The extended length of the introducer 50 beyond the distal end of the catheter 40 allows the introducer to be used as a dilator prior to the insertion of the catheter in the breast duct. As illustrated in Figure 18, the distal end of the introducer 50 may be tapered. The distal tip of the introducer 50 may be tapered to an OD in the range of 0.008 to 0.010 inches from the proximal end of the introducer that may have an OD equal to or slightly less than the ID of the distal end of the catheter 42. The small diameter allows for easier insertion of the introducer into the breast milk duct. The introducer 50 may be formed of a stiff material such as a metal wire or a flexible material such as plastic. In an embodiment of the invention, the introducer 50 may be formed of Nitinol. Nitinol is preferred due to its flexibility, durability, and biocompatibility. The introducer 50 may be constructed from a Nitinol wire that is ground to include a tapered small diameter tip. In an alternative embodiment, the introducer 50 may be formed of multiple materials or the same materials having different stiffnesses. As a result, the introducer 50 may have sections that are more flexible than adjacent sections of the same introducer 50. As a result, for example, the introducer 50 may have a first, stiff portion for guiding the introducer 50 within the ductal lumen and a second, flexible portion that allows the introducer 50 to conform to the shape of the ductal lumen or lumen branch into which it is introduced. In any of the above-discussed embodiments, the introducer 50 may be coated with a liquid or dry lubricant material that reduces the friction between the introducer 50 and the breast duct during the introduction and advancement of the introducer 50 in the duct.

The introducer 50 may be made of metal or plastics, including shape memory metals and plastics, and may have a tapered and/or an atraumatic tip for gently probing and accessing a breast duct. Preferably, a tapered tip 52 will extend distally of the catheter 40 during the introduction of the catheter 40 into the breast duct. After access of the duct is complete, the catheter 40 may be slipped over the introducer 50 and positioned at a location distal to the ductal sphincter.

In another embodiment of the invention, the proximal end of the introducer 50 is formed into a ring shaped handle 51 (see Figures 32, 33 and 35). The advantage of the ring shaped handle 51 is that is allows the device 10 to be used with one hand. The ring shaped handle 51 may allow the practitioner to grasp the flexible side arms 100 of the device 10 using their thumb and forefinger and to manipulate the introducer 50 by using their index finger which may be positioned through the ring shaped handle 51. The ring shaped handle 51 may be constructed of a thermoplastic such as ABS or polycarbonate or pellathane, and may be shaped in alternative forms that will allow the practitioner to operate the device 10 with one hand.

During the process of introducing the catheter 40 into the duct, a ductal opening is located on the surface of a nipple by a practitioner or attendant and an introducer 50 is advanced through the ductal opening into the duct. The introducer 50 may be a long flexible guide wire, a shorter dilator or any of the other above-mentioned introducers. Prior to, or after the introducer 50 is positioned within the duct, the manifold hub 20 and catheter 40 may receive the introducer 50. As previously discussed, compressing the flexible side arms 100 thus actuating the interlocking side arms 110 away from the fitting 70 may open the fitting 70. Once pressure is released from the flexible side arms 100, the interlocking arms 110 are forced away from the manifold hub 20 by the springs 120 thus closing the aperture 74 about the received introducer 50 to form a fluid tight seal at the distal end of the manifold hub 20 so that fluid does not exit the manifold hub 20 around the introducer 50 during the insertion catheter 40 into the duct (See Figures 9, 10, 12, 13, 32, 33, and 34).

When the catheter 40 is positioned within the duct as intended, the fitting 70 is opened and the introducer 50 removed. The 70 may then be closed again to seal the hub 20. Fluid is then introduced into the manifold hub 20, through the catheter 40 and into the breast duct until resistance is met during the infusion. At this time, it is assumed that the duct is filled. The infusion tube, for example tube 34, may then be closed and the fluid allowed to remain in the duct for a preselected time. During this preselected time, the breast may be massaged and squeezed to stimulate mixing of the wash fluid and ductal fluid, and also ultimately to encourage the fluid to leave the duct and enter the manifold hub 20. The collection tube, for example tube 36, may be opened and the breast squeezed to urge the fluid to progress through the catheter 40 and into the manifold hub 20. If desired, when cloudy return fluid is seen in the hub 20 (which may be transparent or include a transparent window), the infusion tube 34 may be opened and fluid infused into the manifold hub 20 to push the ductal fluid sample that has collected in the hub 20 into the collection tube 36 and a waiting collection receptacle. Alternatively, and possibly additionally, aspiration pressure may be applied within the manifold hub 20 and at the collection tube 36 to aspirate any fluid remaining in the hub 20 into the collection receptacle. The process is repeated either following another infusion of fluid into the duct or by another round of squeezing to encourage return and collection of the infused fluid and cellular material from within the breast duct.

In an embodiment, the method of lavage may include seating a patient substantially upright in a chair during the lavage procedure, rather than the standard or classic supine (face up) position. Alternatively, the patient may be lavaged in a prone position, face down, with nipples and breast down. The prone face down position takes advantage of gravity and allows the breast ducts to drain into the collection receptacle during the procedure when the outflow port is open. Thus, as discussed above, the lavaging procedure may include infusing the breast duct with a wash fluid through an open inflow lumen while an outflow lumen is closed; closing the inflow lumen when the duct is filled; squeezing or massaging the breast or both; and opening the outflow lumen to collect the wash fluid.

The cells collected may comprise ductal epithelial cells; the ductal fluid collected may comprise molecular and cellular material. Analysis of the ductal epithelial cells and/or the molecular and cellular material in the ductal fluid may proceed as described below discussing the diagnostic methods possible of these collected materials. The collected cells and fluid and fluid components may be analyzed. The lavage fluid including the ductal cells may be analyzed for diagnostic purposes. Conditions in a breast milk duct that are desirable to diagnose include a cancer or precancer condition. The precancer condition may include atypical ductal hyperplasia (ADH) or low-grade ductal carcinoma in situ (LG-DCIS). The diagnostic agent may also have the ability to diagnose other breast related conditions, including, e.g. fibrotic, cystic or conditions relating to lactation. Diagnostic agents may be mixed with the ductal fluid (either in the lavage procedure, or after the fluid is collected).

The fluid infused into the duct to lavage the duct may include known, biocompatible fluids. These lavage fluids may include saline, phosphate buffered saline, a nonabsorbable fluid, an isotonic solution, an osmotic solution, a hypotonic solution, and a hypertonic solution. The wash fluid may comprise for example, saline, phosphate buffered saline, a nonabsorbable fluid, an isotonic solution, an osmotic solution, a hypotonic solution, a hypertonic solution.a protein, a colloid, a sugar, a polymer, mannitol, sorbitol, glucose, glycerol, sucrose, raffinose, fructose, lactulose, sodium chloride, polyethyleneglycol (PEG), maltodextrin, dextran (e.g. dextran 70), hydroxyethyl starch, fluid gelatin, a synthetic colloid, an antibody, a binding protein, or albumin.

As mentioned above, a diagnostic or therapeutic agent may be introduced into a breast duct through the manifold hub 20 and catheter 40. The introduced agent for infusing into the duct may comprise a non-absorbable fluid and/or an oncotic agent and/or an osmotic agent. The agent may be soluble. The agent may comprise a molecule that is a protein, a colloid, a sugar, or a polymer. The agent may be mannitol, sorbitol, glucose, glycerol, sucrose, raffinose, fructose, lactulose, sodium chloride, polyethyleneglycol (PEG), maltodextrin, dextran (e.g. dextran 70), hydroxyethyl starch, fluid gelatin, or a synthetic colloid. The agent may comprise a protein and the protein may be a binding protein or an antibody. The binding protein may be albumin. Administering may comprise administering locally, and local administration may comprise administering intraductally. A system for increasing or standardizing an amount of fluid collectable from a milk duct of a breast may comprise infusing a nonabsorbable fluid and/or an osmotic agent and/or an oncotic agent into the ductal lumen, a medical tool for delivering the agent to the ductal lumen, and instructions for use.

Any number of alternative combinations could exist for defining the invention, which incorporate one or more elements from the specification, including the description, claims, and drawings, in various combinations or sub combinations. It will be apparent to those skilled in the relevant technology, in light of the present specification, that alternate combinations of aspects of the invention, either alone or in combination with one or more elements or steps defined herein, may be utilized as modifications or alterations of the invention or as part of the invention. It may be intended that the written description of the invention contained herein covers all such modifications and alterations.

### CLAUSES:

1. A device for being introduced and positioned within a breast duct for introducing or removing material within the breast duct, said apparatus comprising:
   a manifold hub comprising a plurality of port openings in fluid communication with an interior of said manifold hub, at least two of said openings being in fluid communication with a pair of elongated channels that extend through a portion of said manifold hub;
   a catheter extending from the manifold hub, said catheter being sized and configured for positioning within a breast duct; and
   a retractable spacer that may be moved in a direction parallel to the length of the catheter to space the manifold hub a distance above the surface of a nipple.
2. The device of clause 1 wherein said catheter includes a longitudinal axis; and
   said manifold hub includes a height that extends substantially parallel to said longitudinal axis of said catheter and a length that extends substantially perpendicular to said longitudinal axis of said catheter, wherein said length of said manifold hub is greater than the height of said manifold hub.
3. The device of clause 2 wherein said plurality of ports of said manifold hub comprises at least four ports in fluid communication with an interior of said manifold hub, and wherein said pair of channels form a portion of said manifold hub.
4. The device of clause 1 wherein said catheter extends from a first end of said manifold hub, and further comprising a sealing fitting positioned at a second end of said manifold hub opposite said catheter, said sealing fitting including an aperture that may be repeatedly opened and closed.
5. The device of clause 4 wherein said sealing fitting includes a Touhy-Borst fitting for sealing about a member extending through said manifold hub at said second end of said manifold hub.
6. The device of clause 5 wherein said Touhy-Borst fitting comprises a silicone plug comprising said aperture and a threaded cap such that when said cap is turned in a first direction, the silicone plug is altered and a size of said opening within said silicone plug is reduced, thereby forming a seal about said member extending through said manifold hub.
7. The device of clause 1 further including an anchoring member secured to manifold hub for securing the apparatus to the body of a patient.
8. The device of clause 7 wherein said anchoring member has a lower surface for contacting the body of the patient, and said lower surface includes a biocompatible adhesive.
9. The device of clause 1 further comprising a source of negative pressure in communication with said manifold hub for directing a ductal fluid sample from within said manifold hub to a ductal fluid collection receptacle.
10. The device of clause 9 wherein said source of negative pressure comprises a powered vacuum source in fluid communication with said manifold hub.
11. The device of clause 9 wherein said source of negative pressure comprises a mechanically controlled negative pressure source in fluid communication with said manifold hub.
12. The device of clause 1 further comprising a fluid container for holding a fluid being delivered to the breast duct, a fluid infusion member for delivering fluid from said fluid container to said manifold hub, a ductal fluid collection receptacle and a ductal fluid collection member extending between said manifold hub and said collection receptacle for delivering ductal fluid samples from said manifold hub to said collection receptacle.
13. The device of clause 12 wherein said fluid container includes a fluid bag for housing a biocompatible fluid, said fluid bag being positionable vertically above said manifold hub for delivering the biocompatible fluid to said manifold hub.
14. The device of clause 12 wherein said fluid infusion member includes a syringe.
15. The device of clause 12 wherein said ductal fluid collection receptacle includes a syringe.
16. The device of clause 12 wherein said ductal fluid collection receptacle includes a fluid collection bag for being vertically spaced below said manifold hub to receive a ductal fluid sample from within said manifold hub.
17. The device of clause 12 wherein said fluid infusion member and/or said ductal fluid collection member includes a one-way check valve.
18. The device of clause 1 wherein said catheter includes a microcatheter having a lubricious coating that allows it to be easily introduced into the breast duct openings.
19. The device of clause 18 wherein said lubricious coating may include a lubricant, a cleaning agent, anesthetic and/or a dilating agent.
20. The device of clause 18 wherein a proximal end of said microcatheter includes an atraumatic tip.
21. The device of clause 1 wherein said manifold hub is formed of a transparent material.
22. The device of clause 21 wherein said transparent material includes an ABS plastic.
23. The device of clause 1 wherein said spacer comprises at least two telescoping members extending between the manifold hub and a portion of the catheter.
24. The device of clause 1 wherein said spacer includes a member that is moveable relative to the manifold hub such that a portion of the manifold hub is received within said moveable member.
25. The device of clause 1 wherein said manifold hub includes vertical sidewalls with recesses for receiving fingers of a practitioner or attendant for the easy manipulation of said manifold hub relative to the breast.
26. A medical device for introducing a fluid into a breast duct, said device comprising:
   a catheter sized and configured to extend within a breast duct, said catheter having an internal lumen extending substantially parallel to a longitudinal axis of said catheter;
   a manifold hub connected to and in fluid communication with said catheter, said manifold hub including at least four ports in fluid communication within an interior of said manifold hub, at least two of said at least four ports being in fluid communication with channels formed within said manifold hub for receiving a fluid introduction line and a ductal fluid collection line, respectively, said manifold hub having a height extending parallel the longitudinal axis of said catheter and a length extending perpendicular to the longitudinal axis of said catheter, said length of said manifold hub being greater than said height of said manifold hub.
27. The medical device of clause 26 wherein said catheter extends from a first end of said manifold hub, and further comprising a sealing fitting positioned at a second end of said manifold hub opposite said catheter, said sealing fitting including an aperture that may be repeatedly opened and closed.
28. The medical device of clause 27 wherein said sealing fitting includes a Touhy-Borst fitting for sealing about a member extending through said manifold hub at said second end of said manifold hub.
29. The medical device of clause 28 wherein said Touhy-Borst fitting comprises a silicone plug comprising said aperture and a threaded cap such that when said cap is turned in a first direction, the silicone plug is altered and a size of said opening within said silicone plug is reduced, thereby forming a seal about said member extending through said manifold hub.
30. The medical device of clause 26 further comprising an anchoring member secured to manifold hub for securing the device to the body of a patient, said anchoring member having a lower surface carrying a biocompatible adhesive for contacting the body of the patient.
31. The medical device of clause 26 further comprising a source of negative pressure in communication with said manifold hub for directing a ductal fluid sample from within said manifold hub to a ductal fluid collection receptacle.
32. The medical device of clause 31 wherein said source of negative pressure comprises a powered vacuum source in fluid communication with said manifold hub.
33. The medical device of clause 31 wherein said source of negative pressure comprises a mechanically controlled negative pressure source in fluid communication with said manifold hub.
34. The medical device of clause 26 further comprising a fluid container for holding a fluid being delivered to the breast duct, a fluid infusion member for delivering fluid from said fluid container to said manifold hub, a ductal fluid collection receptacle and a ductal fluid collection member extending between said manifold hub and said collection receptacle for delivering ductal fluid samples from said manifold hub to said collection receptacle.
35. The medical device of clause 34 wherein said fluid container includes a fluid bag for housing a biocompatible fluid, said fluid bag being positionable vertically above said manifold hub for delivering the biocompatible fluid to said manifold hub.
36. The medical device of clause 34 wherein said fluid infusion member includes a syringe.
37. The medical device of clause 34 wherein said ductal fluid collection receptacle includes a syringe.
38. The medical device of clause 34 wherein said ductal fluid collection receptacle includes a fluid collection bag for being vertically spaced below said manifold hub to receive a ductal fluid sample from within said manifold hub.
39. The medical device of clause 34 wherein said fluid infusion member and/or said ductal fluid collection member includes a one-way check valve.
40. The medical device of clause 26 wherein said catheter includes a microcatheter having a lubricious coating that allows it to be easily introduced into the breast duct openings.
41. The medical device of clause 40 wherein said lubricious coating may include a lubricant, a cleaning agent, anesthetic and/or a dilating agent.
42. The medical device of clause 40 wherein a proximal end of said microcatheter includes an atraumatic tip.
43. The medical device of clause 26 further comprising an adjustable spacer for positioning said manifold hub a distance above the surface of a nipple.
44. The medical device of clause 43 wherein said spacer comprises at least two telescoping members extending between the manifold hub and a portion of the catheter.
45. The medical device of clause 43 wherein said spacer includes a member that is moveable relative to the manifold hub such that a portion of the manifold hub is received within said moveable member.
46. A ductal access device for aspirating fluid from a breast duct, said device comprising:
   a manifold hub for receiving a fluid to be introduced into a breast duct;
   a catheter extending from said manifold hub, said catheter being sized for positioning within a breast duct and including a lumen for introducing and receiving fluid within the breast duct, said lumen being in fluid communication with said manifold hub and sized to receive a ductal fluid sample from within the breast duct; and
   a source of negative pressure in fluid communication with said manifold hub such that ductal fluid samples within the manifold hub are drawn to said source of negative pressure for being received within a collection device.
47. The ductal access device of clause 46 wherein said catheter includes a longitudinal axis; and said manifold hub includes a height that extends substantially parallel to said longitudinal axis of said catheter and a length that extends substantially perpendicular to said longitudinal axis of said catheter, wherein said length of said manifold hub is greater than the height of said manifold hub.
48. The ductal access device of clause 46 wherein said manifold hub comprises at least four ports in fluid communication with an interior of said manifold hub, and wherein two of said ports being in fluid communication with a pair of channels forming a portion of said manifold hub.
49. The ductal access device of clause 46 wherein said catheter extends from a first end of said manifold hub, and further comprising a Touhy-Borst fitting positioned at a second end of said manifold hub opposite said catheter.
50. The ductal access device of clause 46 further comprising an anchoring member including a lower surface carrying a biocompatible adhesive for securing the device to the body of a patient.
51. The ductal access device of clause 46 wherein said source of negative pressure comprises a powered vacuum source in fluid communication with said manifold hub.
52. The ductal access device of clause 46 wherein said source of negative pressure comprises a mechanically controlled negative pressure source in fluid communication with said manifold hub.
53. The ductal access device of clause 46 further comprising a fluid container for holding a fluid being delivered to the breast duct, a fluid infusion member for delivering fluid from said fluid container to said manifold hub and a ductal fluid collection member extending between said manifold hub and said collection device for delivering ductal fluid samples from said manifold hub to said collection device.
54. The ductal access device of clause 53 wherein said fluid container includes a fluid bag for housing a biocompatible fluid, said fluid bag being positionable vertically above said manifold hub for delivering the biocompatible fluid to said manifold hub.
55. The ductal access device of clause 53 wherein said ductal fluid collection device includes a fluid collection bag for being vertically spaced below said manifold hub to receive a ductal fluid sample from within said manifold hub.
56. The ductal access device of clause 46 wherein said catheter includes a microcatheter having a lubricious coating that allows it to be easily introduced into the breast duct openings.
57. The ductal access device of clause 56 wherein said lubricious coating may include a lubricant, a cleaning agent, anesthetic and/or a dilating agent.
58. The ductal access device of clause 46 further comprising an adjustable spacer for positioning said manifold hub a distance above the surface of a nipple.
59. The ductal access device of clause 58 wherein said spacer comprises at least two telescoping members extending between the manifold hub and a portion of the catheter.
60. The ductal access device of clause 58 wherein said spacer includes a member that is moveable relative to the manifold hub such that a portion of the manifold hub is received within said moveable member.
61. A ductal access device, comprising:
   an elongated member sized for positioning within a breast duct, said elongated member comprising an elongated internal lumen extending along a length of said elongated member and an elongated internal pathway extending along at least a portion of the length of the elongated member for receiving an introducer, said elongated member including a central longitudinal axis positioned and extending within the internal lumen and said elongated internal passageway including a centrally disposed longitudinal axis that is spaced from and offset from said central longitudinal axis; and
   said elongated member including an elongated distal tip extending beyond a distal end of the internal lumen and along said longitudinal axis of said elongated internal pathway.
62. The ductal access device of clause 61, the elongated distal tip including said elongated internal passageway therein.
63. The ductal access device of clause 62, further comprising a multiport body attached to a proximal end of the internal lumen and communicating with said internal lumen for infusing fluid into the breast duct and for retrieving fluid from the breast duct.
64. The ductal access device of clause 61, in which said elongated distal tip includes a plurality of sections extending along a length each having a different stiffness.
65. The ductal access device of clause 61, in which the elongated distal member includes a closed distal tip configured to abut a distal end of said introducer.
66. The ductal access device of clause 61, in which the introducer includes a plurality of sections extending along a length thereof and each section having a different stiffness.
67. The ductal access device of clause 61, in which the elongated passageway is configured to receive a plurality of introducers of different predetermined lengths.
68. A ductal access device for accessing a breast duct, said device comprising:
   an elongated member comprising an internal lumen and being sized for positioning within the breast; said elongated member having a first centrally positioned axis extending through the internal lumen and a second axis being substantially parallel and offset relative to said first centrally positioned axis, said internal lumen having a distal end and an opposing proximal end; an elongated distal tip extending beyond said distal end of the internal lumen and along said second axis, said elongated distal tip having a closed distal end for navigating within the breast duct; and
   a multiport body attached to the proximal end of the internal lumen and communicating with said lumen for infusing fluid into the breast duct and for retrieving fluid from the breast duct.
69. The device according to clause 68, in which the elongated distal tip includes an internal passageway sized to removably receive an elongated introducer therein.
70. The device according to clause 69, in which the said passageway extends through said body.
71. The device according to clause 69, in which the said passageway extends within a wall of the elongated member to the proximal end of the lumen.
72. The device according to clause 68, in which the elongated distal tip extends from a wall of said lumen.
73. The device according to clause 68, further including a beveled surface being disposed between the distal end of the internal lumen and a proximal end of the elongated distal tip.
74. A ductal access system, said system, comprising:
   a cannula sized for positioning within the breast duct and an internal lumen; said cannula having a first axis extending through the internal lumen and a second axis being offset and substantially parallel to said first axis, said internal lumen having a distal end and an opposing proximal end, an elongated distal member extending beyond said distal end of the internal lumen and along said second axis, said elongated distal member including an internal passageway sized to slidiably receive an elongated introducer therein; and
   a multiport body attached to the proximal end of the internal lumen and communicating with said lumen for infusing fluid into the breast duct and for retrieving fluid from the breast duct.
75. The system according to clause 74, in which the said passageway extends through said body.
76. The system according to clause 74, in which the elongated distal member includes a closed distal tip configured to abut a distal end of said introducer.
77. The system according to clause 74, in which the elongated distal member includes an opened distal tip in which said introducer is extensible therethrough.
78. The system according to clause 74, in which the elongated distal member extends from a wall of said lumen.
79. The system according to clause 74, in which the introducer member is composed of a superelastic material.
80. The system according to clause 74, in which the elongated introducer includes a plurality of introducers each being of a different stiffness.
81. A method for obtaining cellular material from a human breast milk duct using a ductal access device of clause 1 includes the steps of:
   inserting the introducer into the internal passageway of the elongated distal member to form a composite insertion member;
   inserting the composite insertion member into a ductal orifice of the breast duct; and
   advancing the elongated member into the breast duct.
82. The method of clause 81 including a step of removing the introducer from the internal passageway.
83. A method for obtaining cellular material from a human breast milk duct using a ductal access device of clause 81 includes the steps of :
   inserting the elongated distal member into a ductal orifice;
   inserting the introducer into the internal passageway of the elongated distal member to form a composite insertion member; and
   advancing the composite insertion member and the internal lumen into the breast duct.
84. A device for being introduced and positioned within a breast duct for introducing or removing material within the breast duct, said apparatus comprising:
   a manifold hub comprising a plurality of port openings in fluid communication with an interior of said manifold hub, at least two of said openings being in fluid communication with a pair of elongated channels that extend through a portion of said manifold hub; and
   a catheter extending from the manifold hub, said catheter comprising an elongated internal lumen extending substantially parallel to a longitudinal axis of said catheter, said lumen being in fluid communication with said manifold hub and adapted to slidably receive a ductal introducer therein, said introducer extends at least 0.250 inches beyond the distal tip of said catheter.
85. The device of clause 84 wherein said introducer includes, but is not limited to, guidewires, dilators, stylettes or any portion thereof that may be inserted within said internal lumen of said catheter.
86. The device of clause 84, in which said catheter includes a closed distal tip configured to abut a distal end of said introducer.
87. The device of clause 84, in which the introducer extends at least 0.492 inches beyond the distal tip of said catheter.
88. The device of clause 84, in which the introducer extends at least 0.984 inches beyond the distal tip of said catheter.
89. The device of clause 84 in which the distal tip of the catheter has an atraumatic configuration.
90. A device for being introduced and positioned within a breast duct for introducing or removing material within the breast duct, said apparatus comprising:
   a manifold hub comprising a plurality of port openings in fluid communication with an interior of said manifold hub, at least two of said openings being in fluid communication with a pair of elongated channels that extend through a portion of said manifold hub; and
   a catheter extending from the manifold hub; and
   a sheath which extends internally through both the hub and the catheter, comprising an elongated internal lumen extending substantially parallel to a longitudinal axis of said sheath, said lumen being in fluid communication with said manifold hub and adapted to slideably receive a ductal introducer therein and said sheath extends beyond the distal tip of said catheter.
91. The device of clause 90, in which said sheath includes a closed distal tip configured to abut a distal end of said introducer.
92. The device of clause 90, in which the introducer extends at least 0.250 inches beyond the distal tip of said catheter.
93. The device of clause 90, in which the introducer extends at least 0.492 inches beyond the distal tip of said catheter.
94. The device of clause 90, in which the introducer extends at least 0.984 inches beyond the distal tip of said catheter.
95. The device of clause 90 in which the distal tip of the catheter and the sheath has an atraumatic configuration.
96. The device of clause 90 in which the distal tip of the sheath extends at least 1.5 mm inches beyond the distal tip of said catheter.
97. The device of clause 90 in which the distal tip of the sheath extends at least 6.0 mm inches beyond the distal tip of said catheter.
98. The device of clause 90 in which the distal tip of the sheath extends at least 18.5 mm inches beyond the distal tip of said catheter.
99. A method for obtaining cellular material from a human breast milk duct using the device of clause 84 including the steps of :
   inserting the introducer into the internal passageway of a breast duct; and
   advancing the catheter into the breast duct.
100. A method for obtaining cellular material from a human breast milk duct using the device of clause 90 including the steps of:
   inserting the introducer into the internal passageway of a breast duct;
   advancing the sheath into the breast duct;
   and advancing the catheter into the breast duct.
101. A method for passing a medical instrument through a sphincter, said method comprising:
   bringing a medical instrument into proximity to a sphincter;
   introducing a pressurized fluid through said medical instrument such that said fluid comes into contact with said sphincter;
   passing the medical instrument through to said opened sphincter.
102. The method of clause 101 wherein said medical instrument is a catheter.
103. The method of clause 101 wherein an increase in fluid pressure causes said sphincter to open.
104. The method of clause 101 wherein said fluid pressure is applied by a fluid pump, mouth pipetting, or gravity.
105. The method of clause 101 wherein said fluid is warmed to body temperature before being introduced into said catheter.
106. A method for passing a catheter through a ductal sphincter, said method comprising:
   bringing a catheter into proximity to a ductal sphincter;
   introducing fluid through said catheter such that said fluid comes into contact with said ductal sphincter;
   applying pressure to said fluid such that an increase in fluid pressure causes said ductal sphincter to open; and
   passing the catheter through to said opened ductal sphincter.
107. The method of clause 106 wherein said fluid pressure is applied by a fluid pump, mouth pipetting, or gravity.
108. The method of clause 106 wherein said ductal sphincter is located in a breast duct.
109. The method of clause 106 wherein said fluid is warmed to body temperature before being introduced into said catheter.
110. A method for passing a catheter through a duct that is difficult to cannulate, said method comprising:
   bringing a catheter into proximity to a duct;
   introducing fluid through said catheter such that said fluid comes into contact with said duct;
   applying pressure to said fluid such that an increase in fluid pressure causes said duct to open or straighten;
   passing the catheter through to said opened or straightened duct.
111. The method of clause 110 wherein said fluid pressure is applied by a fluid pump, mouth pipetting, or gravity.
112. The method of clause 110 wherein said fluid is warmed to body temperature before being introduced into said catheter.
113. A device for being introduced and positioned within a breast duct for introducing or removing material within the breast duct, said apparatus comprising:
   a manifold hub comprising a fitting for sealing about a ductal introducer extending through said manifold hub at one end of said manifold hub said fitting including an aperture that may be repeatedly opened and closed, the sides of said manifold hub comprising two opposing flexible arms connected at one end to the base of said hub and unconnected at the other end and substantially parallel to one another, said arms are held in a sprung position such that said unconnected ends of said arms compress said fitting such that said aperture is closed and when said arms are compressed, said unconnected ends of said arms no longer compress said fitting and said aperture is opened.
114. The device of clause 113 wherein said sealing fitting is a silicone gasket.
115. The device of clause 113 wherein springs are located between said flexible arms and the body of said hub such that the springs keep said arms in actuated position.
116. The device of clause 113 wherein the free ends of said arms are interlocking hooks.
117. The device of clause 113 wherein said ductal introducer has a ring shaped handle.
118. The device of clause 113 wherein said ductal introducer is made of Nitinol and has a distal tip that tapers.
119. The device of clause 113 wherein said introducer tapers from approximately 0.025 inches to approximately 0.008 inches.
120. A method for obtaining cellular material from a human breast milk duct using a device comprising a manifold hub comprising a fitting for sealing about a ductal introducer extending through said manifold hub at one end of said manifold hub said fitting including an aperture that may be repeatedly opened and closed, the sides of said manifold hub comprising two opposing flexible arms connected at one end to the base of said hub and unconnected at the other end and substantially parallel to one another, said arms are held in a sprung position such that said unconnected ends of said arms compress said fitting such that said aperture is closed and when said arms are compressed, said unconnected ends of said arms no longer compress said fitting and said aperture is opened; and including the steps of:
   inserting the introducer into the internal passageway of a breast duct; and
   advancing the catheter into the breast duct.
121. A method for obtaining cellular material from a human breast milk duct using a device comprising a manifold hub comprising a fitting for sealing about a ductal introducer extending through said manifold hub at one end of said manifold hub said fitting including an aperture that may be repeatedly opened and closed, the sides of said manifold hub comprising two opposing flexible arms connected at one end to the base of said hub and unconnected at the other end and substantially parallel to one another, said arms are held in a sprung position such that said unconnected ends of said arms compress said fitting such that said aperture is closed and when said arms are compressed, said unconnected ends of said arms no longer compress said fitting and said aperture is opened ; and including the steps of:
   inserting the introducer into the internal passageway of a breast duct;
   advancing the sheath into the breast duct; and advancing the catheter into the breast duct.

## Claims

1. A device for being introduced and positioned within a breast duct for introducing or removing material within the breast duct, said apparatus comprising:
a manifold hub comprising a fitting for sealing about a ductal introducer extending through said manifold hub at one end of said manifold hub said fitting including an aperture that may be repeatedly opened and closed, the sides of said manifold hub comprising two opposing flexible arms connected at one end to the base of said hub and unconnected at the other end and substantially parallel to one another, said arms are held in a sprung position such that said unconnected ends of said arms compress said fitting such that said aperture is closed and when said arms are compressed, said unconnected ends of said arms no longer compress said fitting and said aperture is opened.

2. The device of claim 1 wherein said sealing fitting is a silicone gasket.

3. The device of claim 1 wherein springs are located between said flexible arms and the body of said hub such that the springs keep said arms in actuated position.

4. The device of claim 1 wherein the free ends of said arms are interlocking hooks.

5. The device of claim 1 wherein said ductal introducer has a ring shaped handle.

6. The device of claim 1 wherein said ductal introducer is made of Nitinol and has a distal tip that tapers.

7. The device of claim 1 wherein said introducer tapers from approximately 0.025 inches to approximately 0.008 inches.

8. A method for obtaining cellular material from a human breast milk duct using a device comprising a manifold hub comprising a fitting for sealing about a ductal introducer extending through said manifold hub at one end of said manifold hub said fitting including an aperture that may be repeatedly opened and closed, the sides of said manifold hub comprising two opposing flexible arms connected at one end to the base of said hub and unconnected at the other end and substantially parallel to one another, said arms are held in a sprung position such that said unconnected ends of said arms compress said fitting such that said aperture is closed and when said arms are compressed, said unconnected ends of said arms no longer compress said fitting and said aperture is opened; and including the steps of:
inserting the introducer into the internal passageway of a breast duct; and
advancing the catheter into the breast duct.

9. A method for obtaining cellular material from a human breast milk duct using a device comprising a manifold hub comprising a fitting for sealing about a ductal introducer extending through said manifold hub at one end of said manifold hub said fitting including an aperture that may be repeatedly opened and closed, the sides of said manifold hub comprising two opposing flexible arms connected at one end to the base of said hub and unconnected at the other end and substantially parallel to one another, said arms are held in a sprung position such that said unconnected ends of said arms compress said fitting such that said aperture is closed and when said arms are compressed, said unconnected ends of said arms no longer compress said fitting and said aperture is opened ; and including the steps of:
inserting the introducer into the internal passageway of a breast duct;
advancing the sheath into the breast duct; and advancing the catheter into the breast duct.
